# EUROPÄISCHE PATENTSCHRIFT

(11) **EP 3 548 486 B1**
(45) Veröffentlichungstag und Bekanntmachung des Hinweises auf die Patenterteilung: **27.10.2021**
(21) Anmeldenummer: 17807884.6
(22) Anmeldetag: 04.12.2017
(51) Int. Cl.: C07D 471/16, H01L 51/00, H01L 51/50, C07D 491/16, C07D 495/16, C07D 519/00, C09K 11/06

(54) **MATERIALIEN FÜR ORGANISCHE ELEKTROLUMINESZENZVORRICHTUNGEN**
MATERIALS FOR ORGANIC ELECTROLUMINESCENT DEVICES
MATÉRIAUX POUR DISPOSITIFS ÉLECTROLUMINESCENTS ORGANIQUES

(30) Priorität: 05.12.2016 EP 16202146
(43) Veröffentlichungstag der Anmeldung: 09.10.2019
(73) Patentinhaber: Merck Patent GmbH, 64293 Darmstadt (DE)
(72) Erfinder: PARHAM, Amir, 60486 Frankfurt am Main (DE); JOOSTEN, Dominik, 60487 Frankfurt am Main (DE); LUDEMANN, Aurélie, 60322 Frankfurt am Main (DE); GROSSMANN, Tobias, 64297 Darmstadt (DE); KROEBER, Jonas, 60311 Frankfurt am Main (DE); EICKHOFF, Christian, 68259 Mannheim (DE)
(86) Internationale Anmeldenummer: PCT/EP2017/081291
(87) Internationale Veröffentlichungsnummer: WO 2018/104194

(56) Entgegenhaltungen:
- WO-A1-2014/056567
- KR-A- 20150 065 381
- KR-A- 20150 065 383
- US-A- 5 432 172
- US-A1- 2017 352 820
- DATABASE PUBCHEM compound [Online] 19 December 2011 (2011-12-19), XP055689519, retrieved from NCBI Database accession no. 54608964
- DATABASE PUBCHEM compound [Online] 19 December 2011 (2011-12-19), XP055689524, retrieved from NCBI Database accession no. 54608892
- DATABASE PUBCHEM compound [Online] 19 December 2011 (2011-12-19), XP55689530, retrieved from NCBI Database accession no. 54608965
- DATABASE PUBCHEM compound [Online] 19 December 2011 (2011-12-19), XP55689531, retrieved from NCBI Database accession no. 54608995
- DATABASE PUBCHEM compound [Online] 19 December 2011 (2011-12-19), XP55689532, retrieved from NCBI Database accession no. 54608996
- DATABASE PUBCHEM compound [Online] 19 December 2011 (2011-12-19), XP55689534, retrieved from NCBI Database accession no. 54608997

## Beschreibung

Die vorliegende Erfindung betrifft Materialien für die Verwendung in elektronischen Vorrichtungen, insbesondere in organischen Elektrolumineszenzvorrichtungen, sowie elektronische Vorrichtungen, insbesondere organische Elektrolumineszenzvorrichtungen enthaltend diese Materialien.

US 5432172 offenbart polyzyklische, naturstoffähnliche Alkaloide mit biologisch wertvollen Eigenschaften. Weiterhin offenbart die Datenbank PubChem verschiedene relevante Verbindungen. Diese Verbindungen werden nicht für die Verwendung in Elektrolumineszenzvorrichtungen offenbart und sind vom Gegenstand der Ansprüche per Maßgabe ausgeschlossen.

In organischen Elektrolumineszenzvorrichtungen (OLEDs) werden als emittierende Materialien häufig metallorganische Komplexe eingesetzt, die Phosphoreszenz statt Fluoreszenz zeigen. Aus quantenmechanischen Gründen ist unter Verwendung metallorganischer Verbindungen als Phosphoreszenzemitter eine bis zu vierfache Energie- und Leistungseffizienz möglich. Generell gibt es bei OLEDs, insbesondere auch bei OLEDs, die Triplettemission (Phosphoreszenz) zeigen, jedoch immer noch Verbesserungsbedarf, beispielsweise im Hinblick auf Effizienz, Betriebsspannung und Lebensdauer. Die Eigenschaften phosphoreszierender OLEDs werden nicht nur von den eingesetzten Triplettemittern bestimmt. Hier sind insbesondere auch die anderen verwendeten Materialien, wie Matrixmaterialien, von besonderer Bedeutung. Verbesserungen dieser Materialien und deren Ladungstransporteigenschaften können somit auch zu deutlichen Verbesserungen der OLED-Eigenschaften führen. KR 2015/0065381 offenbart polycyclische Verbindungen, die für die Verwendung in organischen Elektrolumineszenzvorrichtungen geeignet sind.

Aufgabe der vorliegenden Erfindung ist die Bereitstellung von Verbindungen, welche sich für den Einsatz in einer OLED eignen, insbesondere als Matrixmaterial für phosphoreszierende Emitter. Eine weitere Aufgabe der vorliegenden Erfindung ist es, weitere organische Halbleiter für organische Elektrolumineszenzvorrichtungen bereitzustellen, um so dem Fachmann eine größere Wahlmöglichkeit an Materialien für die Herstellung von OLEDs zu ermöglichen.

Überraschend wurde gefunden, dass bestimmte, unten näher beschriebene Verbindungen diese Aufgabe lösen, sich gut für die Verwendung in OLEDs eignen und zu Verbesserungen der organischen Elektrolumineszenzvorrichtung führen. Dabei betreffen die Verbesserungen insbesondere die Lebensdauer, die Effizienz und/oder die Betriebsspannung. Diese Verbindungen sowie elektronische Vorrichtungen, insbesondere organische Elektrolumineszenzvorrichtungen, welche derartige Verbindungen enthalten, sind daher der Gegenstand der vorliegenden Erfindung.

Gegenstand der vorliegenden Erfindung ist daher eine Verbindung gemäß Formel (1),
**(1') oder (4),** 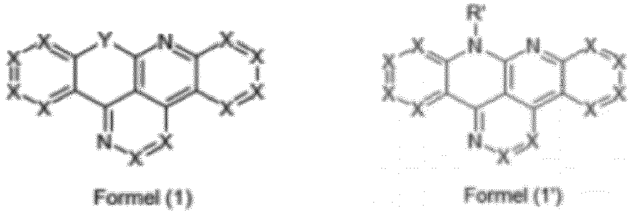 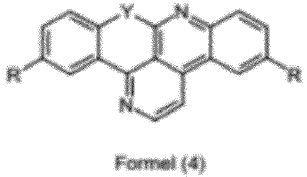 wie in den beigefügten Ansprüchen definiert.

Eine Arylgruppe im Sinne dieser Erfindung enthält 6 bis 40 C-Atome; eine Heteroarylgruppe im Sinne dieser Erfindung enthält 2 bis 40 C-Atome und mindestens ein Heteroatom, mit der Maßgabe, dass die Summe aus C-Atomen und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin, Thiophen, etc., oder eine kondensierte (anellierte) Aryl- oder Heteroarylgruppe, beispielsweise Naphthalin, Anthracen, Phenanthren, Chinolin, Isochinolin, etc., verstanden. Miteinander durch Einfachbindung verknüpfte Aromaten, wie zum Beispiel Biphenyl, werden dagegen nicht als Aryl- oder Heteroarylgruppe, sondern als aromatisches Ringsystem bezeichnet.

Ein aromatisches Ringsystem im Sinne dieser Erfindung enthält 6 bis 60 C-Atome im Ringsystem. Ein heteroaromatisches Ringsystem im Sinne dieser Erfindung enthält 2 bis 60 C-Atome und mindestens ein Heteroatom im Ringsystem, mit der Maßgabe, dass die Summe aus C-Atomen und Heteroatomen mindestens 5 ergibt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit, wie z. B. ein C-, N- oder O-Atom, verbunden sein können. So sollen beispielsweise auch Systeme wie Fluoren, 9,9'-Spirobifluoren, 9,9-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine kurze Alkylgruppe verbunden sind.

Im Rahmen der vorliegenden Erfindung werden unter einem aliphatischen Kohlenwasserstoffrest bzw. einer Alkylgruppe bzw. einer Alkenyl- oder Alkinylgruppe, die 1 bis 40 C-Atome enthalten kann, und in der auch einzelne H-Atome oder CH₂-Gruppen durch die oben genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, neo-Pentyl, Cyclopentyl, n-Hexyl, neo-Hexyl, Cyclohexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl, Heptinyl oder Octinyl verstanden. Unter einer Alkoxygruppe mit 1 bis 40 C-Atomen werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy und 2,2,2-Trifluorethoxy verstanden. Unter einer Thioalkylgruppe mit 1 bis 40 C-Atomen werden insbesondere Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden. Allgemein können Alkyl-, Alkoxy- oder Thioalkylgruppen gemäß der vorliegenden Erfindung geradkettig, verzweigt oder cyclisch sein, wobei eine oder mehrere nicht-benachbarte CH₂-Gruppen durch die oben genannten Gruppen ersetzt sein können; weiterhin können auch ein oder mehrere H-Atome durch D, F, Cl, Br, I, CN oder NO₂, bevorzugt F, Cl oder CN, weiter bevorzugt F oder CN, besonders bevorzugt CN ersetzt sein.

Unter einem aromatischen oder heteroaromatischen Ringsystem mit 5 - 60 aromatischen Ringatomen, welches noch jeweils mit den oben genannten Resten R² oder einem Kohlenwasserstoffrest substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Benzanthracen, Phenanthren, Pyren, Chrysen, Perylen, Fluoranthen, Naphthacen, Pentacen, Benzpyren, Biphenyl, Biphenylen, Terphenyl, Triphenylen, Fluoren, Spirobifluoren, Dihydrophenanthren, Dihydropyren, Tetrahydropyren, cis- oder trans-Indenofluoren, cis- oder trans-Indenocarbazol, cis- oder trans-Indolocarbazol, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Hexaazatriphenylen, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol oder Gruppen, die abgeleitet sind von Kombination dieser Systeme.

Wenn zwei Reste R bzw. bzw. R" bzw. R¹ miteinander ein Ringsystem bilden, so kann dieses mono- oder polycyclisch, aliphatisch, heteroaliphatisch bzw. für Reste R" bzw. R¹ auch aromatisch oder heteroaromatisch sein. Dabei sind die Reste, die miteinander ein Ringsystem bilden, bevorzugt benachbart, d. h. dass diese Reste an dasselbe Kohlenstoffatom oder an Kohlenstoffatome, die direkt aneinander gebunden sind, gebunden sind.

Unter der Formulierung, dass zwei oder mehr Reste miteinander einen Ring bilden können, soll im Rahmen der vorliegenden Beschreibung unter anderem verstanden werden, dass die beiden Reste miteinander durch eine chemische Bindung unter formaler Abspaltung von zwei Wasserstoffatomen verknüpft sind. Dies wird durch das folgende Schema verdeutlicht.

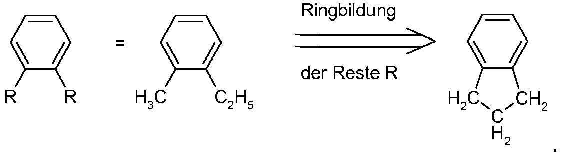

Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet. Dies soll durch das folgende Schema verdeutlicht werden:

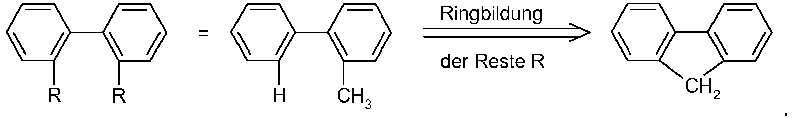

In einer bevorzugten Ausführungsform der Erfindung steht Y für NR', O oder S, besonders bevorzugt für NR' oder O und ganz besonders bevorzugt für NR'. Verbindungen mit Y = C=O eignen sich insbesondere als Zwischenstufe, beispielsweise für die Synthese der entsprechenden Spiro-Verbindungen.

Bevorzugt handelt es sich somit um eine Verbindung gemäß einer der folgenden Formeln (1') oder (1"), 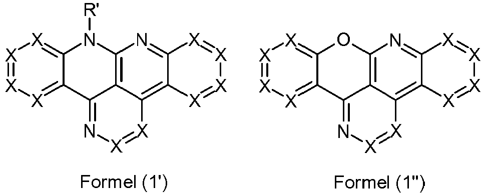 wobei X die in den Ansprüchen genannte Bedeutung aufweist.

Bevorzugt enthalten die Verbindungen maximal ein Stickstoffatom pro Ring, das heißt, es steht maximal ein Symbol X pro Ring für N, bzw. in dem Ring mit dem explizit eingezeichneten Stickstoffatom steht keines der Symbole X für N.

Ganz besonders bevorzugt stehen alle Symbole X für CR.

Bevorzugt sind die Verbindungen der Formeln (2a) bis (2k), 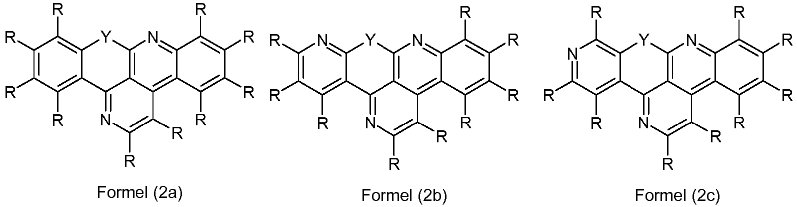 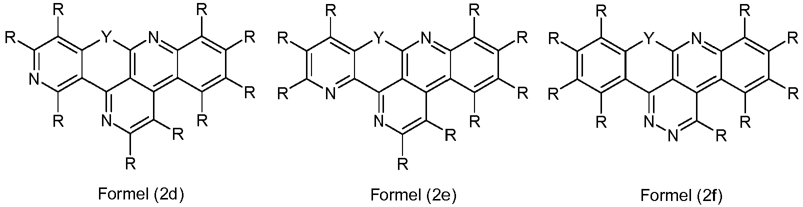 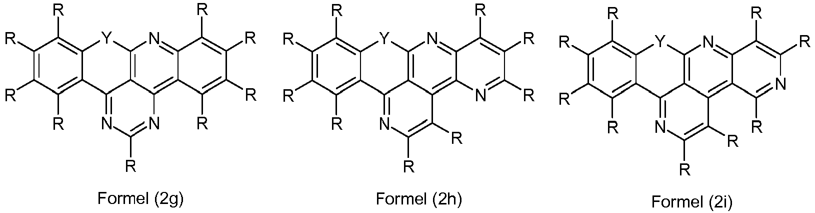 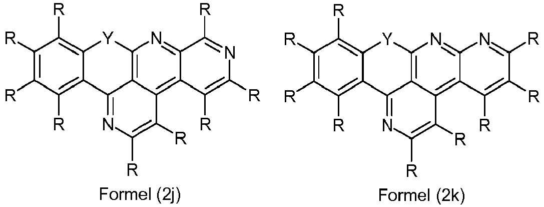 wobei die verwendeten Symbole die in den Ansprüchen genannten Bedeutungen aufweisen.

Bevorzugt sind die Verbindungen (2a) bis (2e) und (2h) bis (2k). Besonders bevorzugt ist die Verbindung der Formel (2a). Ganz besonders bevorzugt sind die Verbindungen der Formeln (2a') oder (2a"), 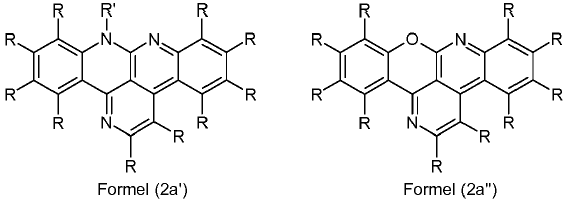 wobei R und R' die in den Ansprüchen genannten Bedeutungen aufweisen und mindestens eine Gruppe R in Formel (2a") ausgewählt ist aus der Gruppe bestehend aus NAr₂ und einem aromatischen oder heteroaromatischen Ringsystem.

Eine bevorzugte Ausführungsform der Verbindungen der Formel (2a) sind die Verbindungen der folgenden Formel (3), 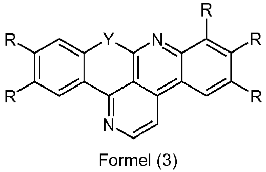 wobei die verwendeten Symbole die in den Ansprüchen genannten Bedeutungen aufweisen.

Besonders bevorzugt sind die Verbindungen der Formeln (3') und (3"), 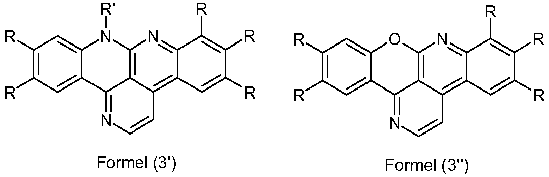 wobei R und R' die oben genannten Bedeutungen aufweisen und mindestens eine Gruppe R in Formel (3") ausgewählt ist aus der Gruppe bestehend aus NAr₂ und einem aromatischen oder heteroaromatischen Ringsystem.

Eine bevorzugte Ausführungsform der Verbindungen der Formel (3) sind die Verbindungen der folgenden Formel (4), 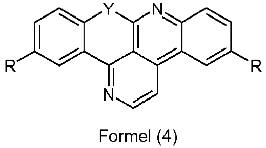 wobei die verwendeten Symbole die in den Ansprüchen genannten Bedeutungen aufweisen.

Besonders bevorzugt sind die Verbindungen der Formeln (4') und (4"), 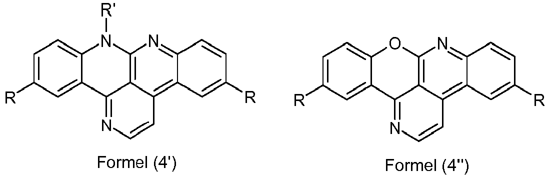 wobei R und R' die oben genannten Bedeutungen aufweisen und mindestens eine Gruppe R in Formel (4") ausgewählt ist aus der Gruppe bestehend aus NAr₂ und einem aromatischen oder heteroaromatischen Ringsystem.

Besonders bevorzugt sind die Verbindungen der folgenden Formeln (5) und (6), 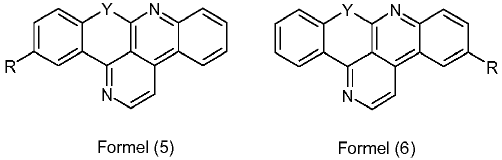 wobei die verwendeten Symbole die in den Ansprüchen genannten Bedeutungen aufweisen.

Ganz besonders bevorzugt sind die Verbindungen der Formeln (5'), (5"), (6') und (6"), 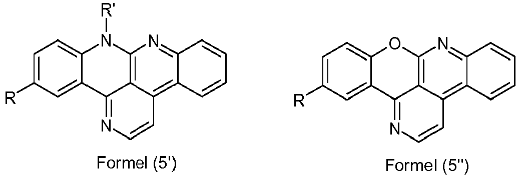 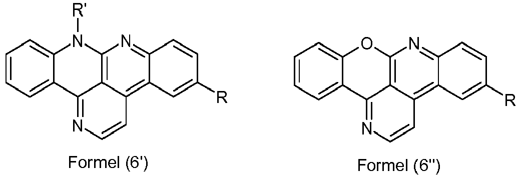 wobei R und R' die oben genannten Bedeutungen aufweisen und die Gruppe R in Formel (5') und (6') ausgewählt ist aus der Gruppe bestehend aus H, NAr₂ und einem aromatischen oder heteroaromatischen Ringsystem und die Gruppe R in Formel (5") und (6") ausgewählt ist aus der Gruppe bestehend aus NAr₂ und einem aromatischen oder heteroaromatischen Ringsystem.

Im Folgenden werden bevorzugte Substituenten R und R' an den erfindungsgemäßen Verbindungen beschrieben.

In einer bevorzugten Ausführungsform der Erfindung ist R bei jedem Auftreten gleich oder verschieden ausgewählt aus der Gruppe bestehend aus H, D, F, NAr₂, CN, OR¹, einer geradkettige Alkylgruppe mit 1 bis 10 C-Atomen oder einer Alkenylgruppe mit 2 bis 10 C-Atomen oder einer verzweigten oder cyclischen Alkylgruppe mit 3 bis 10 C-Atomen, wobei die Alkyl- bzw. Alkenylgruppe jeweils mit einem oder mehreren Resten R¹ substituiert sein kann und wobei eine oder mehrere nicht benachbarte CH₂-Gruppen durch O ersetzt sein können, oder einem aromatischen oder heteroaromatischen Ringsystem mit 6 bis 30 aromatischen Ringatomen, das jeweils durch einen oder mehrere Reste R¹ substituiert sein kann; dabei können zwei Reste R auch miteinander ein aliphatisches Ringsystem bilden. Besonders bevorzugt ist R bei jedem Auftreten gleich oder verschieden ausgewählt aus der Gruppe bestehend aus H, NAr₂, einer geradkettige Alkylgruppe mit 1 bis 6 C-Atomen, insbesondere mit 1, 2, 3 oder 4 C-Atomen, oder einer verzweigten oder cyclischen Alkylgruppe mit 3 bis 6 C-Atomen, wobei die Alkylgruppe jeweils mit einem oder mehreren Resten R¹ substituiert sein kann, bevorzugt aber unsubstituiert ist, oder einem aromatischen oder heteroaromatischen Ringsystem mit 6 bis 24 aromatischen Ringatomen, das jeweils durch einen oder mehrere, bevorzugt nicht-aromatische, Reste R¹ substituiert sein kann. Ganz besonders bevorzugt ist R bei jedem Auftreten gleich oder verschieden ausgewählt aus der Gruppe bestehend aus H oder einem aromatischen oder heteroaromatischen Ringsystem mit 6 bis 24 aromatischen Ringatomen, das jeweils durch einen oder mehrere, bevorzugt nicht-aromatische, Reste R¹ substituiert sein kann.

In einer weiteren bevorzugten Ausführungsform der Erfindung ist R' ein aromatisches oder heteroaromatisches Ringsystem mit 6 bis 30 aromatischen Ringatomen, das durch einen oder mehrere Reste R¹ substituiert sein kann. In einer besonders bevorzugten Ausführungsform der Erfindung ist R' ein aromatisches oder heteroaromatisches Ringsystem mit 6 bis 24 aromatischen Ringatomen, das durch einen oder mehrere, bevorzugt nicht-aromatische, Reste R¹ substituiert sein kann.

In einer weiteren bevorzugten Ausführungsform der Erfindung ist R¹ gleich oder verschieden bei jedem Auftreten ausgewählt aus der Gruppe bestehend aus H, D, F, CN, OR², einer geradkettigen Alkylgruppe mit 1 bis 10 C-Atomen oder einer Alkenylgruppe mit 2 bis 10 C-Atomen oder einer verzweigten oder cyclischen Alkylgruppe mit 3 bis 10 C-Atomen, wobei die Alkyl- bzw. Alkenylgruppe jeweils mit einem oder mehreren Resten R² substituiert sein kann und wobei eine oder mehrere nicht benachbarte CH₂-Gruppen durch O ersetzt sein können, oder einem aromatischen oder heteroaromatischen Ringsystem mit 6 bis 30 aromatischen Ringatomen, das jeweils durch einen oder mehrere Reste R² substituiert sein kann; dabei können zwei oder mehrere Reste R¹ miteinander ein aliphatisches Ringsystem bilden. In einer besonders bevorzugten Ausführungsform der Erfindung ist R¹ gleich oder verschieden bei jedem Auftreten ausgewählt aus der Gruppe bestehend aus H, einer geradkettigen Alkylgruppe mit 1 bis 6 C-Atomen, insbesondere mit 1, 2, 3 oder 4 C-Atomen, oder einer verzweigten oder cyclischen Alkylgruppe mit 3 bis 6 C-Atomen, wobei die Alkylgruppe mit einem oder mehreren Resten R² substituiert sein kann, bevorzugt aber unsubstituiert ist, oder einem aromatischen oder heteroaromatischen Ringsystem mit 6 bis 24 aromatischen Ringatomen, das jeweils durch einen oder mehrere Reste R² substituiert sein kann, bevorzugt aber unsubstituiert ist.

In einer weiteren bevorzugten Ausführungsform der Erfindung ist R² gleich oder verschieden bei jedem Auftreten H, eine Alkylgruppe mit 1 bis 4 C-Atomen oder eine Arylgruppe mit 6 bis 10 C-Atomen, welche mit einer Alkylgruppe mit 1 bis 4 C-Atomen substituiert sein kann, bevorzugt aber unsubstituiert ist.

Wie oben beschrieben, enthält die erfindungsgemäße Verbindung mindestens eine Gruppe R', und/oder sie enthält mindestens eine Gruppe R, welche ausgewählt ist aus der Gruppe bestehend aus NAr₂ und einem aromatischen oder heteroaromatischen Ringsystem mit 5 bis 60 aromatischen Ringatomen und welches durch einen oder mehrere Reste R¹ substituiert sein kann. Dabei kann es für Y = NR' bevorzugt sein, wenn das Grundgerüst unsubstituiert ist, das heißt, wenn R = H ist.

Nachfolgend werden bevorzugte aromatische und heteroaromatische Ringsysteme beschrieben, welche als Substituent R und/oder R' oder als Gruppe Ar innerhalb des Substituenten NAr₂ in der erfindungsgemäßen Verbindung vorliegen können.

Geeignete aromatische bzw. heteroaromatische Ringsystem R, R' bzw. Ar sind ausgewählt aus Phenyl, Biphenyl, insbesondere ortho-, meta- oder para-Biphenyl, Terphenyl, insbesondere ortho-, meta-, para- oder verzweigtem Terphenyl, Quaterphenyl, insbesondere ortho-, meta-, para- oder verzweigtem Quaterphenyl, Fluoren, welches über die 1-, 2-, 3- oder 4-Position verknüpft sein kann, Spirobifluoren, welches über die 1-, 2-, 3- oder 4-Position verknüpft sein kann, Naphthalin, insbesondere 1- oder 2-verknüpftem Naphthalin, Indol, Benzofuran, Benzothiophen, Carbazol, welches über die 1-, 2-, 3- oder 4-Position verknüpft sein kann, Dibenzofuran, welches über die 1-, 2-, 3- oder 4-Position verknüpft sein kann, Dibenzothiophen, welches über die 1-, 2-, 3- oder 4-Position verknüpft sein kann, Indenocarbazol, Indolocarbazol, Pyridin, Pyrimidin, Pyrazin, Pyridazin, Triazin, Phenanthren, Triphenylen oder einer Kombination aus zwei oder drei dieser Gruppen, welche jeweils mit einem oder mehreren Resten R¹ substituiert sein können.

Dabei sind die Gruppen R, R' bzw. Ar bevorzugt gewählt aus den Gruppen der folgenden Formeln Ar-1 bis Ar-75, 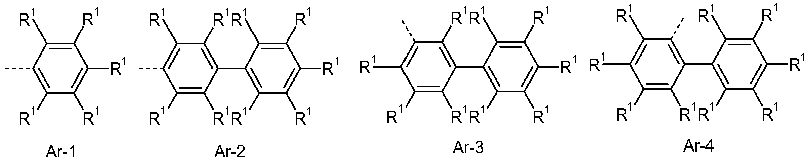 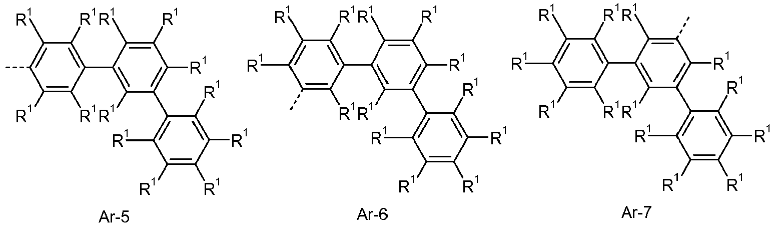 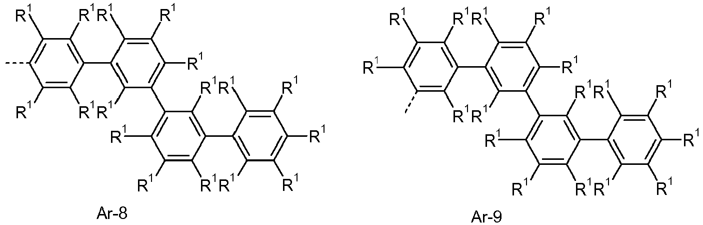 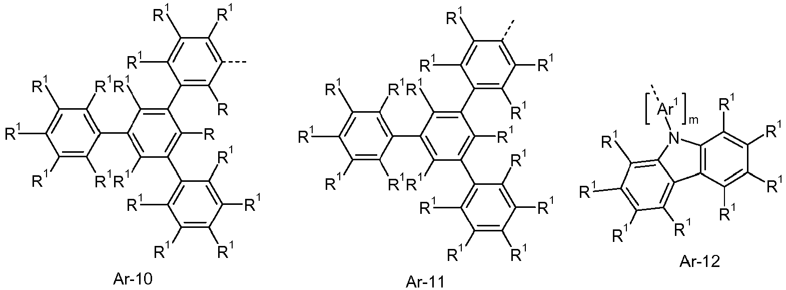 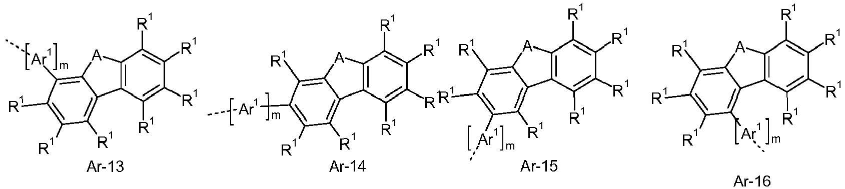 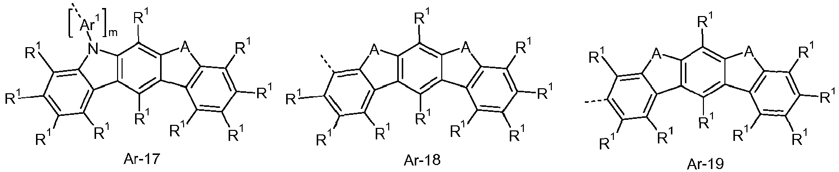 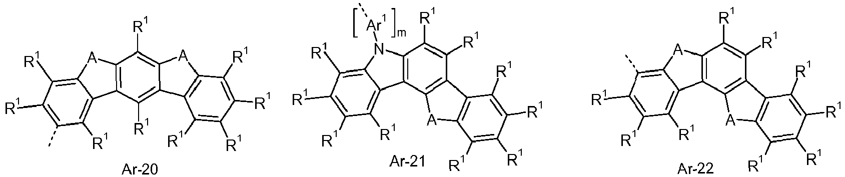 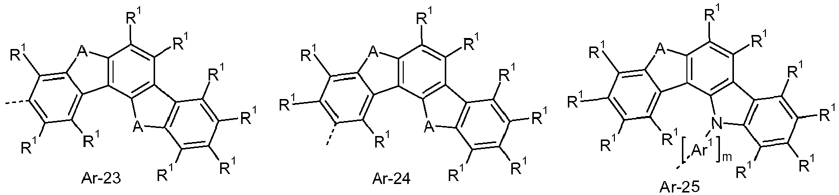 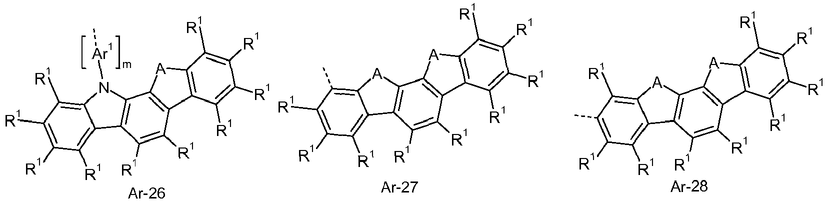 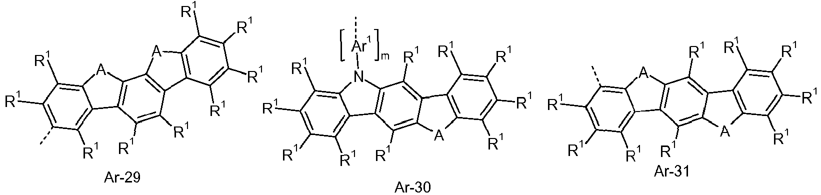 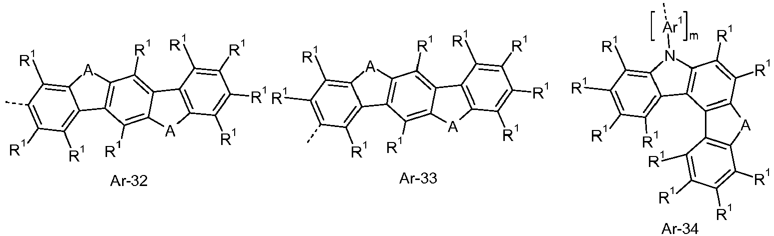 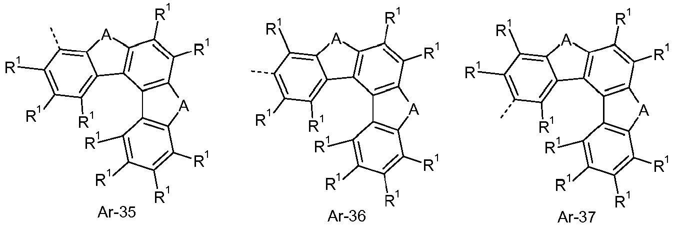 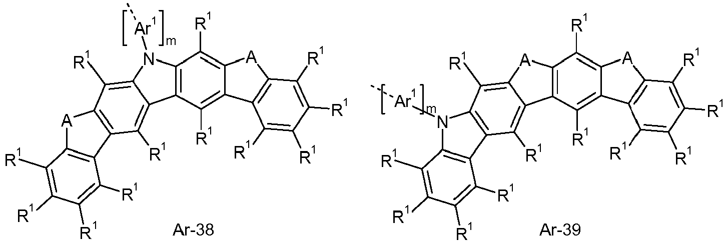 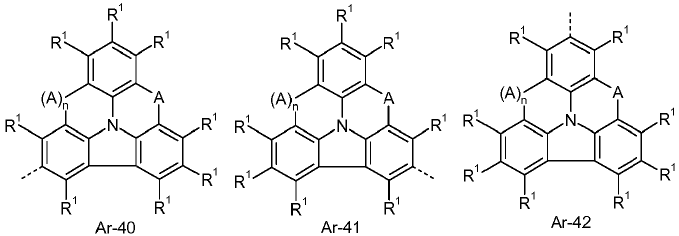 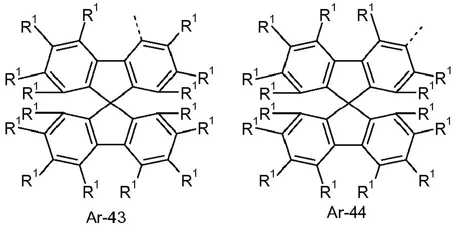 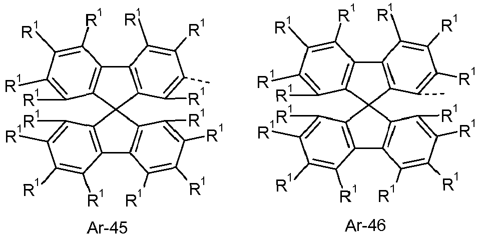 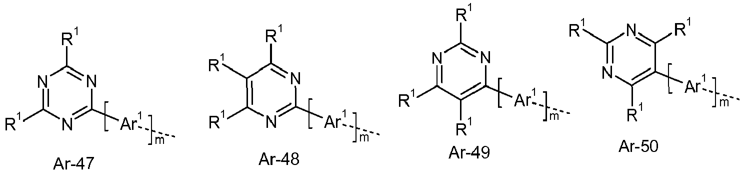 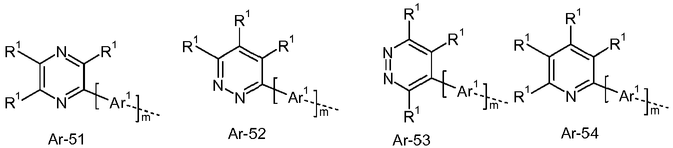 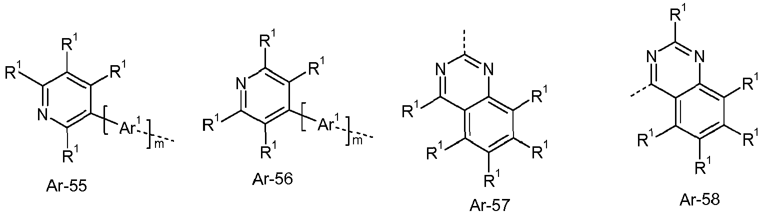 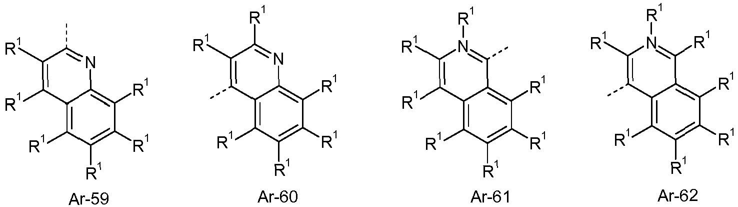 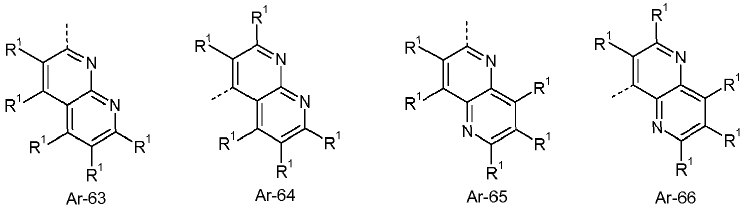 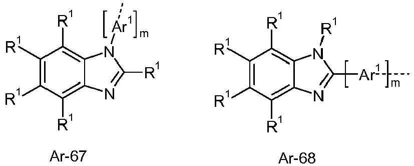 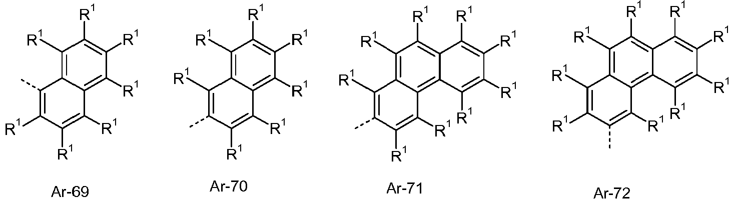 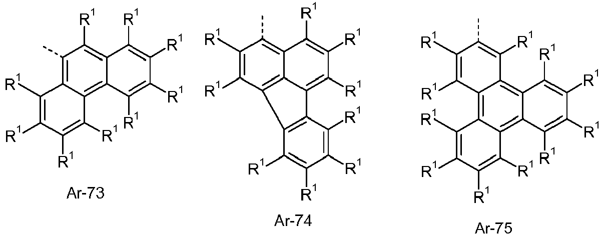 wobei R¹ die oben genannten Bedeutungen aufweist, die gestrichelte Bindung die Bindung an Y bzw. an ein Kohlenstoffatom des Grundgerüsts in Formel (1) bzw. in den bevorzugten Ausführungsformen bzw. an das Stickstoffatom in der Gruppe NAr₂ darstellt und weiterhin gilt:
- Ar¹: ist bei jedem Auftreten gleich oder verschieden ein bivalentes aromatisches oder heteroaromatisches Ringsystem mit 6 bis 18 aromatischen Ringatomen, welches jeweils mit einem oder mehreren Resten R substituiert sein kann;
- A: ist bei jedem Auftreten gleich oder verschieden C(R¹)₂, NR¹, O oder S;
- n: ist 0 oder 1, wobei n = 0 bedeutet, dass an dieser Position keine Gruppe A gebunden ist und an den entsprechenden Kohlenstoffatomen statt dessen Reste R¹ gebunden sind;
- m: ist 0 oder 1, wobei m = 0 bedeutet, dass die Gruppe Ar¹ nicht vorhanden ist und dass die entsprechende aromatische bzw. heteroaromatische Gruppe direkt an Y bzw. ein Kohlenstoffatom des Grundgerüsts in Formel (1) bzw. in den bevorzugten Ausführungsformen bzw. an das Stickstoffatom in der Gruppe NAr₂ gebunden ist; mit der Maßgabe, dass m = 1 ist für die Strukturen (Ar-12), (Ar-17), (Ar-21), (Ar-25), (Ar-26), (Ar-30), (Ar-34), (Ar-38) und (Ar-39), wenn es sich bei diesen Gruppen um Ausführungsformen von R' oder Ar handelt.

Wenn die oben genannten Gruppen für R, R' bzw. Ar mehrere Gruppen A aufweisen, so kommen hierfür alle Kombinationen aus der Definition von A in Frage. Bevorzugte Ausführungsformen sind dann solche, in denen eine Gruppe A für NR¹ und die andere Gruppe A für C(R¹)₂ steht oder in denen beide Gruppen A für NR¹ stehen oder in denen beide Gruppen A für O stehen. In einer besonders bevorzugten Ausführungsform der Erfindung steht in Gruppen R, R' bzw. Ar, die mehrere Gruppen A aufweisen, mindestens eine Gruppe A für C(R¹)₂ oder für NR¹.

Wenn A für NR¹ steht, steht der Substituent R¹, der an das Stickstoffatom gebunden ist, bevorzugt für ein aromatisches oder heteroaromatisches Ringsystem mit 5 bis 24 aromatischen Ringatomen, welches auch durch einen oder mehrere Reste R² substituiert sein kann. In einer besonders bevorzugten Ausführungsform steht dieser Substituent R¹ gleich oder verschieden bei jedem Auftreten für ein aromatisches oder heteroaromatisches Ringsystem mit 6 bis 24 aromatischen Ringatomen, welches keine kondensierten Arylgruppen aufweist und welches keine kondensierten Heteroarylgruppen, in denen zwei oder mehr aromatische bzw. heteroaromatische 6-Ring-Gruppen direkt aneinander ankondensiert sind, aufweist, und welches jeweils auch durch einen oder mehrere Reste R² substituiert sein kann. Besonders bevorzugt sind Phenyl, Biphenyl, Terphenyl und Quaterphenyl mit Verknüpfungsmustern, wie vorne für Ar-1 bis Ar-11 aufgeführt, wobei diese Strukturen durch einen oder mehrere Reste R² substituiert sein können, bevorzugt aber unsubstituiert sind.

Wenn A für C(R¹)₂ steht, stehen die Substituenten R¹, die an dieses Kohlenstoffatom gebunden sind, bevorzugt gleich oder verschieden bei jedem Auftreten für eine lineare Alkylgruppe mit 1 bis 10 C-Atomen oder für eine verzweigte oder cyclische Alkylgruppe mit 3 bis 10 C-Atomen oder für ein aromatisches oder heteroaromatisches Ringsystem mit 5 bis 24 aromatischen Ringatomen, welches auch durch einen oder mehrere Reste R² substituiert sein kann. Ganz besonders bevorzugt steht R¹ für eine Methylgruppe oder für eine Phenylgruppe. Dabei können die Reste R¹ auch miteinander ein Ringsystem bilden, was zu einem Spirosystem führt.

Weitere geeignete Gruppen R, R' bzw. Ar sind Gruppen der Formel -Ar⁴-N(Ar²)(Ar³), wobei Ar², Ar³ und Ar⁴ gleich oder verschieden bei jedem Auftreten für ein aromatisches oder heteroaromatisches Ringsystem mit 5 bis 24 aromatischen Ringatomen stehen, welches jeweils mit einem oder mehreren Resten R¹ substituiert sein kann. Dabei beträgt die Gesamtzahl der aromatischen Ringatome von Ar², Ar³ und Ar⁴ maximal 60 und bevorzugt maximal 40.

Dabei können Ar⁴ und Ar² miteinander und/oder Ar² und Ar³ miteinander auch durch eine Gruppe ausgewählt aus C(R¹)₂, NR¹, O oder S verbunden sein. Bevorzugt erfolgt die Verknüpfung von Ar⁴ und Ar² miteinander bzw. von Ar² und Ar³ miteinander jeweils ortho zur Position der Verknüpfung mit dem Stickstoffatom. In einer weiteren Ausführungsform der Erfindung sind keine der Gruppen Ar², Ar³ bzw. Ar⁴ miteinander verbunden.

Bevorzugt ist Ar⁴ ein aromatisches oder heteroaromatisches Ringsystem mit 6 bis 24 aromatischen Ringatomen, bevorzugt mit 6 bis 12 aromatischen Ringatomen, das jeweils mit einem oder mehreren Resten R¹ substituiert sein kann. Besonders bevorzugt ist Ar⁴ ausgewählt aus der Gruppe bestehend aus ortho-, meta- oder para-Phenylen oder ortho-, meta- oder para-Biphenyl, welche jeweils durch einen oder mehrere Reste R¹ substituiert sein können, bevorzugt aber unsubstituiert sind. Ganz besonders bevorzugt ist Ar⁴ eine unsubstituierte Phenylengruppe. Dies gilt insbesondere, wenn Ar⁴ mit Ar² durch einen Einfachbindung verbunden ist.

Bevorzugt sind Ar² und Ar³ gleich oder verschieden bei jedem Auftreten ein aromatisches oder heteroaromatisches Ringsystem mit 6 bis 24 aromatischen Ringatomen, das jeweils mit einem oder mehreren Resten R¹ substituiert sein kann. Besonders bevorzugte Gruppen Ar² bzw. Ar³ sind gleich oder verschieden bei jedem Auftreten ausgewählt aus der Gruppe bestehend aus Benzol, ortho-, meta- oder para-Biphenyl, ortho-, meta-, para- oder verzweigtem Terphenyl, ortho-, meta-, para- oder verzweigtem Quaterphenyl, 1-, 2-, 3- oder 4-Fluorenyl, 1-, 2-, 3- oder 4-Spirobifluorenyl, 1-oder 2-Naphthyl, Indol, Benzofuran, Benzothiophen, 1-, 2-, 3- oder 4-Carbazol, 1-, 2-, 3- oder 4-Dibenzofuran, 1-, 2-, 3- oder 4-Dibenzothiophen, Indenocarbazol, Indolocarbazol, 2-, 3- oder 4-Pyridin, 2-, 4- oder 5-Pyrimidin, Pyrazin, Pyridazin, Triazin, Phenanthren, Triphenylen oder Kombinationen aus zwei, drei oder vier dieser Gruppen, welche jeweils mit einem oder mehreren Resten R¹ substituiert sein können. Besonders bevorzugt stehen Ar² und Ar³ gleich oder verschieden bei jedem Auftreten für ein aromatisches Ringsystem mit 6 bis 24 aromatischen Ringatomen, welches durch einen oder mehrere Reste R¹ substituiert sein kann, insbesondere ausgewählt aus den Gruppen bestehend aus Benzol, Biphenyl, insbesondere ortho-, meta- oder para-Biphenyl, Terphenyl, insbesondere ortho-, meta-, para- oder verzweigtem Terphenyl, Quaterphenyl, insbesondere ortho-, meta-, para- oder verzweigtem Quaterphenyl, Fluoren, insbesondere 1-, 2-, 3- oder 4-Fluoren, oder Spirobifluoren, insbesondere 1-, 2-, 3- oder 4-Spirobifluoren.

Dabei haben in erfindungsgemäßen Verbindungen, die durch Vakuumverdampfung verarbeitet werden, die Alkylgruppen bevorzugt nicht mehr als fünf C-Atome, besonders bevorzugt nicht mehr als 4 C-Atome, ganz besonders bevorzugt nicht mehr als 1 C-Atom. Für Verbindungen, die aus Lösung verarbeitet werden, eignen sich auch Verbindungen, die mit Alkylgruppen, insbesondere verzweigten Alkylgruppen, mit bis zu 10 C-Atomen substituiert sind oder die mit Oligoarylengruppen, beispielsweise ortho-, meta-, para- oder verzweigten Terphenyl- oder Quaterphenylgruppen, substituiert sind.

Wenn die Verbindungen der Formel (1) bzw. die bevorzugten Ausführungsformen als Matrixmaterial für einen phosphoreszierenden Emitter oder in einer Schicht, die direkt an eine phosphoreszierende Schicht angrenzt, verwendet werden, ist es weiterhin bevorzugt, wenn die Verbindung keine kondensierten Aryl- bzw. Heteroarylgruppen enthält, in denen mehr als zwei Sechsringe direkt aneinander ankondensiert sind. Insbesondere ist es bevorzugt, dass die Reste R, R', Ar, R¹ und R² keine kondensierten Aryl- bzw. Heteroarylgruppen enthalten, in denen zwei oder mehr Sechsringe direkt aneinander ankondensiert sind. Eine Ausnahme hiervon bilden Phenanthren und Triphenylen, die aufgrund ihrer hohen Triplettenergie trotz der Anwesenheit kondensierter aromatischer Sechsringe bevorzugt sein können.

Die oben genannten bevorzugten Ausführungsformen können beliebig innerhalb der in Anspruch 1 definierten Einschränkungen miteinander kombiniert werden. In einer besonders bevorzugten Ausführungsform der Erfindung treten die oben genannten Bevorzugungen gleichzeitig auf.

Beispiele für bevorzugte Verbindungen gemäß den oben aufgeführten Ausführungsformen sind die in der folgenden Tabelle aufgeführten Verbindungen.

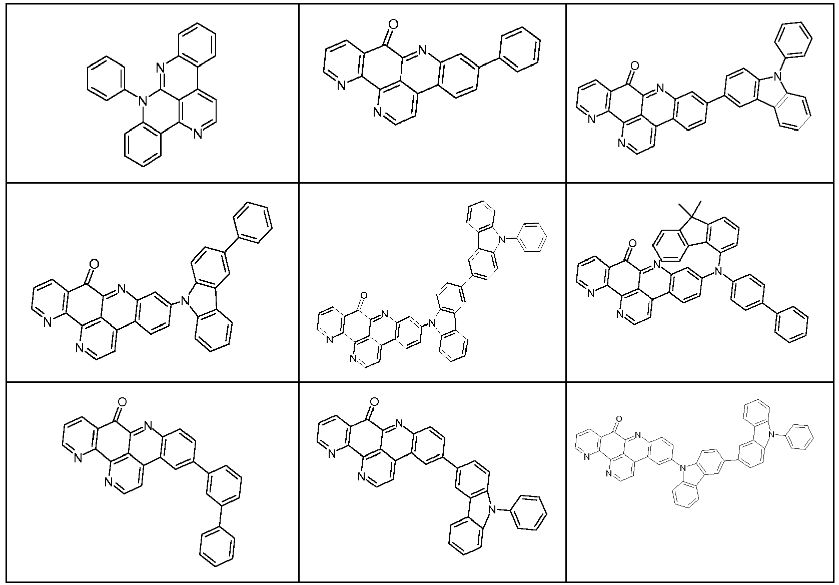

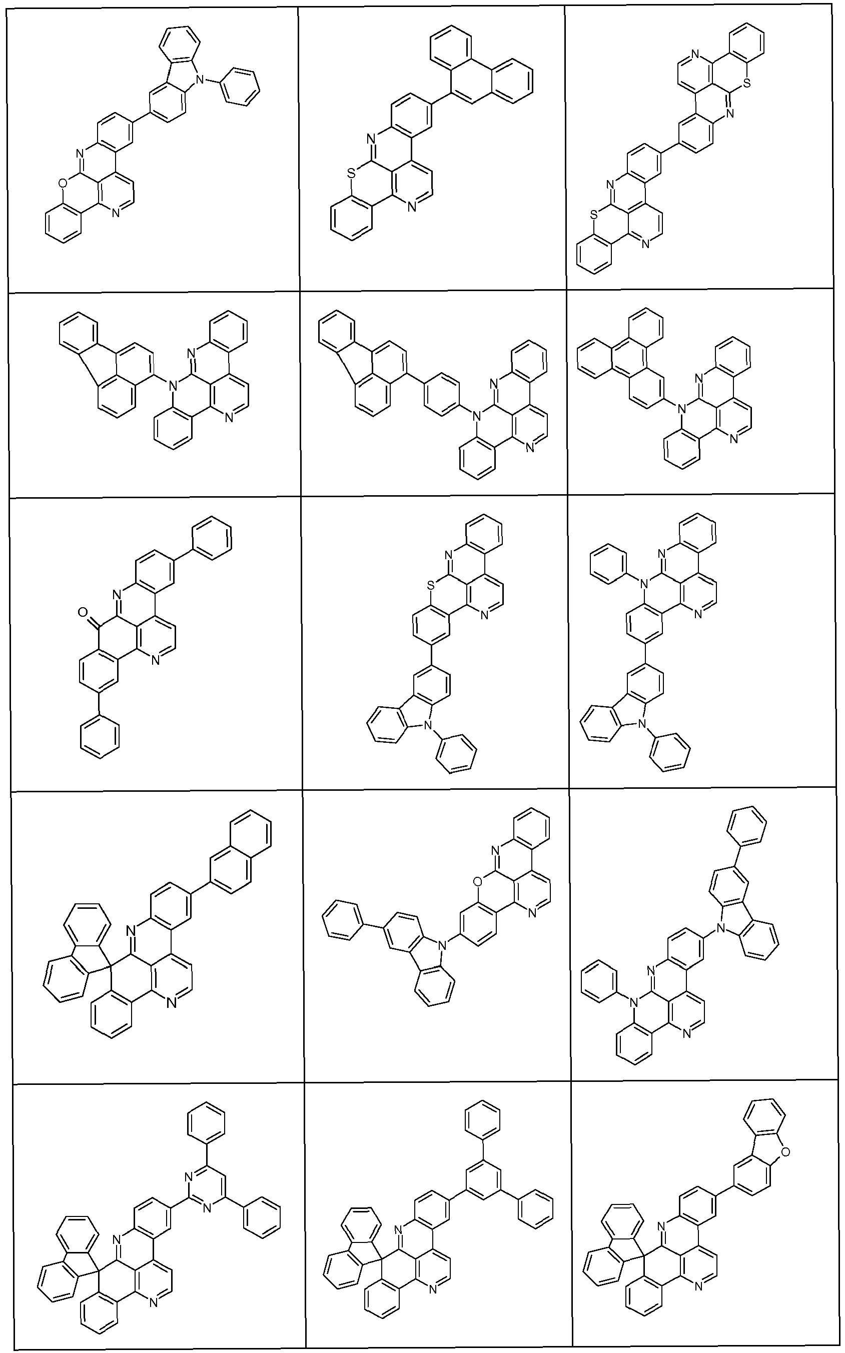

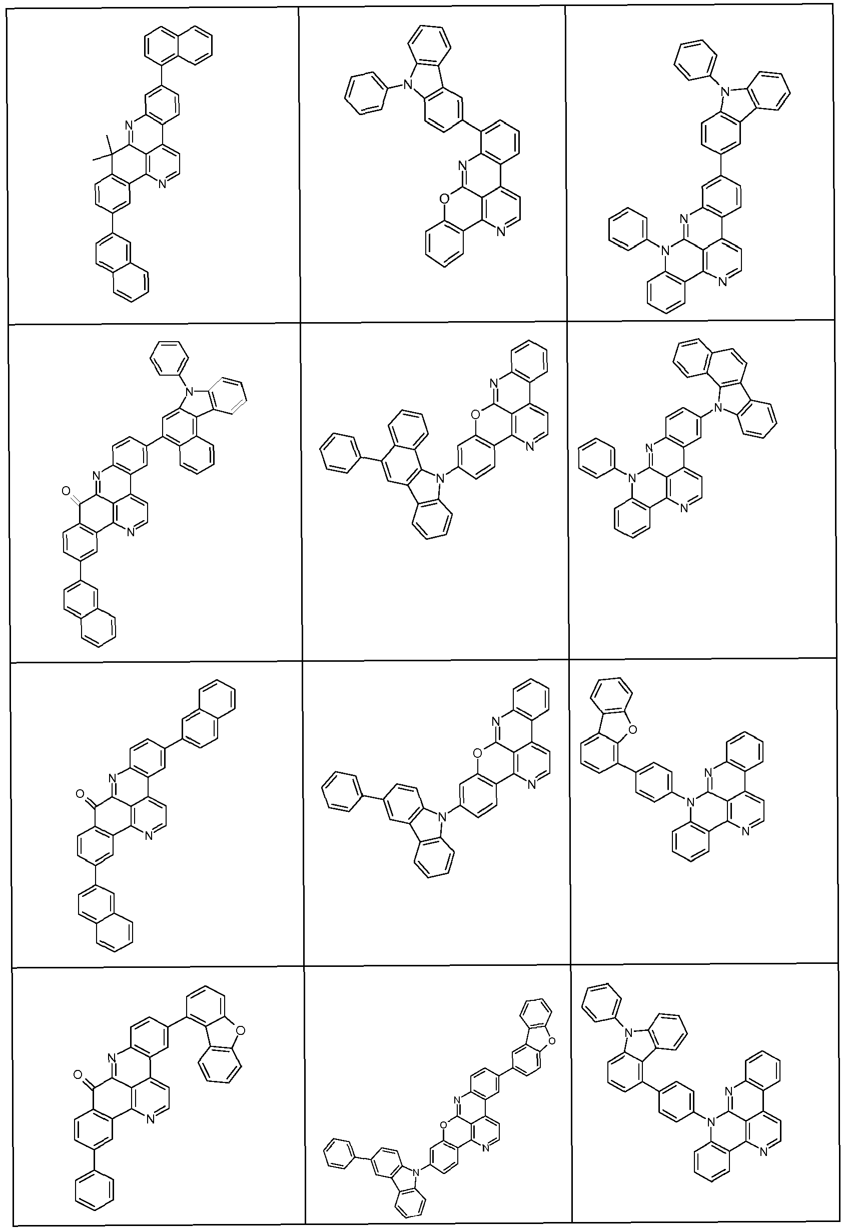

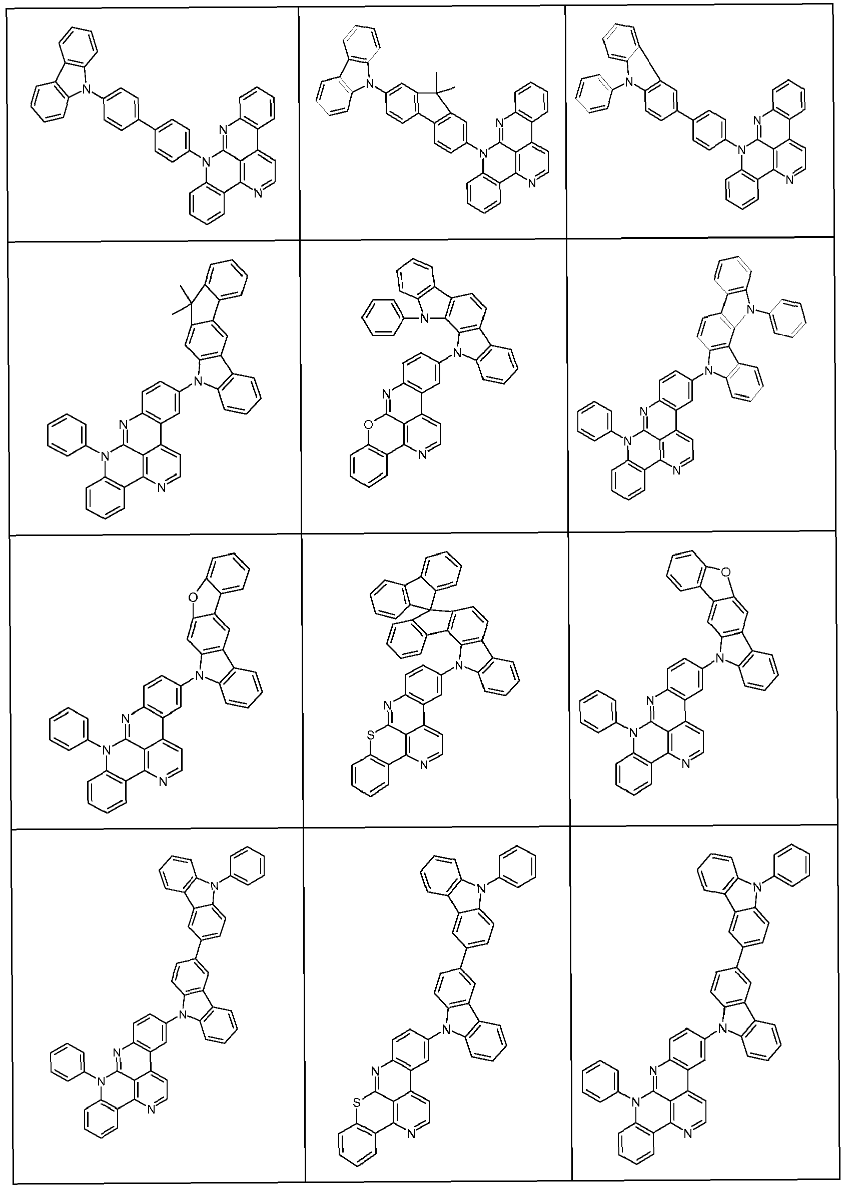

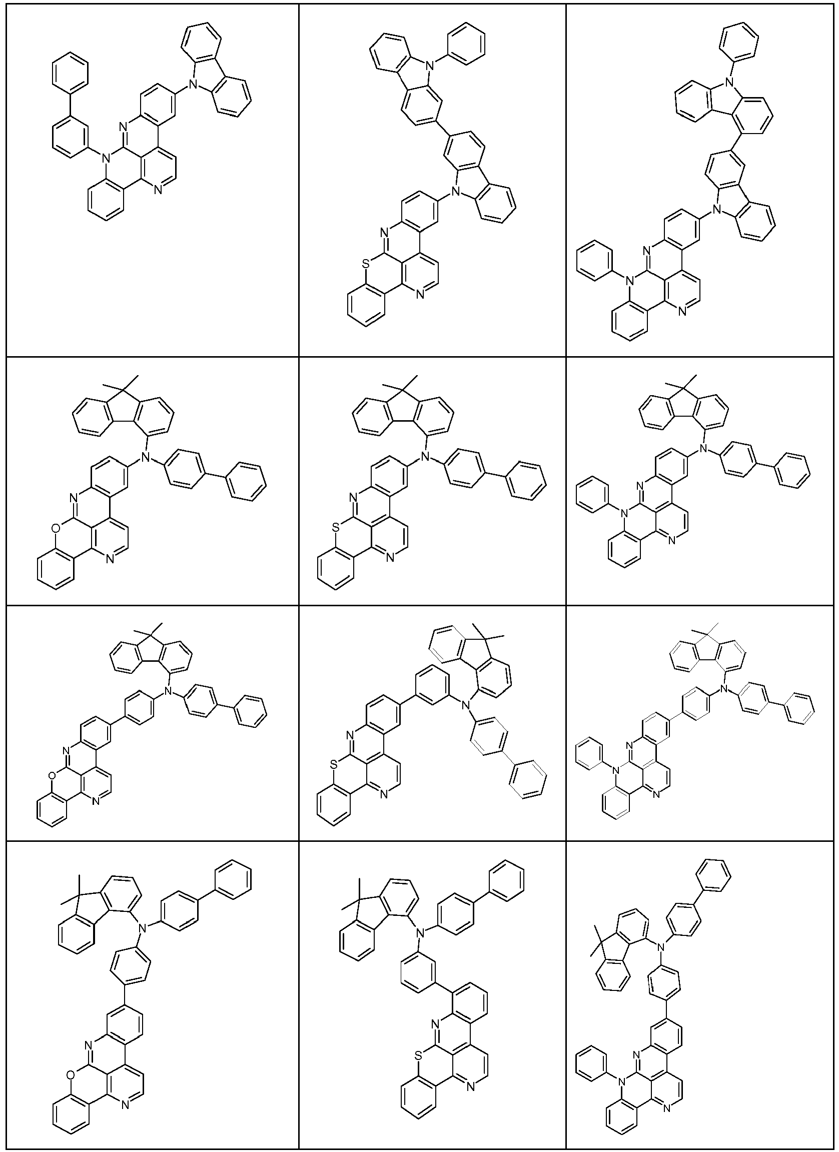

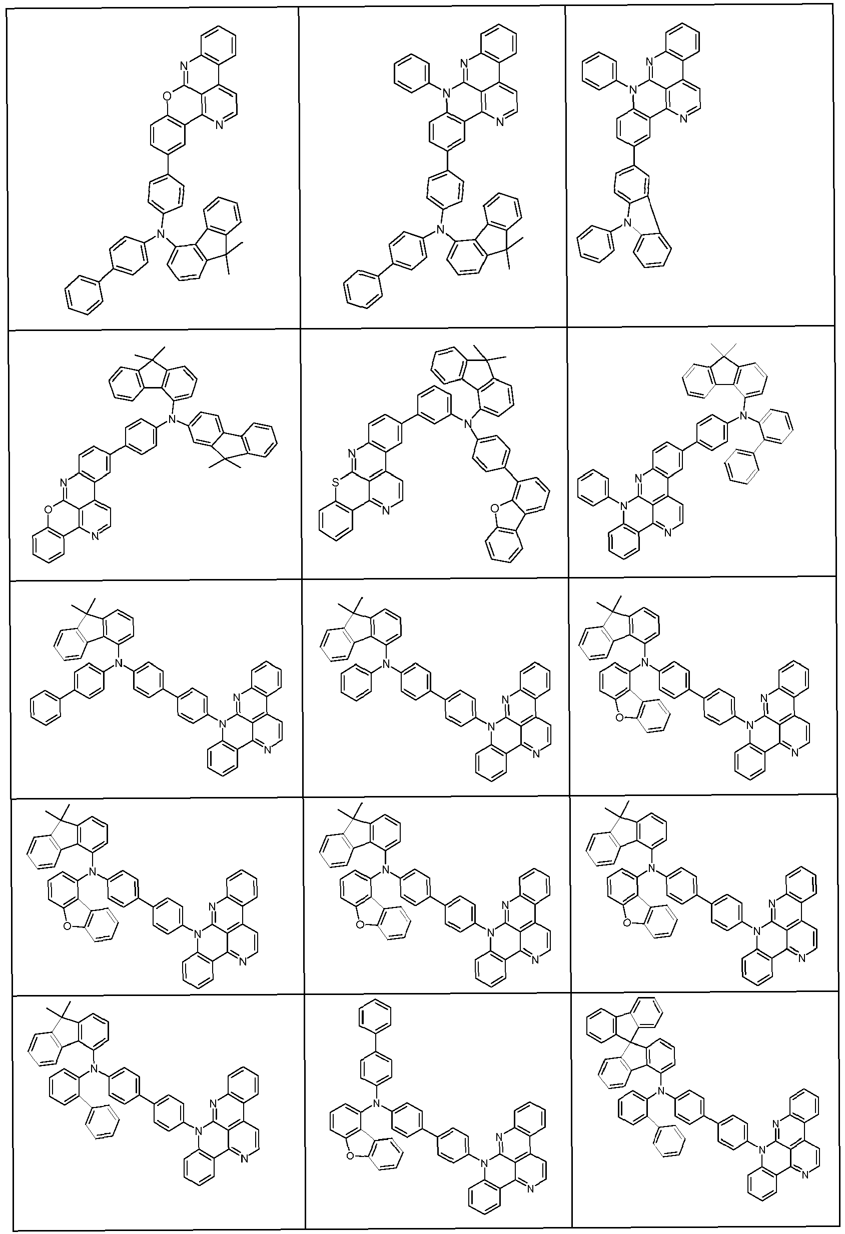

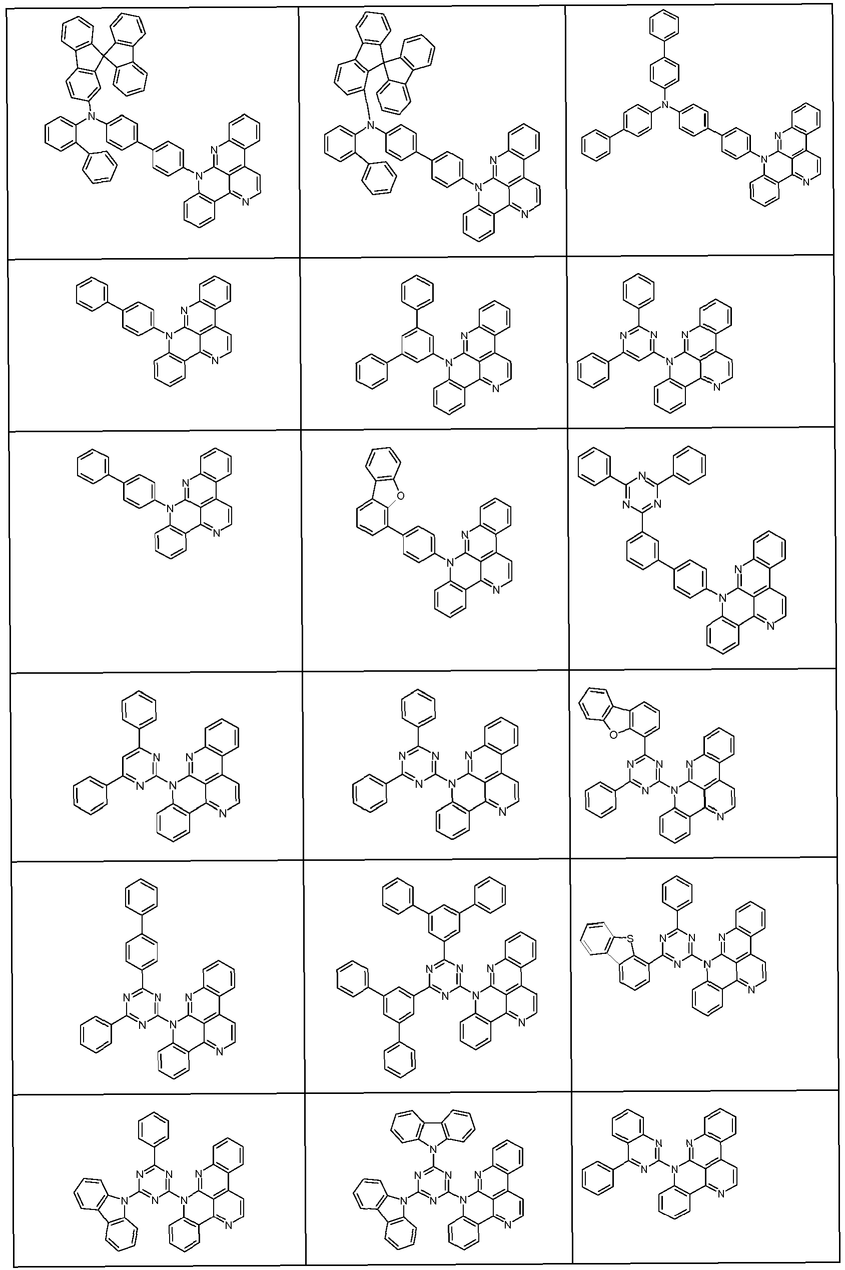

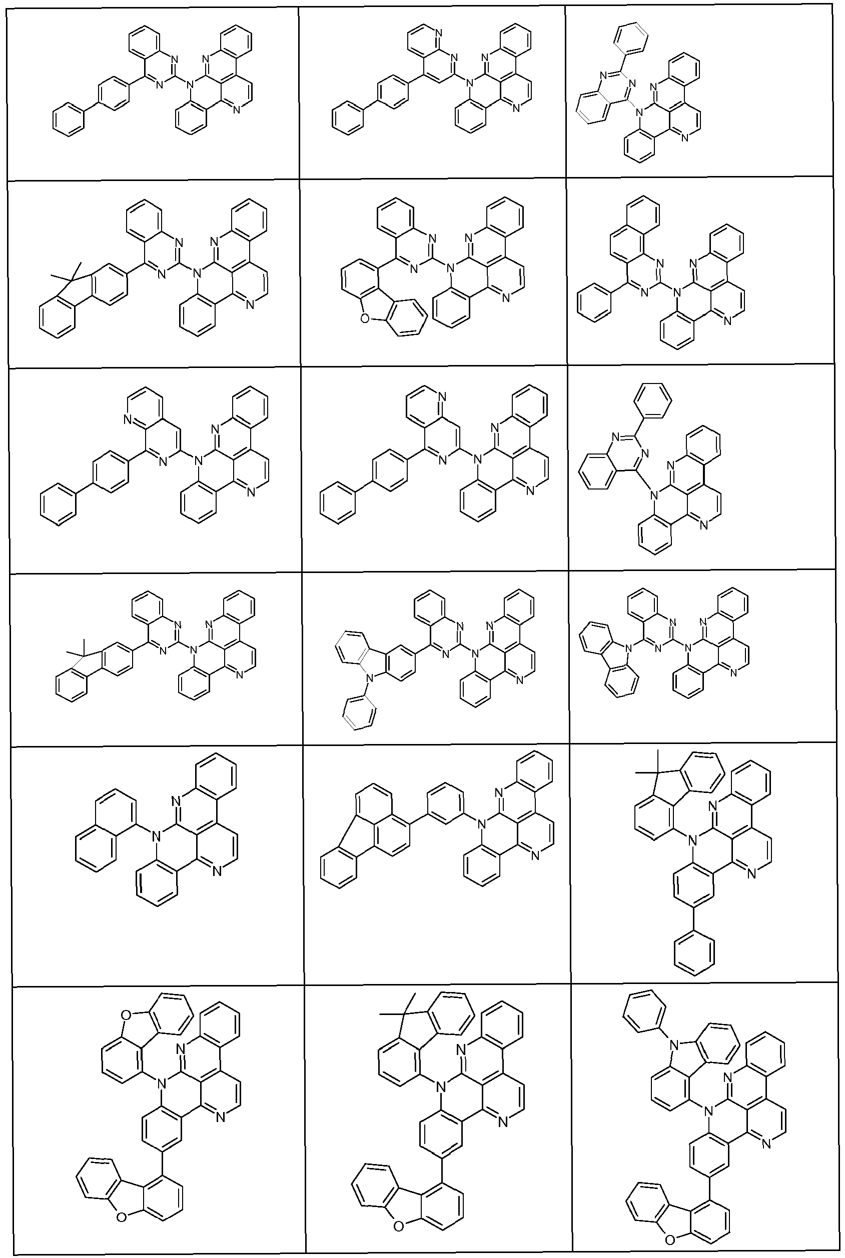

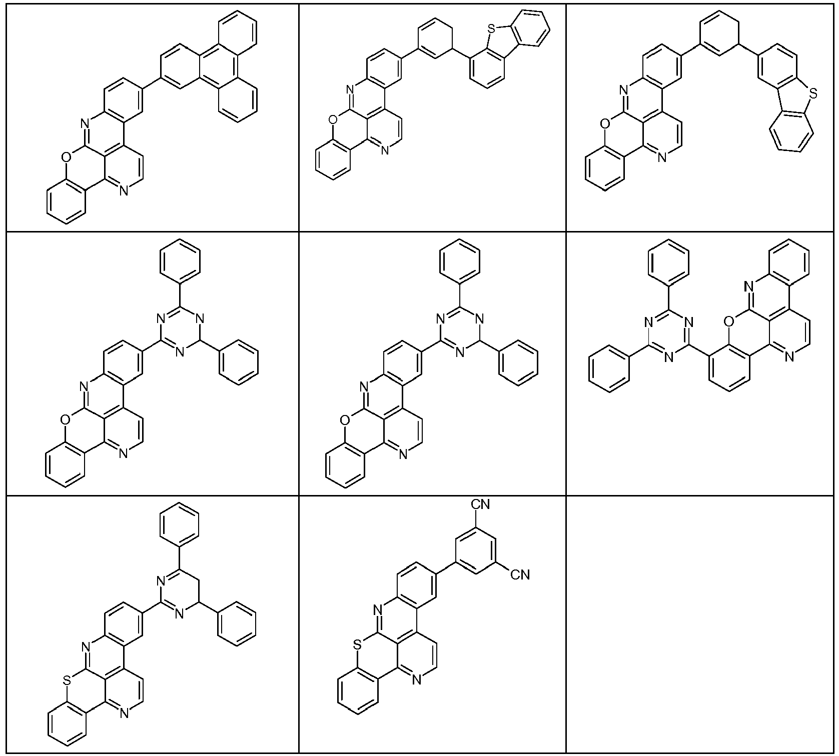

Die Grundstruktur der erfindungsgemäßen Verbindungen kann nach den in Schema 1, 3 und 4 skizzierten Wegen analog zu der Dissertation "Entwicklung neuer Synthesewege zu Pyridoacridinen" (Stephan Rieder, Ludwig-Maximilian-Universität München, 2012) bzw. nach dem in Schema 2 skizzierten Weg analog zu Tetrahedron Letters 2013, 54, 2014-2017 dargestellt werden.

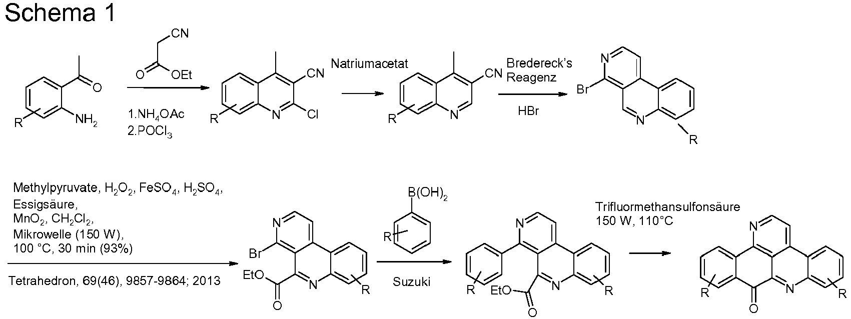

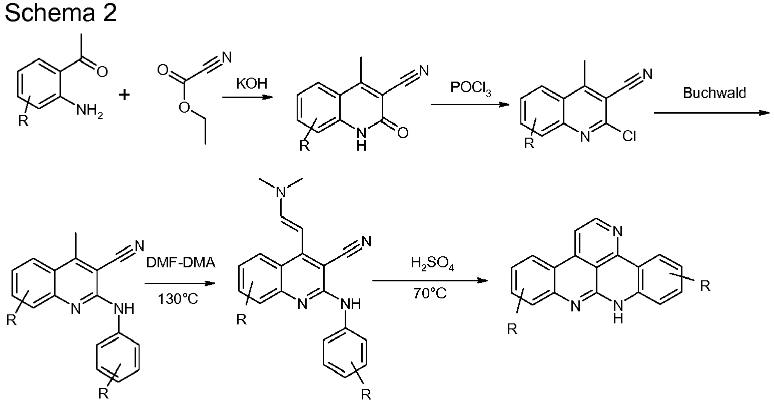

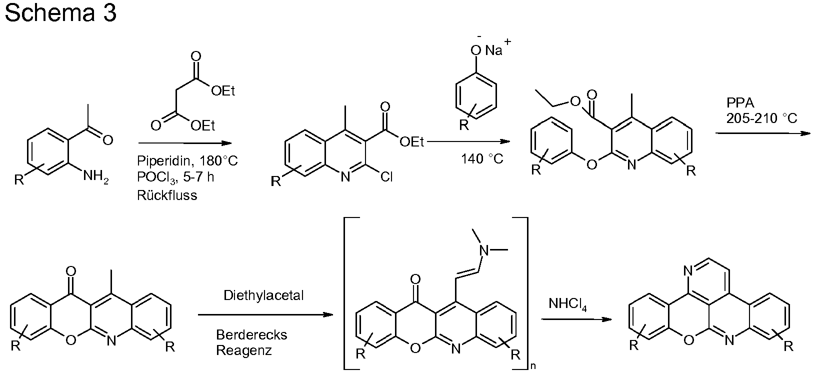

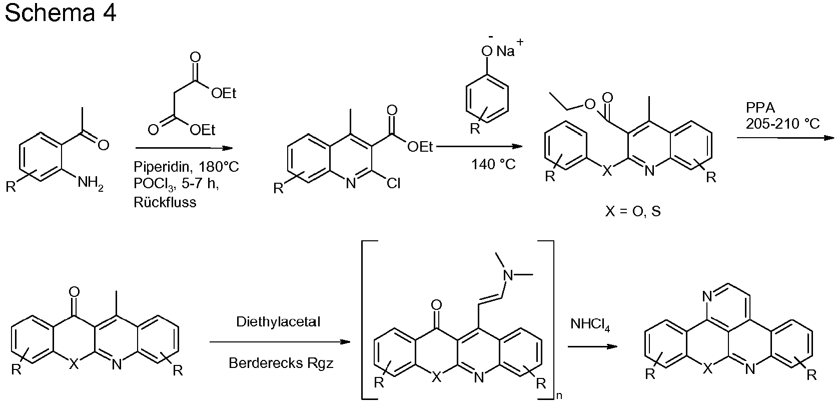

Für die Verarbeitung der erfindungsgemäßen Verbindungen aus flüssiger Phase, beispielsweise durch Spin-Coating oder durch Druckverfahren, sind Formulierungen der erfindungsgemäßen Verbindungen erforderlich. Diese Formulierungen können beispielsweise Lösungen, Dispersionen oder Emulsionen sein. Es kann bevorzugt sein, hierfür Mischungen aus zwei oder mehr Lösemitteln zu verwenden. Geeignete und bevorzugte Lösemittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-Tetramethylbenzol, 1-Methylnaphthalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, α-Terpineol, Benzothiazol, Butylbenzoat, Cumol, Cyclohexanol, Cyclohexanon, Cyclohexylbenzol, Decalin, Dodecylbenzol, Ethylbenzoat, Indan, NMP, p-Cymol, Phenetol, 1,4-Diisopropylbenzol, Dibenzylether, Diethylenglycolbutylmethylether, Triethylenglycolbutylmethylether, Diethylenglycoldibutylether, Triethylenglycoldimethylether, Diethylenglycolmonobutylether, Tripropyleneglycoldimethylether, Tetraethylenglycoldimethylether, 2-Isopropylnaphthalin, Pentylbenzol, Hexylbenzol, Heptylbenzol, Octylbenzol, 1,1-Bis(3,4-dimethylphenyl)ethan, 2-Methylbiphenyl, 3-Methylbiphenyl, 1-Methylnaphthalin, 1-Ethylnaphthalin, Ethyloctanoat, Sebacinsäure-diethylester, Octyloctanoat, Heptylbenzol, Menthylisovalerat, Cyclohexylhexanoat oder Mischungen dieser Lösemittel.

Ein weiterer Gegenstand der vorliegenden Erfindung ist daher eine Formulierung, enthaltend eine erfindungsgemäße Verbindung und mindestens eine weitere Verbindung. Die weitere Verbindung kann beispielsweise ein Lösemittel sein, insbesondere eines der oben genannten Lösemittel oder eine Mischung dieser Lösemittel. Die weitere Verbindung kann aber auch mindestens eine weitere organische oder anorganische Verbindung sein, die ebenfalls in der elektronischen Vorrichtung eingesetzt wird, beispielsweise eine emittierende Verbindung und/oder ein weiteres Matrixmaterial. Geeignete emittierende Verbindungen und weitere Matrixmaterialien sind hinten im Zusammenhang mit der organischen Elektrolumineszenzvorrichtung aufgeführt. Diese weitere Verbindung kann auch polymer sein.

Die erfindungsgemäßen Verbindungen eignen sich für die Verwendung in einer elektronischen Vorrichtung, insbesondere in einer organischen Elektrolumineszenzvorrichtung.

Ein weiterer Gegenstand der vorliegenden Erfindung ist daher die Verwendung einer erfindungsgemäßen Verbindung in einer elektronischen Vorrichtung, insbesondere in einer organischen Elektrolumineszenzvorrichtung.

Ein nochmals weiterer Gegenstand der vorliegenden Erfindung ist eine elektronische Vorrichtung enthaltend mindestens eine erfindungsgemäße Verbindung.

Eine elektronische Vorrichtung im Sinne der vorliegenden Erfindung ist eine Vorrichtung, welche mindestens eine Schicht enthält, die mindestens eine organische Verbindung enthält. Das Bauteil kann dabei auch anorganische Materialien enthalten oder auch Schichten, welche vollständig aus anorganischen Materialien aufgebaut sind.

Die elektronische Vorrichtung ist bevorzugt ausgewählt aus der Gruppe bestehend aus organischen Elektrolumineszenzvorrichtungen (OLEDs), organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), farbstoffsensibilisierten organischen Solarzellen (DSSCs), organischen optischen Detektoren, organischen Photorezeptoren, organischen Feld-Quench-Devices (O-FQDs), lichtemittierenden elektrochemischen Zellen (LECs), organischen Laserdioden (O-Laser) und "organic plasmon emitting devices", bevorzugt aber organischen Elektrolumineszenzvorrichtungen (OLEDs), besonders bevorzugt phosphoreszierenden OLEDs.

Die organische Elektrolumineszenzvorrichtung enthält Kathode, Anode und mindestens eine emittierende Schicht. Außer diesen Schichten kann sie noch weitere Schichten enthalten, beispielsweise jeweils eine oder mehrere Lochinjektionsschichten, Lochtransportschichten, Lochblockierschichten, Elektronentransportschichten, Elektroneninjektionsschichten, Exzitonenblockierschichten, Elektronenblockierschichten und/oder Ladungserzeugungsschichten (Charge-Generation Layers). Ebenso können zwischen zwei emittierende Schichten Interlayer eingebracht sein, welche beispielsweise eine exzitonenblockierende Funktion aufweisen. Es sei aber darauf hingewiesen, dass nicht notwendigerweise jede dieser Schichten vorhanden sein muss. Dabei kann die organische Elektrolumineszenzvorrichtung eine emittierende Schicht enthalten, oder sie kann mehrere emittierende Schichten enthalten. Wenn mehrere Emissionsschichten vorhanden sind, weisen diese bevorzugt insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können. Insbesondere bevorzugt sind Systeme mit drei emittierenden Schichten, wobei die drei Schichten blaue, grüne und orange oder rote Emission zeigen. Es kann sich bei der erfindungsgemäßen organischen Elektrolumineszenzvorrichtung auch um eine Tandem-OLED handeln, insbesondere für weiß emittierende OLEDs.

Die erfindungsgemäße Verbindung gemäß den oben aufgeführten Ausführungsformen kann dabei in unterschiedlichen Schichten eingesetzt werden, je nach genauer Struktur. Bevorzugt ist eine organische Elektrolumineszenzvorrichtung, enthaltend eine Verbindung gemäß Formel (1) bzw. die oben ausgeführten bevorzugten Ausführungsformen in einer emittierenden Schicht als Matrixmaterial für phosphoreszierende Emitter oder für Emitter, die TADF (thermally activated delayed fluorescence) zeigen, insbesondere für phosphoreszierende Emitter. Dabei kann die organische Elektrolumineszenzvorrichtung eine emittierende Schicht enthalten, oder sie kann mehrere emittierende Schichten enthalten, wobei mindestens eine emittierende Schicht mindestens eine erfindungsgemäße Verbindung als Matrixmaterial enthält. Weiterhin kann die erfindungsgemäße Verbindung auch in einer Elektronentransportschicht und/oder in einer Lochtransportschicht und/oder in einer Exzitonenblockierschicht und/oder in einer Lochblockierschicht eingesetzt werden. In einer bevorzugten Ausführungsform der Erfindung wird die erfindungsgemäße Verbindung als Matrixmaterial für rot phosphoreszierende Emitter und/oder als Elektronentransportmaterial in einer Elektronentransportschicht oder eine Lochblockierschicht eingesetzt.

Wenn die erfindungsgemäße Verbindung als Matrixmaterial für eine phosphoreszierende Verbindung in einer emittierenden Schicht eingesetzt wird, wird sie bevorzugt in Kombination mit einem oder mehreren phosphoreszierenden Materialien (Triplettemitter) eingesetzt. Unter Phosphoreszenz im Sinne dieser Erfindung wird die Lumineszenz aus einem angeregten Zustand mit höherer Spinmultiplizität verstanden, also einem Spinzustand > 1, insbesondere aus einem angeregten Triplettzustand. Im Sinne dieser Anmeldung sollen alle lumineszierenden Komplexe mit Übergangsmetallen oder Lanthaniden, insbesondere alle Iridium-, Platin- und Kupferkomplexe als phosphoreszierende Verbindungen angesehen werden.

Die Mischung aus der erfindungsgemäßen Verbindung und der emittierenden Verbindung enthält zwischen 99 und 1 Vol.-%, vorzugsweise zwischen 98 und 10 Vol.-%, besonders bevorzugt zwischen 97 und 60 Vol.-%, insbesondere zwischen 95 und 80 Vol.-% der erfindungsgemäßen Verbindung bezogen auf die Gesamtmischung aus Emitter und Matrixmaterial. Entsprechend enthält die Mischung zwischen 1 und 99 Vol.-%, vorzugsweise zwischen 2 und 90 Vol.-%, besonders bevorzugt zwischen 3 und 40 Vol.-%, insbesondere zwischen 5 und 20 Vol.-% des Emitters bezogen auf die Gesamtmischung aus Emitter und Matrixmaterial.

Eine weitere bevorzugte Ausführungsform der vorliegenden Erfindung ist der Einsatz der erfindungsgemäßen Verbindung als Matrixmaterial für einen phosphoreszierenden Emitter in Kombination mit einem weiteren Matrixmaterial. Geeignete Matrixmaterialien, welche in Kombination mit den erfindungsgemäßen Verbindungen eingesetzt werden können, sind aromatische Ketone, aromatische Phosphinoxide oder aromatische Sulfoxide oder Sulfone, z. B. gemäß WO 2004/013080, WO 2004/093207, WO 2006/005627 oder WO 2010/006680, Triarylamine, Carbazolderivate, z. B. CBP (N,N-Biscarbazolylbiphenyl) oder die in WO 2005/039246, US 2005/0069729, JP 2004/288381, EP 1205527, WO 2008/086851 oder WO 2013/041176, Indolocarbazolderivate, z. B. gemäß WO 2007/063754 oder WO 2008/056746, Indenocarbazolderivate, z. B. gemäß WO 2010/136109, WO 2011/000455, WO 2013/041176 oder WO 2013/056776, Azacarbazolderivate, z. B. gemäß EP 1617710, EP 1617711, EP 1731584, JP 2005/347160, bipolare Matrixmaterialien, z. B. gemäß WO 2007/137725, Silane, z. B. gemäß WO 2005/111172, Azaborole oder Boronester, z. B. gemäß WO 2006/117052, Triazinderivate, z. B. gemäß WO 2007/063754, WO 2008/056746, WO 2010/015306, WO 2011/057706, WO 2011/060859 oder WO 2011/060877, Zinkkomplexe, z. B. gemäß EP 652273 oder WO 2009/062578, Diazasilol- bzw. Tetraazasilol-Derivate, z. B. gemäß WO 2010/054729, Diazaphosphol-Derivate, z. B. gemäß WO 2010/054730, verbrückte Carbazol-Derivate, z. B. gemäß WO 2011/042107, WO 2011/060867, WO 2011/088877 und WO 2012/143080, Triphenylenderivate, z. B. gemäß WO 2012/048781, oder Dibenzofuranderivate, z. B. gemäß WO 2015/169412, WO 2016/015810, WO 2016/023608 oder den noch nicht offen gelegten Anmeldungen EP 16158460.2 oder EP 16159829.7. Ebenso kann ein weiterer phosphoreszierender Emitter, welcher kürzerwellig als der eigentliche Emitter emittiert, als Co-Host in der Mischung vorhanden sein oder eine Verbindung, die nicht oder nicht in wesentlichem Umfang am Ladungstransport teilnimmt, wie beispielsweise in WO 2010/108579 beschrieben.

Weiterhin eignen sich in Kombination mit der erfindungsgemäßen Verbindung als Co-Matrix-Material Verbindungen, welche eine große Bandlücke aufweisen und selber nicht oder zumindest nicht in wesentlichem Maße am Ladungstransport der emittierenden Schicht teilnehmen. Es handelt sich bei solchen Materialien bevorzugt um reine Kohlenwasserstoffe. Beispiele für solche Materialien finden sich beispielsweise in der WO 2009/124627 oder in der WO 2010/006680.

Als phosphoreszierende Verbindungen (= Triplettemitter) eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten, insbesondere ein Metall mit dieser Ordnungszahl. Bevorzugt werden als Phosphoreszenzemitter Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium oder Platin enthalten.

Beispiele der oben beschriebenen Emitter können den Anmeldungen WO 00/70655, WO 2001/41512, WO 2002/02714, WO 2002/15645, EP 1191613, EP 1191612, EP 1191614, WO 05/033244, WO 05/019373, US 2005/0258742, WO 2009/146770, WO 2010/015307, WO 2010/031485, WO 2010/054731, WO 2010/054728, WO 2010/086089, WO 2010/099852, WO 2010/102709, WO 2011/032626, WO 2011/066898, WO 2011/157339, WO 2012/007086, WO 2014/008982, WO 2014/023377, WO 2014/094961, WO 2014/094960, WO 2015/036074, WO 2015/104045, WO 2015/117718, WO 2016/015815, WO 2016/124304, WO 2017/032439 und der noch nicht offen gelegten Anmeldung EP 16179378.1 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenz bekannt sind, und der Fachmann kann ohne erfinderisches Zutun weitere phosphoreszierende Komplexe verwenden.

Explizite Beispiele für phosphoreszierende Dotanden sind in der folgenden Tabelle aufgeführt.

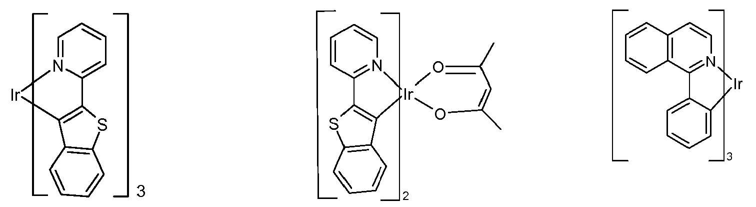

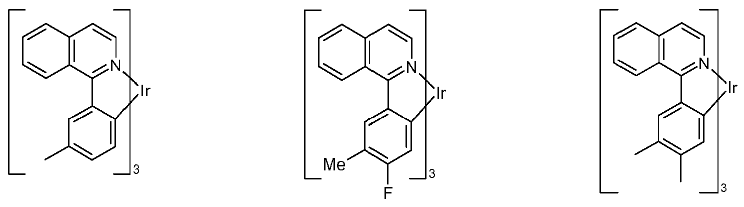

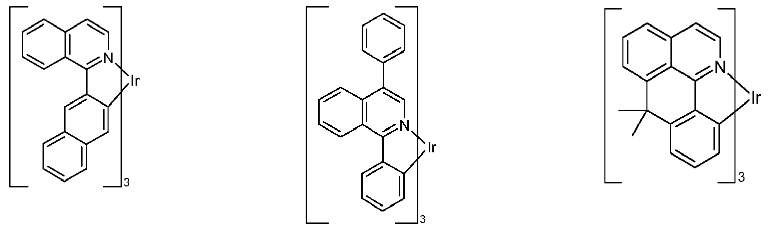

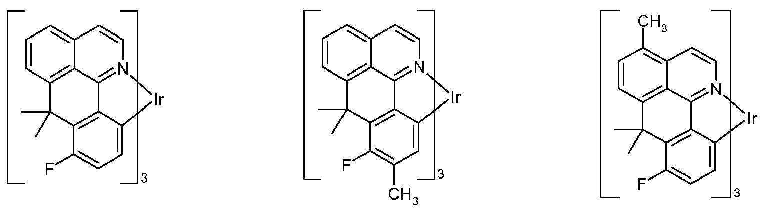

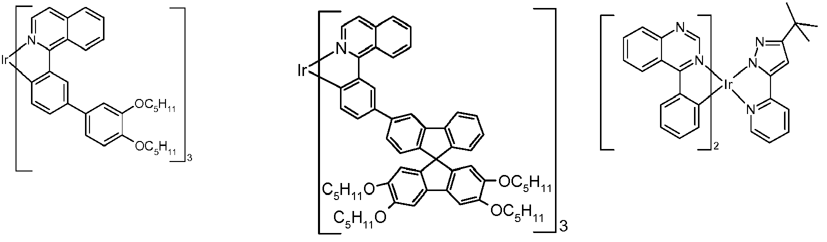

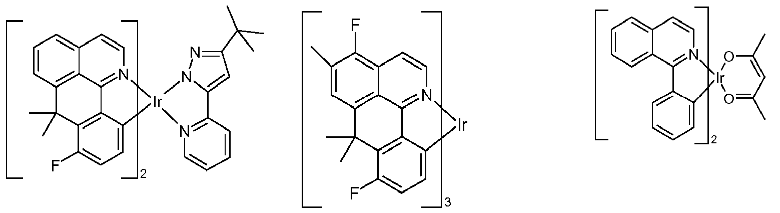

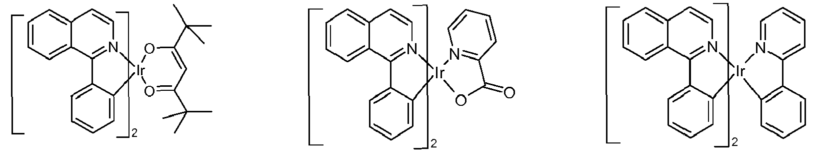

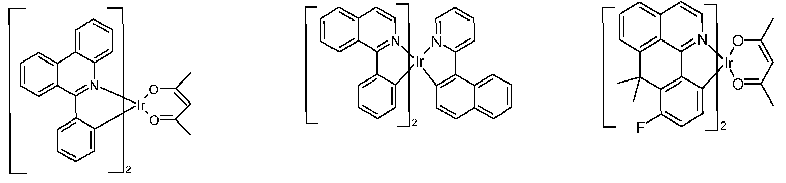

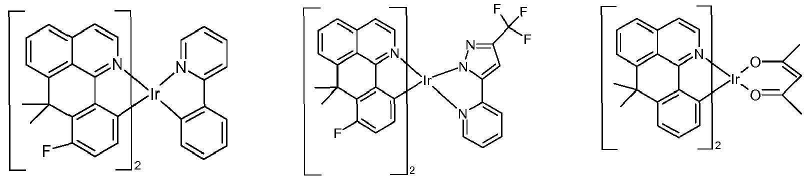

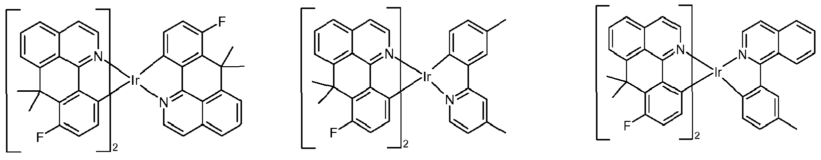

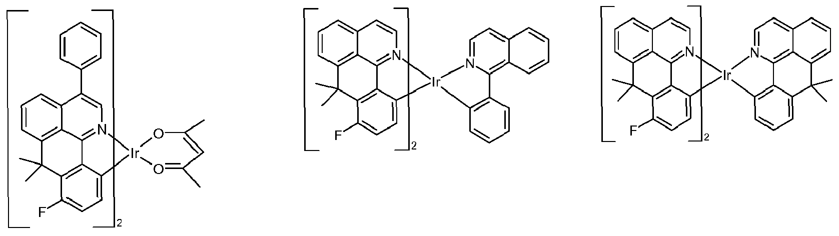

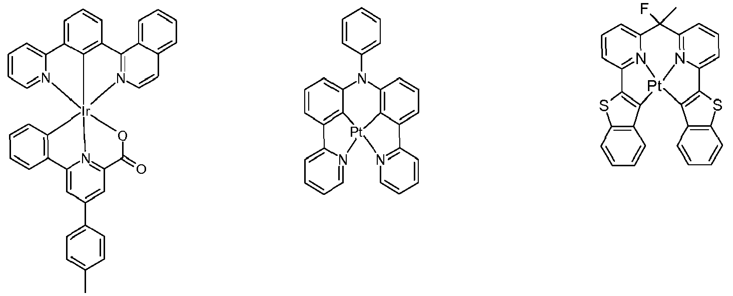

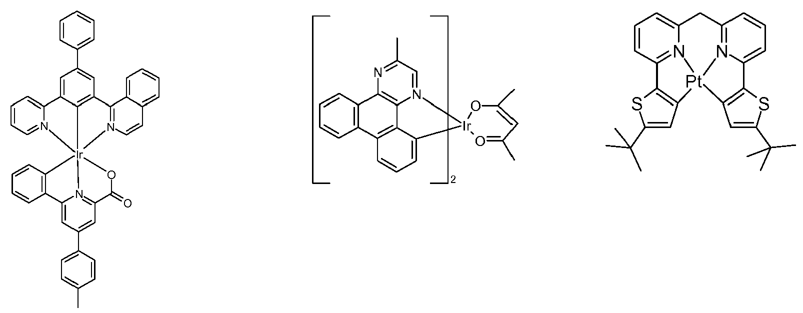

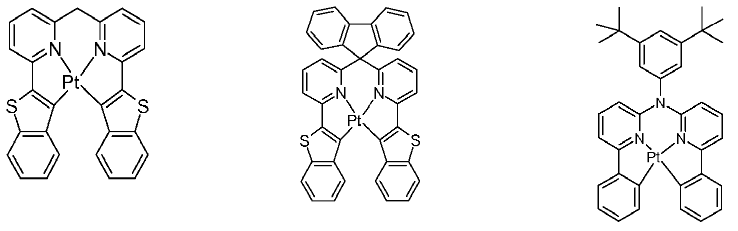

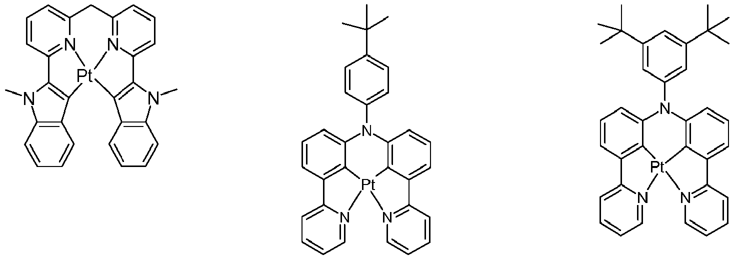

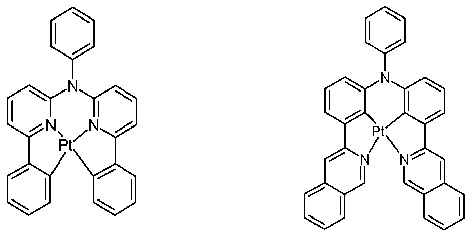

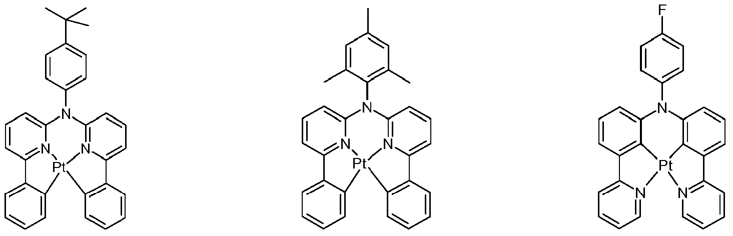

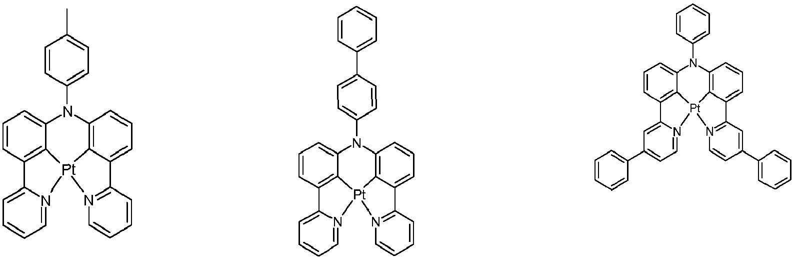

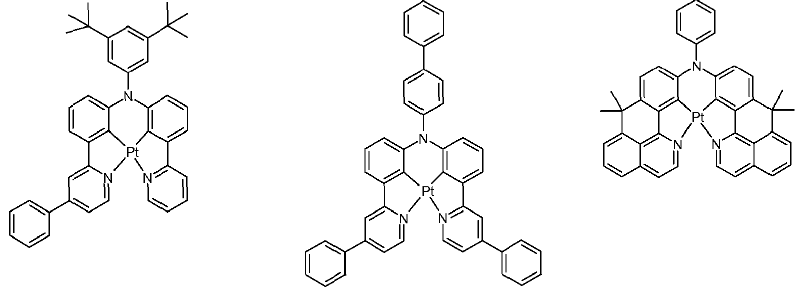

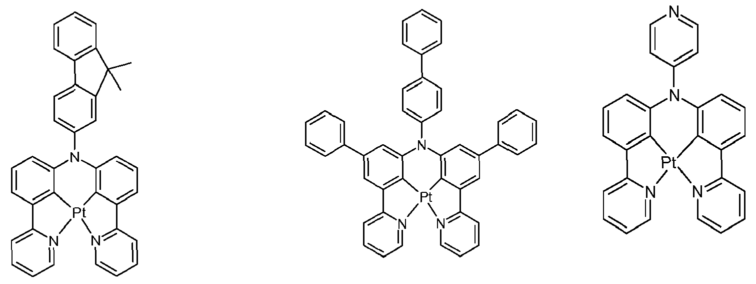

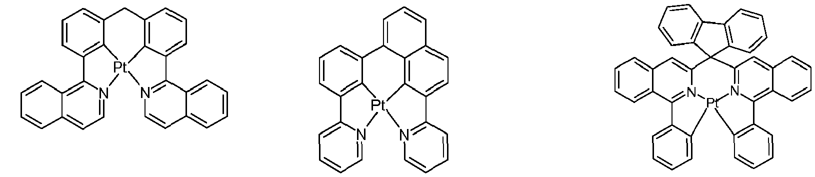

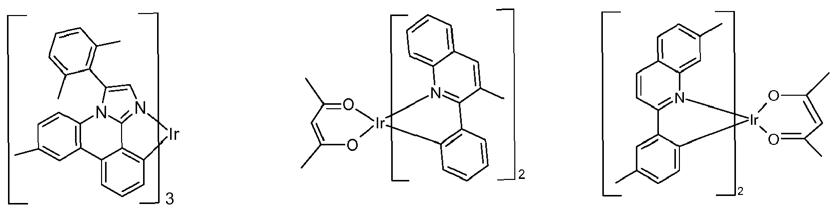

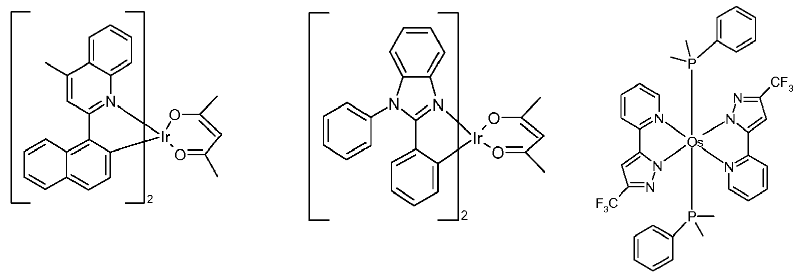

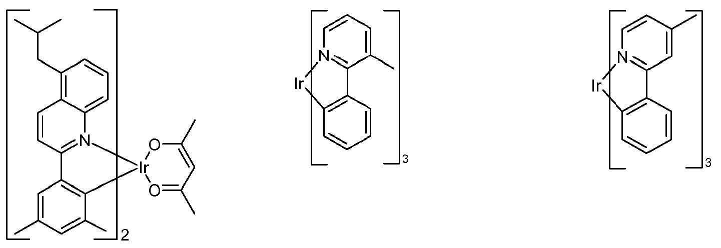

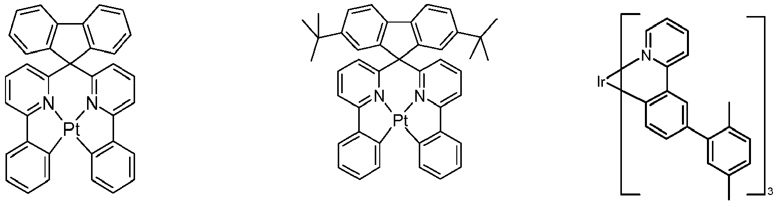

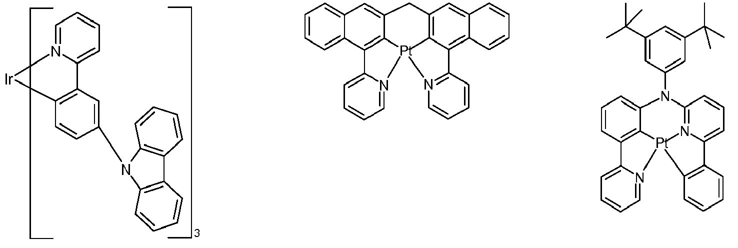

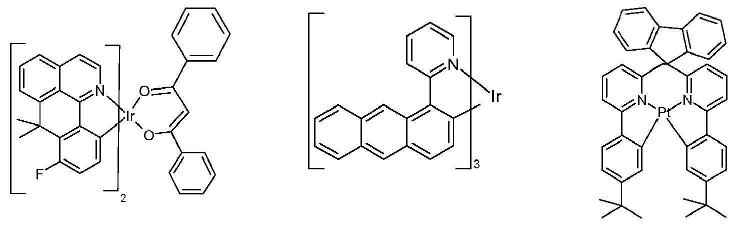

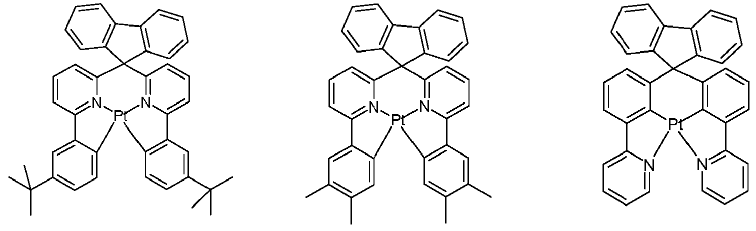

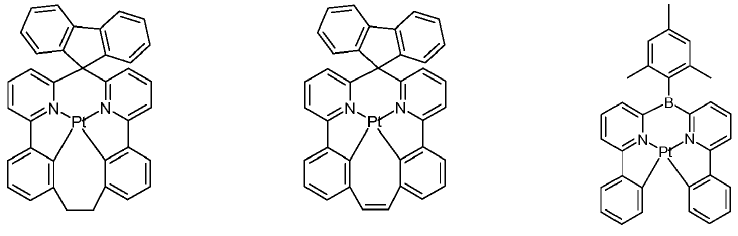

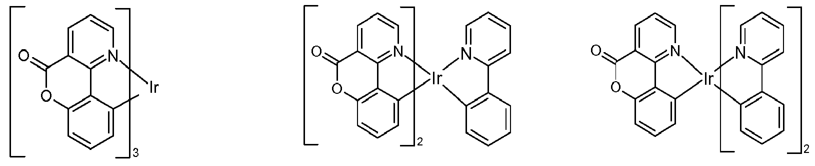

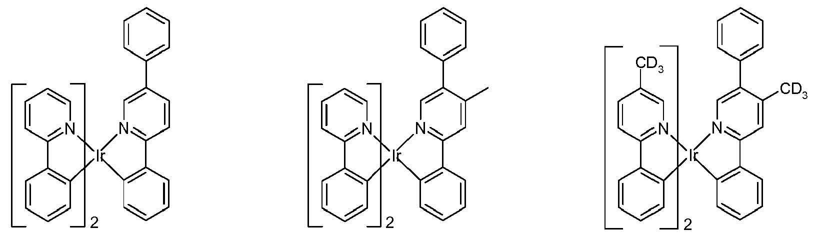

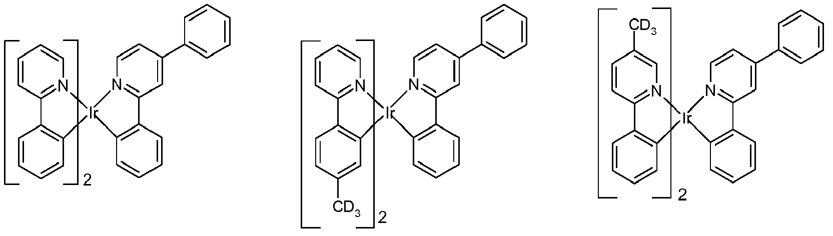

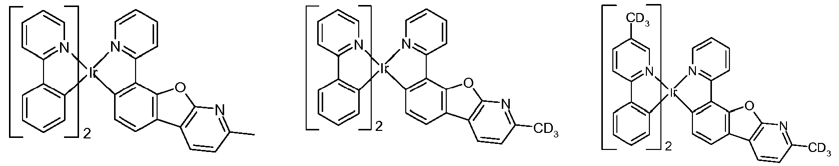

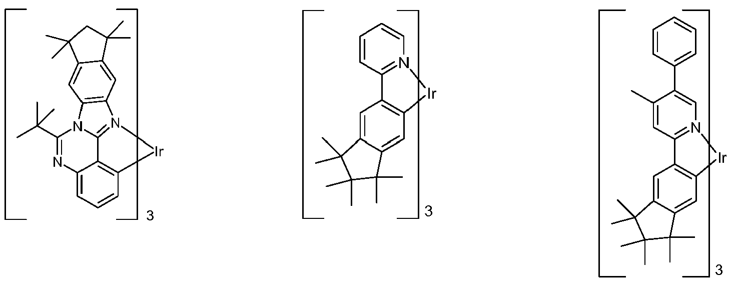

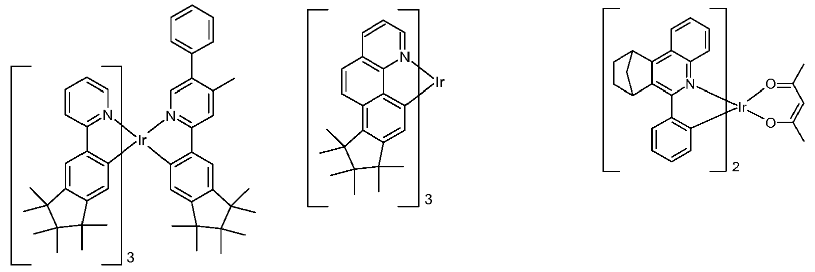

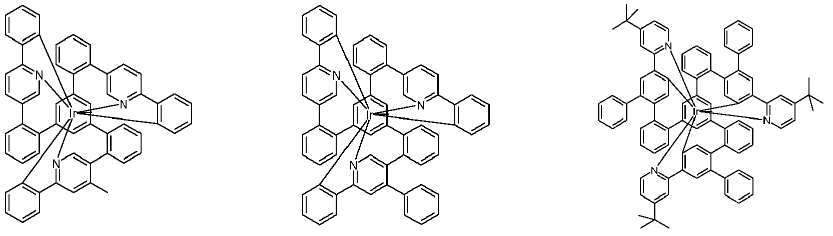

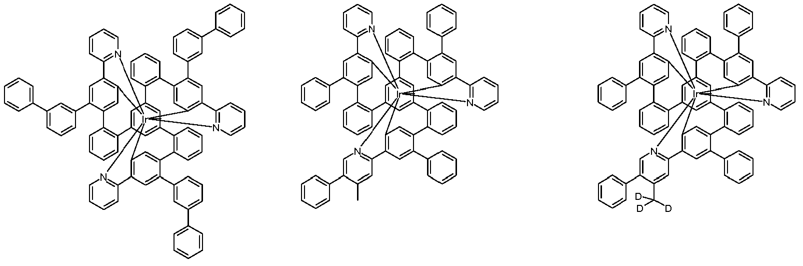

Die erfindungsgemäßen Verbindungen sind insbesondere auch geeignet als Matrixmaterialien für phosphoreszierende Emitter in organischen Elektrolumineszenzvorrichtungen, wie sie z. B. in WO 98/24271, US 2011/0248247 und US 2012/0223633 beschrieben sind. In diesen mehrfarbigen Display-Bauteilen wird eine zusätzliche blaue Emissionsschicht vollflächig auf alle Pixel, auch diejenigen mit einer von Blau verschiedenen Farbe, aufgedampft. Dabei wurde überraschend gefunden, dass die erfindungsgemäßen Verbindungen, wenn sie als Matrixmaterialien für das rote und/oder grüne Pixel eingesetzt werden, zusammen mit der aufgedampften blauen Emissionsschicht zu weiterhin sehr guter Emission führen.

In einer weiteren Ausführungsform der Erfindung enthält die erfindungsgemäße organische Elektrolumineszenzvorrichtung keine separate Lochinjektionsschicht und/oder Lochtransportschicht und/oder Lochblockierschicht und/oder Elektronentransportschicht, d. h. die emittierende Schicht grenzt direkt an die Lochinjektionschicht oder die Anode an, und/oder die emittierende Schicht grenzt direkt an die Elektronentransportschicht oder die Elektroneninjektionsschicht oder die Kathode an, wie zum Beispiel in WO 2005/053051 beschrieben. Weiterhin ist es möglich, einen Metallkomplex, der gleich oder ähnlich dem Metallkomplex in der emittierenden Schicht ist, direkt angrenzend an die emittierende Schicht als Lochtransport- bzw. Lochinjektionsmaterial zu verwenden, wie z. B. in WO 2009/030981 beschrieben.

In einer weiteren bevorzugten Ausführungsform der Erfindung enthält die organische Elektrolumineszenzvorrichtung die erfindungsgemäße Verbindung in einer Elektronentransportschicht bzw. in einer Lochblockierschicht.

In den weiteren Schichten der erfindungsgemäßen organischen Elektrolumineszenzvorrichtung können alle Materialien verwendet werden, wie sie üblicherweise gemäß dem Stand der Technik eingesetzt werden. Der Fachmann kann daher ohne erfinderisches Zutun alle für organische Elektrolumineszenzvorrichtungen bekannten Materialien in Kombination mit den erfindungsgemäßen Verbindungen gemäß Formel (1) bzw. den oben ausgeführten bevorzugten Ausführungsformen einsetzen.

Weiterhin bevorzugt ist eine organische Elektrolumineszenzvorrichtung, dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Es ist aber auch möglich, dass der Anfangsdruck noch geringer ist, beispielsweise kleiner 10⁻⁷ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden.

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck, Offsetdruck, LITI (Light Induced Thermal Imaging, Thermotransferdruck), Ink-Jet Druck (Tintenstrahldruck) oder Nozzle Printing, hergestellt werden. Hierfür sind lösliche Verbindungen nötig, welche beispielsweise durch geeignete Substitution erhalten werden.

Weiterhin sind Hybridverfahren möglich, bei denen beispielsweise eine oder mehrere Schichten aus Lösung aufgebracht werden und eine oder mehrere weitere Schichten aufgedampft werden.

Diese Verfahren sind dem Fachmann generell bekannt und können von ihm ohne erfinderisches Zutun auf organische Elektrolumineszenzvorrichtungen enthaltend die erfindungsgemäßen Verbindungen angewandt werden.

Die erfindungsgemäßen Verbindungen und die erfindungsgemäßen organischen Elektrolumineszenzvorrichtungen zeichnen sich durch einen oder mehrere der folgenden überraschenden Vorteile gegenüber dem Stand der Technik aus:
1. Die erfindungsgemäßen Verbindungen, eingesetzt als Matrixmaterial für phosphoreszierende Emitter, führen zu langen Lebensdauern.
2. Die erfindungsgemäßen Verbindungen führen zu hohen Effizienzen. Dies gilt insbesondere, wenn die Verbindungen als Matrixmaterial für einen phosphoreszierenden Emitter eingesetzt werden.
3. Die erfindungsgemäßen Verbindungen führen zu geringen Betriebsspannungen. Dies gilt insbesondere, wenn die Verbindungen als Matrixmaterial für einen phosphoreszierenden Emitter eingesetzt werden.

Diese oben genannten Vorteile gehen nicht mit einer Verschlechterung der weiteren elektronischen Eigenschaften einher.

Die Erfindung wird durch die nachfolgenden Beispiele näher erläutert, ohne sie dadurch einschränken zu wollen. Der Fachmann kann aus den Schilderungen die Erfindung im gesamten offenbarten Bereich ausführen und ohne erfinderisches Zutun weitere erfindungsgemäße Verbindungen herstellen und diese in elektronischen Vorrichtungen verwenden bzw. das erfindungsgemäße Verfahren anwenden.

### Beispiele:

Die nachfolgenden Synthesen werden, sofern nicht anders angegeben, unter einer Schutzgasatmosphäre in getrockneten Lösungsmitteln durchgeführt. Die Lösungsmittel und Reagenzien können von ALDRICH bzw. ABCR bezogen werden. Die bei den nicht kommerziell erhältlichen Edukten angegeben Nummern sind die entsprechenden CAS-Nummern. Weitere Literatur: Dissertation: "Entwicklung neuer Synthesewege zu Pyridoacridinen" von Stephan Raeder (2012), Universität München.

### a) 4-Methyl-2-oxo-1,2-dihydrochinolin-3-carbonitril

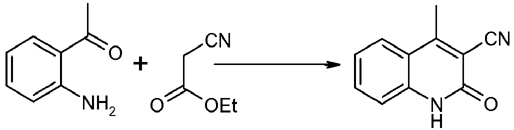

Zu 10.0 g (73.9 mmol) 2-Aminoacetophenon werden 10.0 g (88.4 mmol) Cyanessigsäureethylester gegeben. Der Ansatz wird bei 200 °C im Ölbad 1 h unter Rückfluss erhitzt. Anschließend wird der Ansatz abgekühlt und mit 50 mL Ethanol versetzt. Es entsteht ein heller Niederschlag, welcher abfiltriert wird. Um die Ausbeute zu erhöhen, kann der Überstand nochmals mit 5 g Cyanessigsäureethylester versetzt und weitere 1.5 h auf 200 °C erhitzt werden. Zum Aufreinigen des Rückstands wird dieser in Dichlormethan und anschließend in Petroleumbenzin suspendiert, als weiße Kristalle abgetrennt und getrocknet. Ausbeute: 5.4 g (29 mmol), 40 % der Theorie.

Analog dazu werden die folgenden Verbindungen hergestellt:

| | **Edukt 1** | **Edukt 2** | **Produkt** | **Ausbeute** |
|---|---|---|---|---|
| 1a | 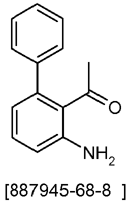 | 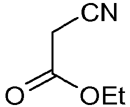 | 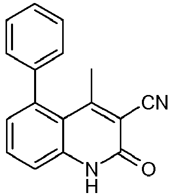 | 37% |
| 2a | 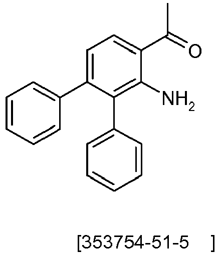 | 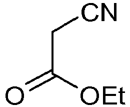 | 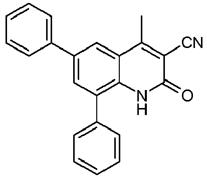 | 34% |

### b) 2-Chlor-4-methylchinolin-3-carbonitril

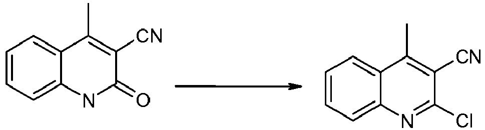

5 g (27 mmol) 4-Methyl-2-oxo-1,2-dihydrochinolin-3-carbonitril werden mit 15 ml Phosphorylchlorid 3 h unter Rückfluss erhitzt. Anschließend wird das Phosphorylchlorid unter Vakuum abgezogen, der Rückstand mit Ammoniumhydrochlorid-Lösung versetzt und abfiltriert. Der Rückstand wird mit Wasser gewaschen und aus Ethanol umkristallisiert. Ausbeute: 5.5 g (27 mmol), 90 % der Theorie.

Analog dazu werden die folgenden Verbindungen hergestellt:

| | **Edukt 1** | **Produkt** | **Ausbeute** |
|---|---|---|---|
| 1b | 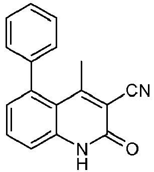 | 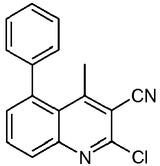 | 89% |
| 2b | 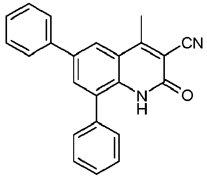 | 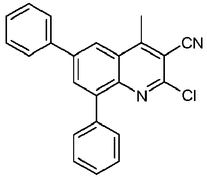 | 88% |

### c) 4-Methylchinolin-3-carbonitril

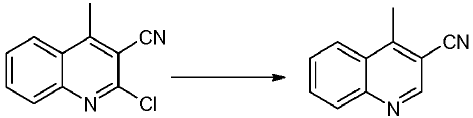

15 g (74 mmol) 2-Chlor-4-methylchinolin-3-carbonitril werden mit 8 g Natriumacetat versetzt und in ca. 800 mL Methanol suspendiert. Dazu werden ca. 200 mg Palladium-Kohle (10 %) gegeben, und der Ansatz wird 24 h bei Raumtemperatur und Normaldruck unter H₂-Atmosphäre hydriert. Nach dem Abfiltrieren des Katalysators erhält man eine gelbgrüne Lösung, die nach Abrotieren des Methanols einen gelben Rückstand bildet. Die Aufreinigung erfolgt mittels Flashsäulenchromatographie über Kieselgel (Fließmittel: Dichlormethan: Ethylacetat = 5:1). Ausbeute: 12 g (71 mmol), 92 % der Theorie.

Analog dazu werden die folgenden Verbindungen hergestellt:

| | **Edukt 1** | **Produkt** | **Ausbeute** |
|---|---|---|---|
| 1c | 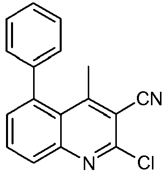 | 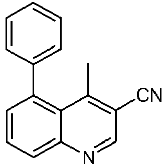 | 90% |
| 2c | 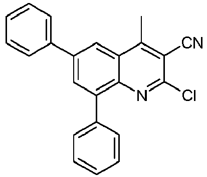 | 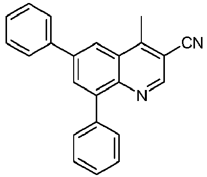 | 85% |

### d) 4-Brombenzo[c][2,7]naphthyridin

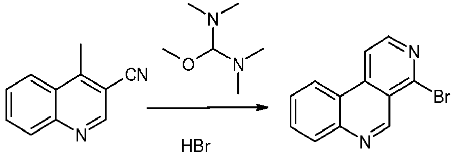

25 g (150 mmol) 4-Methylchinolin-3-carbonitril werden in 500 mL DMF gelöst und mit 50 g (290 mmol) Bredereck's Reagenz versetzt. Der Ansatz wird 15 min bei 150 W und max. 165 °C im Mikrowellenreaktor umgesetzt. Das Lösungsmittel wird am Rotationsverdampfer abdestilliert und der Rückstand in 600 mL Bromwasserstoff in Eisessig (40%) und 400 mL Eisessig gelöst. Der Reaktionsansatz wird 1 h auf 60 °C erhitzt, anschließend in 500 mL Wasser überführt, mit Kaliumcarbonat neutralisiert und mit Dichlormethan (4 x 150 mL) extrahiert. Die organischen Phasen werden vereinigt, über MgSO₄ getrocknet und das Lösungsmittel am Rotationsverdampfer abdestilliert. Der Rückstand wird mittels Flashsäulenchromatographie (Dichlormethan:Methanol = 97:3) aufgereinigt. Ausbeute: 36 g (136 mmol), 95 % der Theorie.

Analog dazu werden die folgenden Verbindungen hergestellt:

| | **Edukt 1** | **Produkt** | **Ausbeute** |
|---|---|---|---|
| 1d | 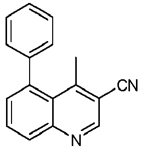 | 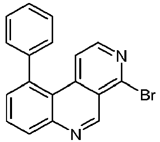 | 92% |
| 2d | 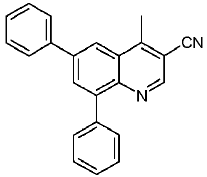 | 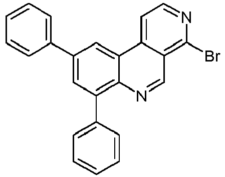 | 90% |

### e) 4-Brombenzo[c][2,7]naphthyridin-5-carbonsäureethylester

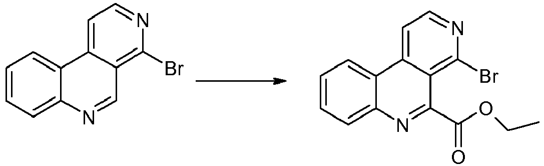

12 g (45 mmol) 4-Brom-5,6-dihydrobenzo[c][2,7]naphthyridin-5-carbonsäureethylester werden in 400 mL Dichlormethan gelöst und mit 39 g (450 mmol) Mangan(IV)oxid versetzt. Der Ansatz wird im Mikrowellenreaktor mit 150 W bei max. 7.0 bar und 100 °C 10 min (ramptime: 6 min) umgesetzt. Nach dem Abkühlen wird der Reaktionsansatz filtriert, mit Dichlormethan gewaschen und das Lösungsmittel am Rotationsverdampfer abdestilliert. Ausbeute: 14 g (42 mmol), 98 % der Theorie.

Analog dazu werden die folgenden Verbindungen hergestellt:

| | **Edukt 1** | **Produkt** | **Ausbeute** |
|---|---|---|---|
| 1e | 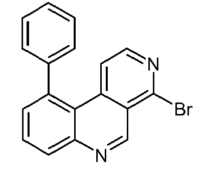 | 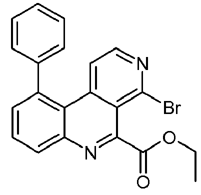 | 93% |
| 2e | 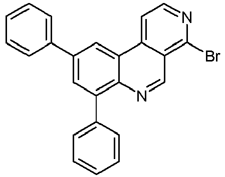 | 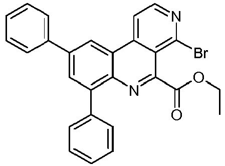 | 90% |

### f) 4-Phenylbenzo[c][2,7]naphthyridin-5-carbonsäureethylester

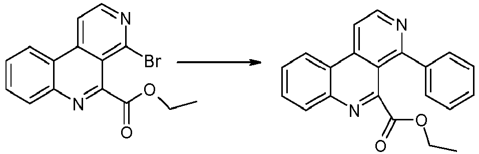

60 g (180 mmol) 4-Brombenzo[c][2,7]naphthyridin-5-carbonsäureethylester und 4 g (18 mmol) Palladium(II)acetat werden in 400 mL THF suspendiert. Dazu wird eine Lösung von 33 g (270mmol) Benzolboronsäure und 450 mL (540 mmol) 1M- Kaliumcarbonatlösung in 400 mL THF gegeben. Der Ansatz wird unter Stickstoffatmosphäre 40 h unter Rückfluss erhitzt, anschließend in 200 mL Wasser überführt und mit Dichlormethan (4 x 200 mL) extrahiert. Die vereinigten organischen Phasen werden über MgSO₄ getrocknet und das Lösungsmittel am Rotationsverdampfer abdestilliert. Der Rückstand wird mittels Flashsäulenchromatographie (Dichlormethan : Ethylacetat = 3 : 1) aufgereinigt. Ausbeute: 35 g (106 mmol), 60 % der Theorie.

Analog dazu werden die folgenden Verbindungen hergestellt:

| | **Edukt 1** | **Produkt** | **Ausbeute** |
|---|---|---|---|
| 1f | 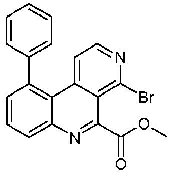 | 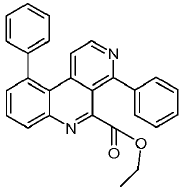 | 57% |
| 2f | 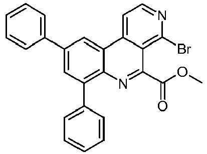 | 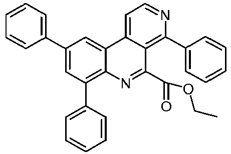 | 53% |

### g) 1,8-Diazabenzo[fg]naphthacen-9-on

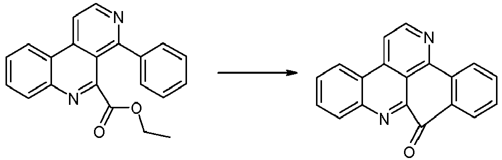

20 g (60 mmol) 4-Phenylbenzo[c][2,7]naphthyridin-5-carbonsäureethylester werden in 100 mL Trifluormethansulfonsäure gelöst und mit Hilfe der Mikrowelle umgesetzt (Reaktionsbedingungen: 150 W, 15 min. Reaktionsdauer, ramptime: 1 min, 7.0 bar Maximaldruck, max. 85 °C). Anschließend wird der Ansatz mit 200 mL Eiswasser versetzt, mit K₂CO₃ neutralisiert und mit Dichlormethan (3 x 250 mL) extrahiert. Die vereinigten organischen Phasen werden über MgSO₄ getrocknet und im Vakuum eingeengt. Der Rückstand wird mittels Flashsäulenchromatographie (Dichlormethan : Ethylacetat = 3 : 1) aufgereinigt. Ausbeute: 7 g (24 mmol), 42 % der Theorie.

Analog dazu werden die folgenden Verbindungen hergestellt:

| | **Edukt 1** | **Produkt** | **Ausbeute** |
|---|---|---|---|
| 1g | 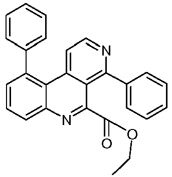 | 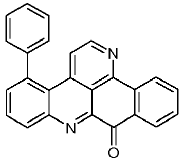 | 41% |
| 2g | 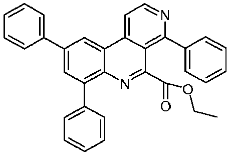 | 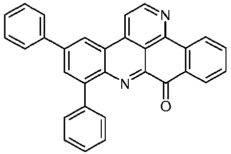 | 37% |

### h) 11-Methyl-5-oxa-6-azanaphthacen-12-on

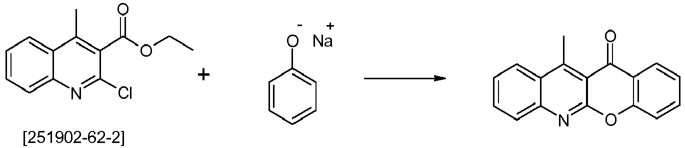

52.6 g (211 mmol) 2-Chlor-4-methylchinolin-3-carbonsäureethylester werden mit 54 g (317 mmol) Natriumphenolat in 100 mL Dimethylformamid gelöst und 6 h bei 155 °C umgesetzt. Im Anschluss wird das Lösemittel am Rotationsverdampfer entfernt, der Rückstand mit der 10-fachen Menge Polyphosphorsäure versetzt und 5 h auf 130 °C erhitzt. Der Ansatz wird auf Eis gegossen und mit 6N-NaOH neutralisiert. Die neutralisierte Lösung wird mit Ethylacetat (4 x 500 mL) extrahiert. Die vereinigten organischen Phasen werden über MgSO₄ getrocknet und das Lösungsmittel am Rotationsverdampfer abdestilliert. Der Rückstand wird mittels Flashsäulenchromatographie (Dichlormethan: Ethylacetat = 3 : 1) aufgereinigt. Ausbeute: 13.8 g (52 mmol), 25% der Theorie.

Analog dazu werden die folgenden Verbindungen hergestellt:

| | **Edukt 1** | **Edukt 2** | **Produkt** | **Ausbeute** |
|---|---|---|---|---|
| 1h | 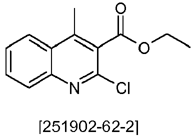 | 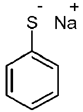 | 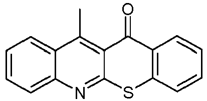 | 26% |
| 2h | 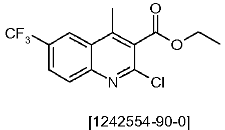 | 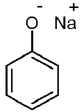 | 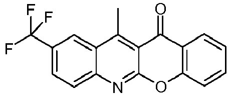 | 25% |

### j) 9-Oxa-1,8-diazabenzo[fg]naphthacen

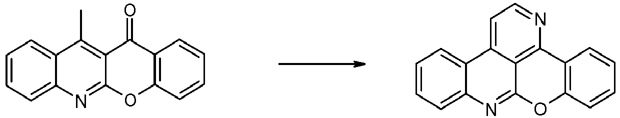

15.7 g (60 mmol) 11-Methyl-5-oxa-6-azanaphthacen-12-on werden mit 22 g (150 mmol) Diethylacetal in 10 mL DMF gelöst und 3 h bei 150 °C und 200 W in der Labormikrowelle umgesetzt. Im Anschluss wird das Lösungsmittel am Rotationsverdampfer entfernt, der Rückstand mit der 10-fachen Menge NH₄OAC versetzt und 2 h auf 114 °C erhitzt. Der Ansatz wird auf Eis gegossen und mit Dichlormethan (4 x 50 mL) extrahiert. Die vereinigten organischen Phasen werden über MgSO₄ getrocknet und das Lösungsmittel am Rotationsverdampfer abdestilliert. Der Rückstand wird mittels Flashsäulenchromatographie (Dichlormethan : Ethylacetat = 3 : 1) aufgereinigt. Ausbeute: 7,8 g (27 mmol), 50% der Theorie.

Analog dazu werden die folgenden Verbindungen hergestellt:

| | **Edukt 1** | **Produkt** | **Ausbeute** |
|---|---|---|---|
| 1j | 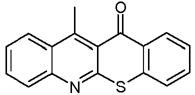 | 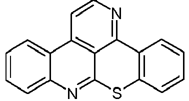 | 52% |
| 2j | 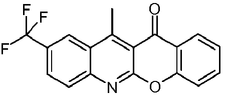 | 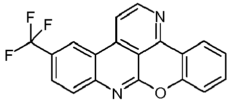 | 46% |
| 3j | 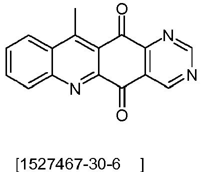 | 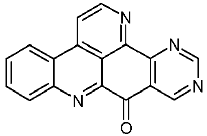 | 48% |

### I) 4-Methyl-2-phenylamino-chinolin-3-carbonitril

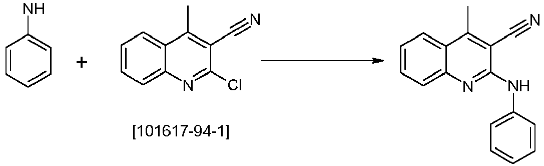

38 g (190 mmol) 2-Chloro-4-methyl-3-chinolincarbonitril werden mit 27 g (285 mmol) Anilin in 100 mL Ethylenglycol gelöst und 6 h auf 140 °C erhitzt. Anschließend wird der Ansatz in 50 mL Wasser überführt und mit Dichlormethan extrahiert (3 x 50 mL). Die vereinigten organischen Phasen werden über MgSO₄ getrocknet und das Lösungsmittel am Rotationsverdampfer abdestilliert. Der Rückstand wird mittels Flashsäulenchromatographie (Dichlormethan : Ethylacetat = 3 : 1) aufgereinigt. Ausbeute: 39 g (152 mmol), 80% der Theorie.

Analog dazu werden die folgenden Verbindungen hergestellt:

| | **Edukt 1** | **Edukt 2** | **Produkt** | **Ausbeute** |
|---|---|---|---|---|
| 1i | 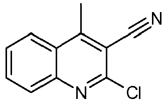 | 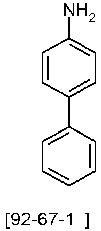 | 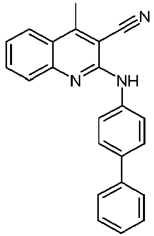 | 76% |
| 2i | 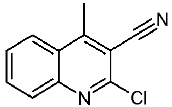 | 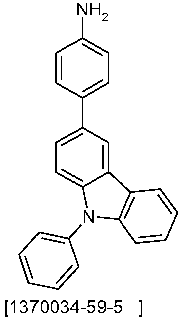 | 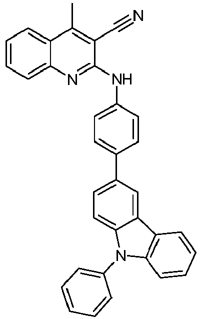 | 78% |

### k) 4-((E)-2-Dimethylamino-vinyl)-2-phenylamino-chinolin-3-carbonitril

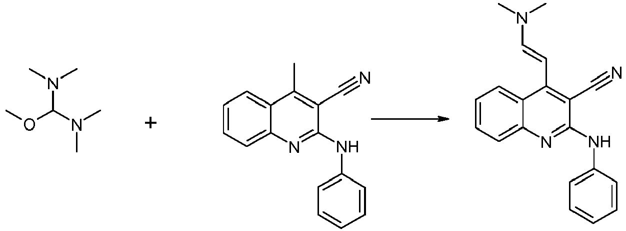

46 g (185 mmol) 4-Methyl-2-phenylamino-chinolin-3-carbonitril werden mit 99.6 g (835 mmol) Dimethoxymethyl-dimethylamin in 400 ml N,N-Dimethylformamid (400 ml) 12 h auf 100 °C erhitzt. Nach dem Abkühlen werden 1000 ml Eiswasser zugeben. Der Feststoff wird abgesaugt und mit etwas Heptan gewaschen. Ausbeute: 50 g (160 mmol), 89% der Theorie.

Analog dazu werden die folgenden Verbindungen hergestellt:

| | **Edukt 1** | **Produkt** | **Ausbeute** |
|---|---|---|---|
| 1k | 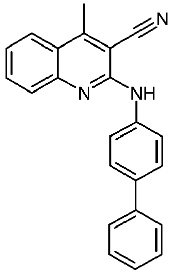 | 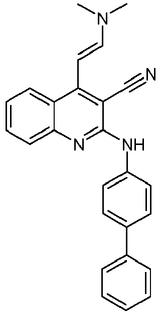 | 80% |
| 2k | 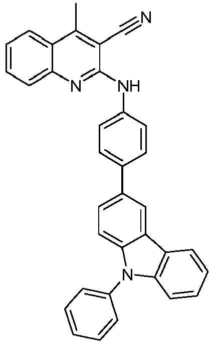 | 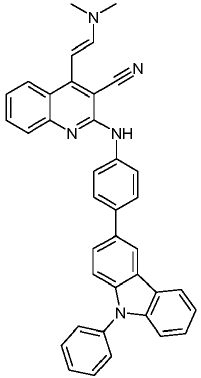 | 77% |
| 3k | 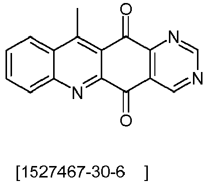 | 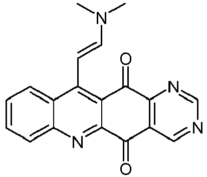 | 63% |
| 4k | 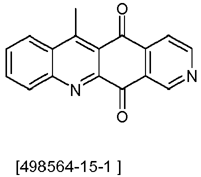 | 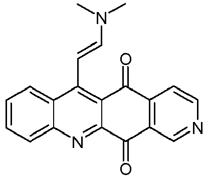 | 64% |
| 5k | 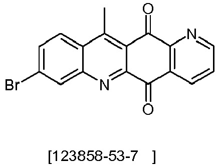 | 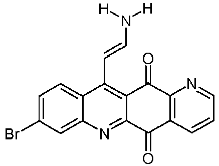 | 63% |
| 6k | 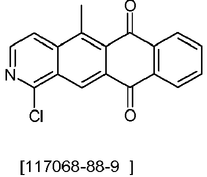 | 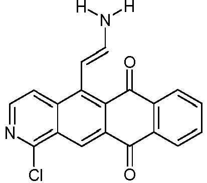 | 60% |

### I) 9H-1,8,9-Triaza-benzo[fg]naphthacen

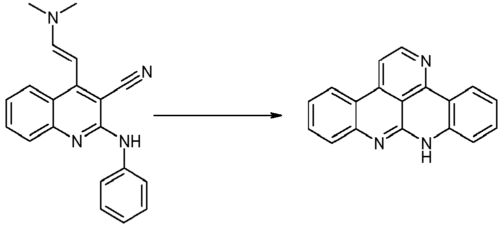

27.3 g (87 mmol) 4-((E)-2-Dimethylamino-vinyl)-2-phenylamino-chinolin-3-carbonitril werden unter Schutzgas in 60 ml konz. H₂SO₄ suspendiert und 4 h auf 70 °C erhitzt. Nach dem Abkühlen wird die Mischung auf Eiswasser gegeben, der pH mit Na₂CO₃-Lösung auf 9 eingestellt und mit Essigester extrahiert. Nach dem Einengen wird das Produkt chromatographisch (Methanol:Dichlormethan 1:2) gereinigt. Ausbeute: 50 g (160 mmol), 89% der Theorie.

Analog dazu werden die folgenden Verbindungen hergestellt:

| | **Edukt 1** | **Produkt** | **Ausbeute** |
|---|---|---|---|
| 1l | 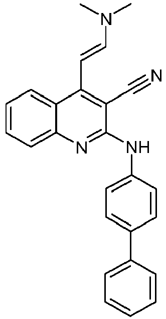 | 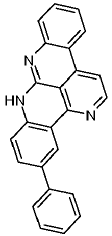 | 84% |
| 2l | 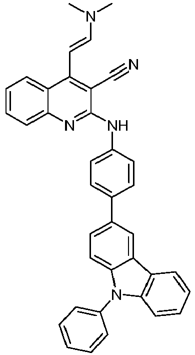 | 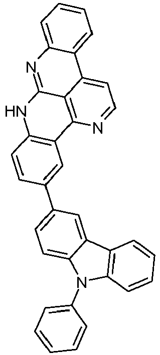 | 86% |

### m) 1,8,11,13-Tetraaza-benzo[fg]naphthacen-9-on

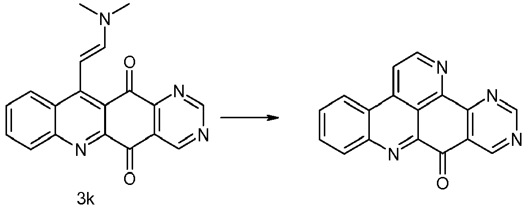

24.7 g (75 mmol) 11-((E)-2-Dimethylamino-vinyl)-1,3,6-triaza-naphthacen-5,12-dion werden mit 28.9 g (375 mmol) Ammoniumacetat in 400 ml Ethanol für 3 h unter Rückfluss erhitzt. Der Feststoff wird abgesaugt und mit etwas Heptan gewaschen. Ausbeute: 19.1 g (67 mmol), 90% der Theorie.

Analog dazu werden die folgenden Verbindungen hergestellt:

| | **Edukt 1** | **Produkt** | **Ausbeute** |
|---|---|---|---|
| 1m | 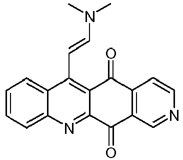 | 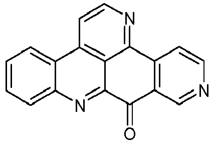 | 86% |
| 2m | 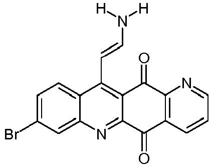 | 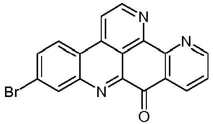 | 83% |
| 3m | 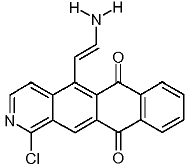 | 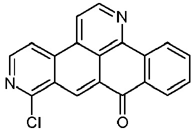 | 84% |

### n) Spirosynthese

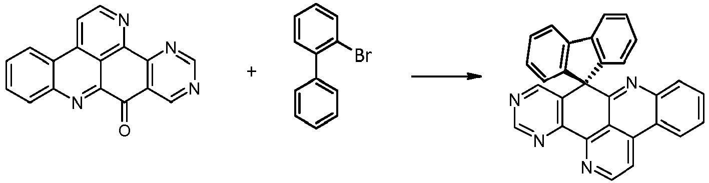

In einem 1L-Vierhalskolben werden 23 g (99 mmol) 2-Brombiphenyl in 100 mL THF vorgelegt und auf -78 °C gekühlt. Über einen Tropftrichter werden 41.0 mL (103 mmol) n-BuLi (2.5 M in n-Hexan) bei dieser Temperatur hinzugetropft und 1 h gerührt. Anschließend werden 13 g (49 mmol) 1,8,11,13-Tetraaza-benzo[fg]naphthacen-9-on, gelöst in 300 mL THF, über einen Tropftrichter hinzugegeben, und innerhalb von 3 h wird die Reaktion auf Raumtemperatur erwärmt. Daraufhin wird mit 500 mL Wasser hydrolysiert und die organischen Lösungsmittel am Rotationsverdampfer entfernt. Der dabei ausfallende Feststoff wird filtriert, in 400 mL Eisessig suspendiert und nach Hinzugabe von 150 mL konzentrierter Salzsäure 2 h bei 100 °C gerührt. Nach Abkühlen auf Raumtemperatur wird mit 400 mL Wasser versetzt, der dabei ausgefallene Feststoff filtriert und mit 200 mL Wasser, 200 mL Ethanol und zuletzt mit 200 mL n-Heptan gewaschen. Der Feststoff wird über Aluminiumoxid mit *n*-Heptan/Toluol heißextrahiert und umkristallisiert. Die weitere Aufreinigung erfolgt mittels Umkristallisation aus Toluol/Heptan und Zonensublimation (310 °C, 10⁻⁵ bar). Ausbeute: 15,9 g (37 mmol), 83% der Theorie

Analog werden die folgenden Verbindungen hergestellt:

| | **Edukt 1** | **Produkt** | **Ausbeute** |
|---|---|---|---|
| 1n | 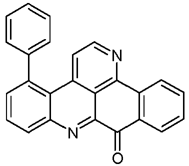 | 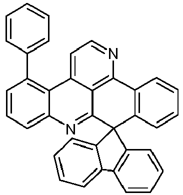 | 82% |
| 2n | 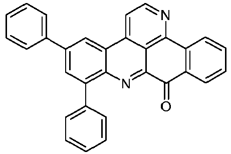 | 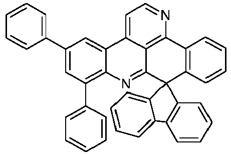 | 79% |
| 3n | 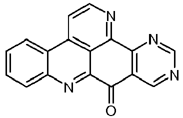 | 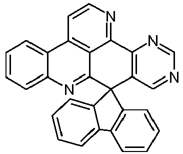 | 81% |
| 4n | 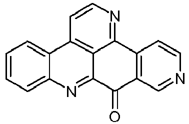 | 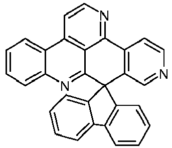 | 86% |
| 5n | 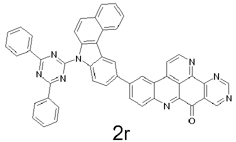 | 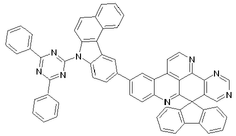 | 65% |

### o) 9-Phenyl-9H-1,8,9-triazabenzo[fg]naphthacen

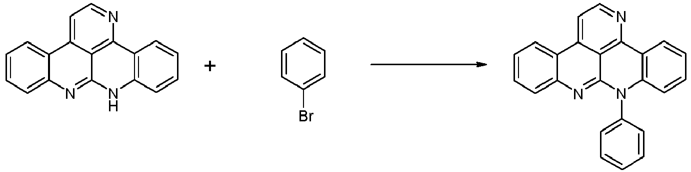

Eine entgaste Lösung von 23 g (147 mmol) Brombenzol und 39 g (147 mmol) 9H-1,8,9-Triaza-benzo[fg]naphthacen in 600 mL Toluol wird 1 h mit N₂ gesättigt. Danach wird die Lösung zuerst mit 2.09 mL (8.6 mmol) P(*t*Bu)₃, dann mit 1.38 g (6.1 mmol) Palladium(II)acetat versetzt und anschließend werden 17.7 g (185 mmol) NaO*t*Bu im festen Zustand zugegeben. Die Reaktionsmischung wird 1 h unter Rückfluss erhitzt. Nach dem Abkühlen auf Raumtemperatur werden vorsichtig 500 mL Wasser zugesetzt. Die wässrige Phase wird mit 3 x 50 mL Toluol gewaschen, über MgSO₄ getrocknet und das Lösungsmittel im Vakuum entfernt. Danach wird das Rohprodukt über Kieselgel mit Heptan/Essigsäureester (20/1) chromatographisch gereinigt. Der Rückstand wird aus Toluol umkristallisiert und abschließend im Hochvakuum (p = 5 x 10⁻⁶ mbar) sublimiert. Die Ausbeute beträgt 40 g (115 mmol), entsprechend 80% der Theorie.

Analog dazu werden die folgenden Verbindungen hergestellt:

| | **Edukt 1** | **Edukt 2** | **Produkt** | **Ausbeute** |
|---|---|---|---|---|
| 1o | 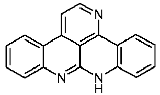 | 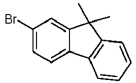 | 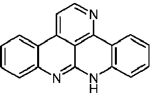 | 83% |
| 2o | 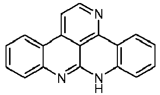 | 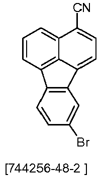 | 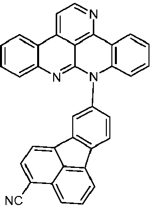 | 81% |
| 3o | 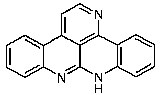 | 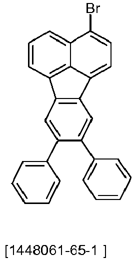 | 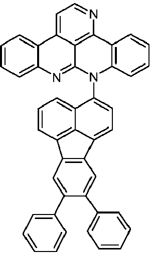 | 77% |
| 4o | 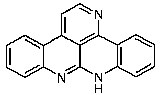 | 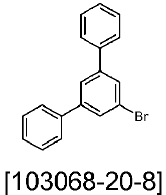 | 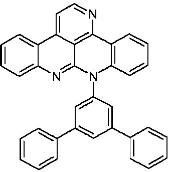 | 72% |
| 5o | 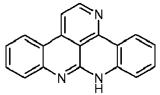 | 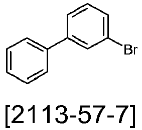 | 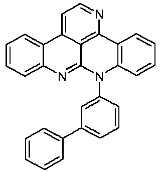 | 63% |
| 6o | 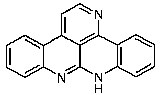 | 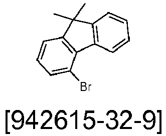 | 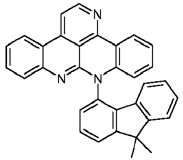 | 52% |
| 7o | 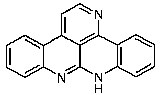 | 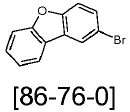 | 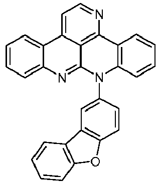 | 81% |
| 8o | 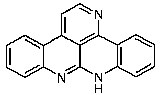 | 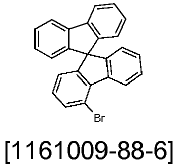 | 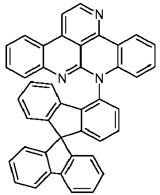 | 84% |
| 9o | 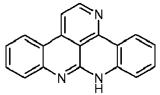 | 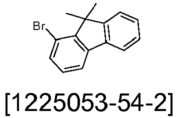 | 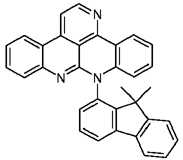 | 87% |
| 10o | 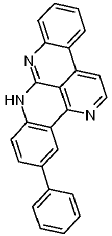 | 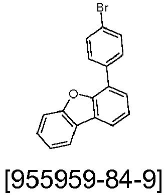 | 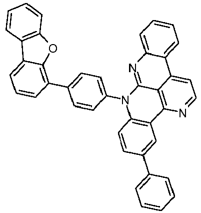 | 80% |
| 11o | 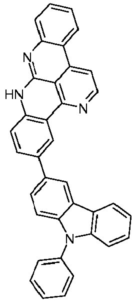 | 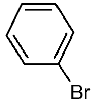 | 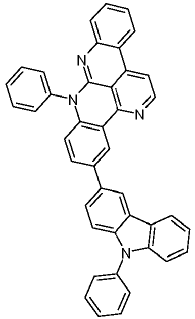 | 78% |
| 12o | 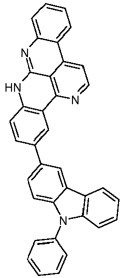 | 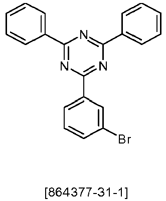 | 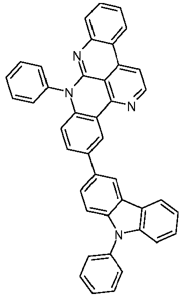 | 77% |
| 13o | 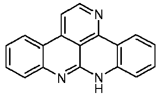 | 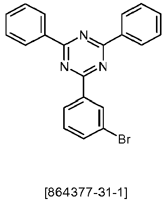 | 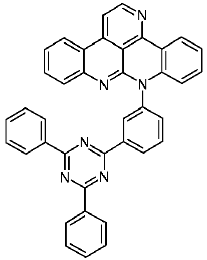 | 79% |
| 14o | 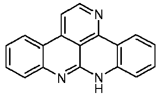 | 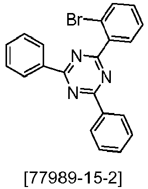 | 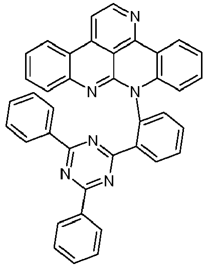 | 60% |
| 15o | 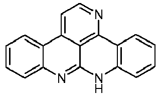 | 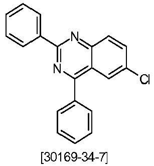 | 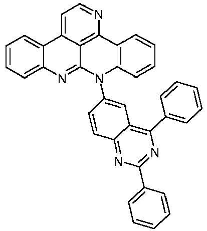 | 61% |
| 16ο | 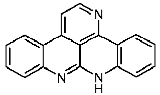 | 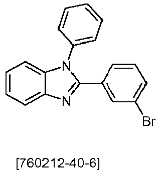 | 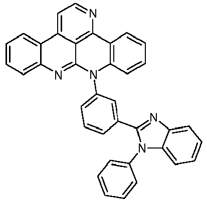 | 68% |
| 17o | 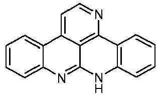 | 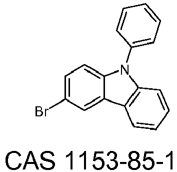 | 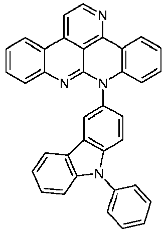 | 65% |
| 18o | 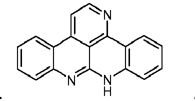 | 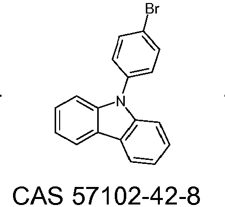 | 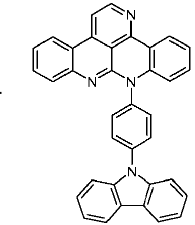 | 62% |

### p) 9-(4,6-Diphenyl-pyridin-2-yl)-9H-1,8,9-triaza-benzo[fg]naphthacen

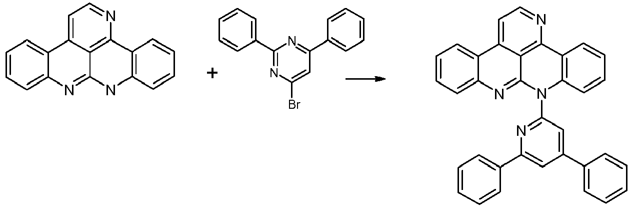

16 g (60 mmol) 9H-1,8,9-Triaza-benzo[fg]naphthacen werden in 300 ml Dimethylformamid unter Schutzgasatmosphäre gelöst und mit 3 g NaH, 60%ig in Mineralöl, (75 mmol) versetzt. Nach 1 h bei Raumtemperatur wird eine Lösung von 19 g (62 mmol) 4-Bromo-2,6-diphenyl-pyrimidin in 150 mL Dimethylformamid zugetropft. Das Reaktionsgemisch wird 12 h bei Raumtemperatur gerührt. Nach dieser Zeit wird das Reaktionsgemisch auf Eis gegossen und dreimal mit Dichlormethan extrahiert. Die vereinigten organischen Phasen werden über Na₂SO₄ getrocknet und eingeengt. Der Rückstand wird aus Toluol umkristallisiert und abschließend zweimal fraktioniert sublimiert (p ca. 10⁻⁶ mbar, T = 330-360 °C). Ausbeute: 23 g, 80 % d. Th.; Reinheit: 99.9 % n. HPLC.

Analog dazu werden die folgenden Verbindungen hergestellt:

| | **Edukt 1** | **Edukt 2** | **Produkt** | **Ausbeute** |
|---|---|---|---|---|
| 1p | 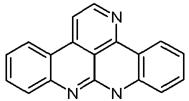 | 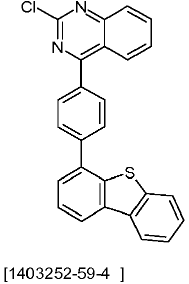 | 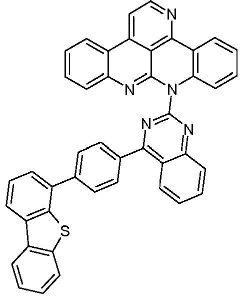 | 76% |
| 2p | 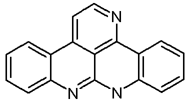 | 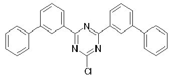 | 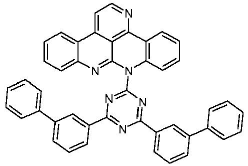 | 77% |
| 3p | 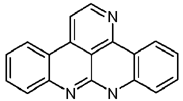 | 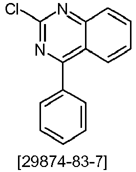 | 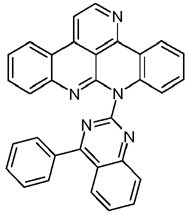 | 82% |
| 4p | 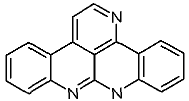 | 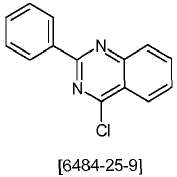 | 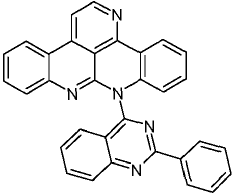 | 67% |
| 5p | 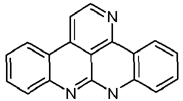 | 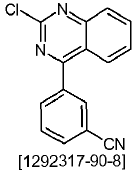 | 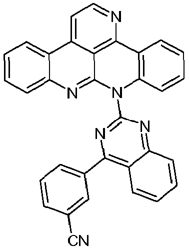 | 87% |
| 6p | 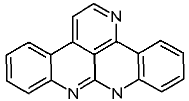 | 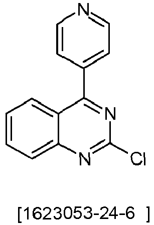 | 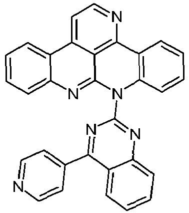 | 88% |
| 7p | 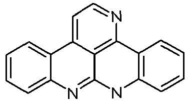 | 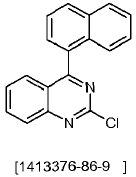 | 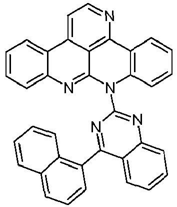 | 74% |
| 8p | 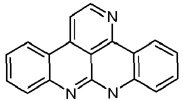 | 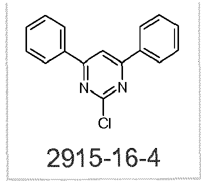 | 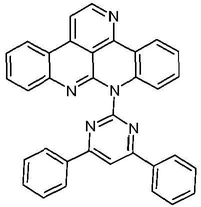 | 75% |
| 9p | 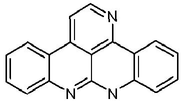 | 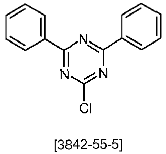 | 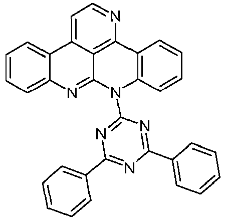 | 76% |
| 10p | 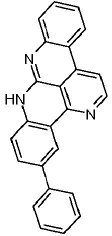 | 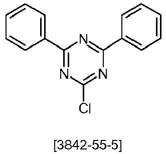 | 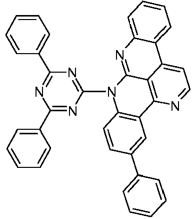 | 81% |
| 11p | 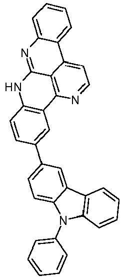 | 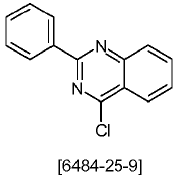 | 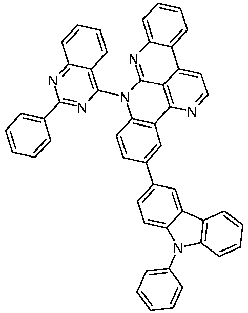 | 82% |
| 12p | 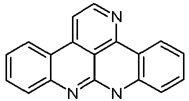 | 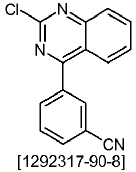 | 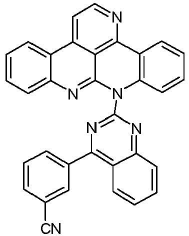 | 84% |
| 13p | 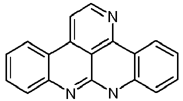 | 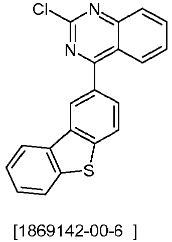 | 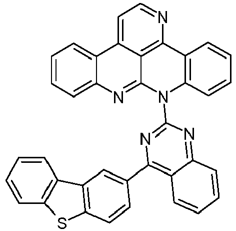 | 86% |
| 14p | 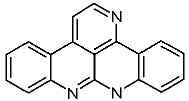 | 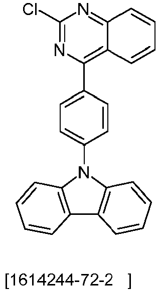 | 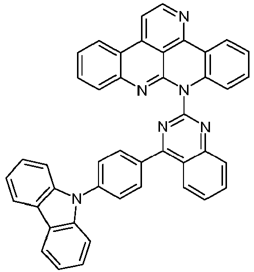 | 80% |

### q) 5-Brom-9-thia-1,8-diaza-benzo[fg]naphthacen

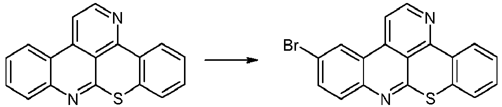

10.6 g (37.3 mmol) 9-Thia-1,8-diaza-benzo[fg]naphthacen werden in 80 mL DMF vorgelegt. Anschließend werden 13.3 g (74.6 mmol) NBS portionsweise zugegeben und die Reaktionsmischung 4 h bei 40 °C gerührt. Anschließend wird die Mischung mit 15 mL Wasser versetzt und mit CH₂Cl₂ extrahiert. Die organische Phase wird über MgSO₄ getrocknet und die Lösungsmittel im Vakuum entfernt. Das Produkt wird mit Hexan heiß ausgerührt und abgesaugt. Ausbeute: 11.4 g (31 mmol), 70 % der Theorie, Reinheit nach ¹H-NMR ca. 97 %.

Analog dazu werden die folgenden Verbindungen hergestellt:

| | **Edukt** | **Produkt** | **Ausbeute** |
|---|---|---|---|
| 1q | 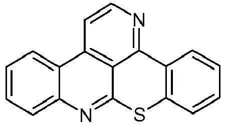 | 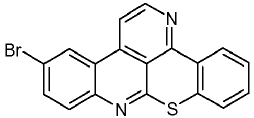 | 71% |
| 2q | 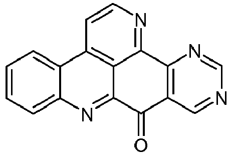 | 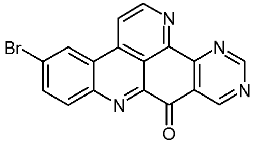 | 68% |
| 3q | 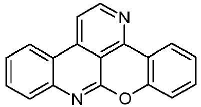 | 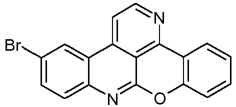 | 66% |
| 4q | 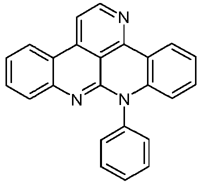 | 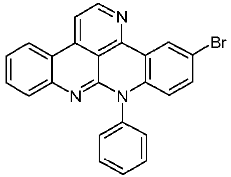 | 67% |

### r) 5-[3-Eth-(Z)-yliden-1-phenyl-2-prop-2-en-(E)-yliden-2,3-dihydro-1H-indol-5-yl]-9-thia-1,8-diaza-benzo[fg]naphthacen

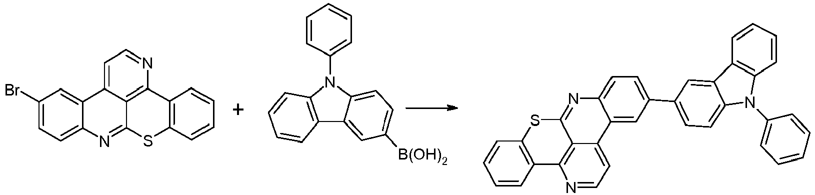

24 g (66 mmol) 5-Brom-9-thia-1,8-diaza-benzo[fg]naphthacen, 17 g (664 mmol) 2-N-Phenyl-carbazol-3-boronsäure und 13.7 g (100 mmol) Natriumtetraborat werden in 100 mL THF und 60 ml Wasser gelöst und entgast. Es wird mit 0.91 g (1.3 mmol) Bis(triphenylphosphin)palladium(II)chlorid und 1 g (20 mmol) Hydraziniumhydroxid versetzt. Die Reaktionsmischung wird anschließend bei 70 °C für 48 h unter Schutzgasatmosphäre gerührt. Die abgekühlte Lösung wird mit Toluol aufgestockt, mehrmals mit Wasser gewaschen, getrocknet und eingeengt. Das Produkt wird via Säulenchromatographie an Kieselgel mit Toluol/Heptan (1:2) gereinigt. Ausbeute: 29 g (55mmol), 84 % der Theorie.

Analog dazu werden die folgenden Verbindungen hergestellt:

| | **Edukt 1** | **Edukt 2** | **Produkt** | **Ausbeute** |
|---|---|---|---|---|
| 1r | 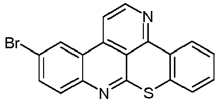 | 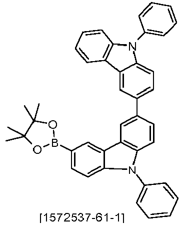 | 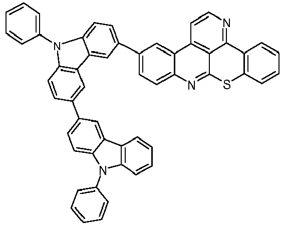 | **80%** |
| 2r | 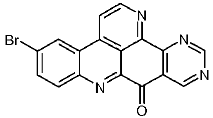 | 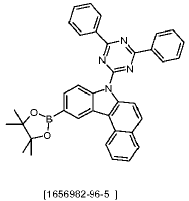 | 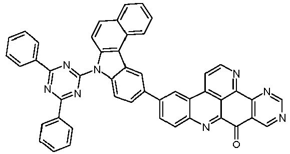 | **78%** |
| 3r | 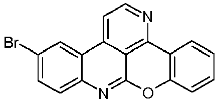 | 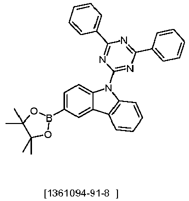 | 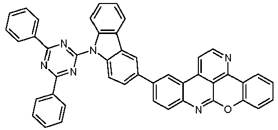 | **76%** |
| 4r | 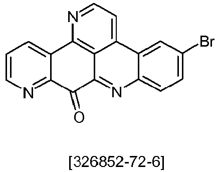 | 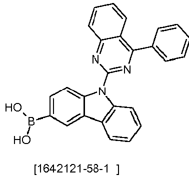 | 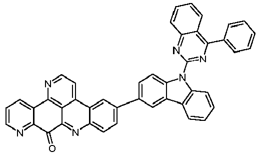 | **81%** |
| 5r | 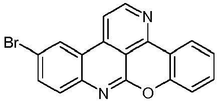 | 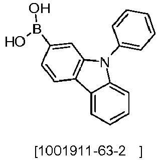 | 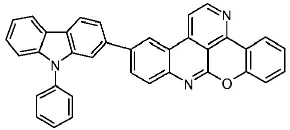 | **76%** |
| 6r | 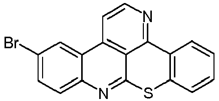 | 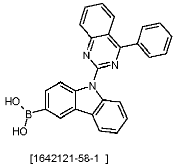 | 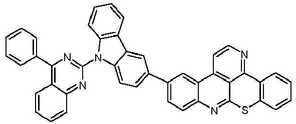 | **79%** |
| 7r | 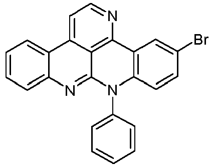 | 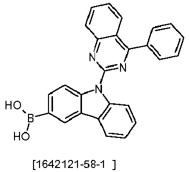 | 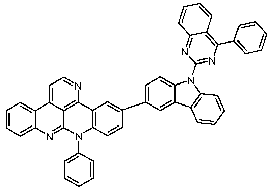 | **69%** |
| 8r | 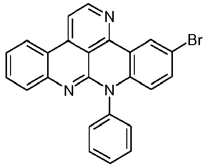 | 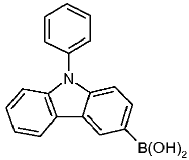 | 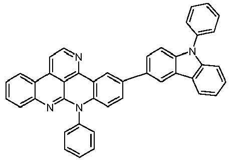 | **66%** |

### Herstellung der OLEDs

In den folgenden Beispielen E1 bis E21 (siehe Tabelle 1) wird der Einsatz der erfindungsgemäßen Materialien in OLEDs vorgestellt.

**Vorbehandlung für die Beispiele E1-E21:** Glasplättchen, die mit strukturiertem ITO (Indium Zinn Oxid) der Dicke 50 nm beschichtet sind, werden vor der Beschichtung zunächst mit einem Sauerstoffplasma, gefolgt von einem Argonplasma, behandelt. Diese mit Plasma behandelten Glasplättchen bilden die Substrate, auf welche die OLEDs aufgebracht werden.

Die OLEDs haben prinzipiell folgenden Schichtaufbau: Substrat / Lochinjektionsschicht (HIL) / Lochtransportschicht (HTL) / Elektronenblockierschicht (EBL) / Emissionsschicht (EML) / optionale Lochblockierschicht (HBL) / Elektronentransportschicht (ETL) / optionale Elektroneninjektionsschicht (EIL) und abschließend eine Kathode. Die Kathode wird durch eine 100 nm dicke Aluminiumschicht gebildet. Der genaue Aufbau der OLEDs ist Tabelle 1 zu entnehmen. Die zur Herstellung der OLEDs benötigten Materialien sind in Tabelle 2 gezeigt.

Alle Materialien werden in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie IC1:IC2:TER1(50%:45%:5%) bedeutet hierbei, dass das Material IC1 in einem Volumenanteil von 50%, IC2 in einem Volumenanteil von 45% und TER1 in einem Volumenanteil von 5% in der Schicht vorliegt. Analog kann auch die Elektronentransportschicht aus einer Mischung von zwei Materialien bestehen.

Die OLEDs werden standardmäßig charakterisiert. Die Elektrolumineszenzspektren werden bei einer Leuchtdichte von 1000 cd/m² bestimmt und daraus die CIE 1931 x und y Farbkoordinaten berechnet.

### Verwendung von erfindungsgemäßen Materialien in OLEDs

Die erfindungsgemäßen Materialien können in der Emissionsschicht in rot phosphoreszierenden OLEDs eingesetzt werden. Die erfindungsgemäßen Verbindungen EG1 bis EG21 werden in den Beispielen E1 bis E21 als Matrixmaterial in der Emissionsschicht eingesetzt. Die Farbkoordinaten der Elektrolumineszenzspektren der OLEDs liegen bei CIEx=0.67 und CIEy= 0.33. Somit eignen sich die Materialien für den Einsatz in der Emissionsschicht von roten OLEDs.

Des Weiteren lassen sich die erfindungsgemäßen Materialien in der Lochblockierschicht (HBL) oder Elektronenblockierschicht (EBL) erfolgreich einsetzen. Dies ist in den Versuchen E9 bzw. E21 gezeigt. Auch hier liegen die Farbkoordinaten des Spektrums der OLED jeweils bei CIEx=0.67 und CIEy= 0.33.

**Tabelle 1: Aufbau der OLEDs**

| Bsp | HIL Dicke | HTL Dicke | EBL Dicke | EML Dicke | HBL Dicke | ETL Dicke | EIL Dicke |
|---|---|---|---|---|---|---|---|
| E1 | HATCN | SpMA1 | SpMA2 | IC1:EG1:TER1 | --- | ST1:LiQ (50%:50%) | --- |
| | 5nm | 125nm | 10nm | (50%:45%:5%) 40nm | | 35nm | |
| E2 | HATCN | SpMA1 | SpMA2 | IC1:EG2:TER1 | --- | ST1:LiQ (50%:50%) | --- |
| | 5nm | 125nm | 10nm | (50%:45%:5%) 40nm | | 35nm | |
| E3 | HATCN | SpMA1 | SpMA2 | IC1:EG3:TER1 | --- | ST1:LiQ (50%:50%) | --- |
| | 5nm | 125nm | 10nm | (50%:45%:5%) 40nm | | 35nm | |
| E4 | HATCN | SpMA1 | SpMA2 | IC1:EG4:TER1 | --- | ST1:LiQ (50%:50%) | --- |
| | 5nm | 125nm | 10nm | (50%:45%:5%) 40nm | | 35nm | |
| E5 | HATCN | SpMA1 | SpMA2 | IC1:EG5:TER1 | --- | ST1:LiQ (50%:50%) | --- |
| | 5nm | 125nm | 10nm | (50%:45%:5%) 40nm | | 35nm | |
| E6 | HATCN | SpMA1 | SpMA2 | EG6:TER1 | --- | ST1:LiQ (50%:50%) | --- |
| | 5nm | 125nm | 10nm | (95%:5%) 40nm | | 35nm | |
| E7 | HATCN | SpMA1 | SpMA2 | IC1:EG7:TER1 | --- | ST1:LiQ (50%:50%) | --- |
| | 5nm | 125nm | 10nm | (50%:45%:5%) 40nm | | 35nm | |
| E8 | HATCN | SpMA1 | SpMA2 | IC1:EG8:TER1 | --- | ST1:LiQ (50%:50%) | --- |
| | 5nm | 125nm | 10nm | (50%:45%:5%) 40nm | | 35nm | |
| E9 | HATCN | SpMA1 | SpMA2 | EG9:TER1 | EG9 | ST1:LiQ (50%:50%) | --- |
| | 5nm | 125nm | 10nm | (95%:5%) 40nm | 5nm | 30nm | |
| E10 | HATCN | SpMA1 | SpMA2 | EG10:TER1 | --- | ST1:LiQ (50%:50%) | --- |
| | 5nm | 125nm | 10nm | (95%:5%) 40nm | | 35nm | |
| E11 | HATCN | SpMA1 | SpMA2 | IC1:EG11:TER1 | --- | ST1:LiQ (50%:50%) | --- |
| | 5nm | 125nm | 10nm | (50%:45%:5%) 40nm | | 35nm | |
| E12 | HATCN | SpMA1 | SpMA2 | EG12:TER1 | --- | ST1:LiQ (50%:50%) | --- |
| | 5nm | 125nm | 10nm | (95%:5%) 40nm | | 35nm | |
| E13 | HATCN | SpMA1 | SpMA2 | EG13:TER1 | --- | ST1:LiQ (50%:50%) | --- |
| | 5nm | 125nm | 10nm | (95%:5%) 40nm | | 35nm | |
| E14 | HATCN | SpMA1 | SpMA2 | IC2:EG14:TER1 | --- | ST1:LiQ (50%:50%) | --- |
| | 5nm | 125nm | 10nm | (50%:45%:5%) 40nm | | 35nm | |
| E15 | HATCN | SpMA1 | SpMA2 | EG15:TER1 | --- | ST1:LiQ (50%:50%) | --- |
| | 5nm | 125nm | 10nm | (95%:5%) 40nm | | 35nm | |
| E16 | HATCN | SpMA1 | SpMA2 | EG16:TER1 | --- | ST1:LiQ (50%:50%) | --- |
| | 5nm | 125nm | 10nm | (95%:5%) 40nm | | 35nm | |
| E17 | HATCN | SpMA1 | SpMA2 | EG17:TER1 | --- | ST1:LiQ (50%:50%) | --- |
| | 5nm | 125nm | 10nm | (95%:5%) 40nm | | 35nm | |
| E18 | HATCN | SpMA1 | SpMA2 | EG18:TER1 | --- | ST1:LiQ (50%:50%) | --- |
| | 5nm | 125nm | 10nm | (95%:5%) 40nm | | 35nm | |
| E19 | HATCN | SpMA1 | SpMA2 | IC2:EG19:TER1 | --- | ST1:LiQ (50%:50%) | --- |
| | 5nm | 125nm | 10nm | (50%:45%:5%) 40nm | | 35nm | |
| E20 | HATCN | SpMA1 | SpMA2 | EG20:TER1 | --- | ST1:LiQ (50%:50%) | --- |
| | 5nm | 125nm | 10nm | (95%:5%) 40nm | | 35nm | |
| E21 | HATCN | SpMA1 | EG21 | IC1:EG21:TER1 | --- | ST1:LiQ (50%:50%) | --- |
| | 5nm | 125nm | 10nm | (50%:45%:5%) 40nm | | 35nm | |

**Tabelle 2: Strukturformeln der Materialien für die OLEDs**

| | |
|---|---|
| 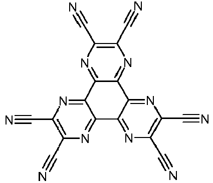 | 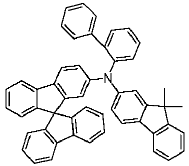 |
| HATCN | SpMA1 |
| 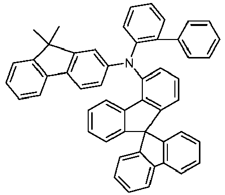 | 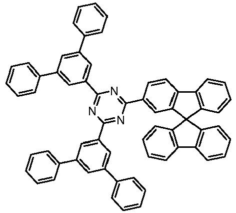 |
| SpMA2 | ST1 |
| 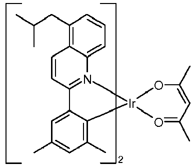 | 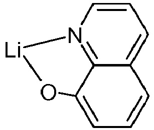 |
| TER1 | LiQ |
| 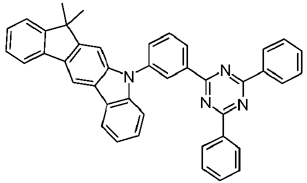 | 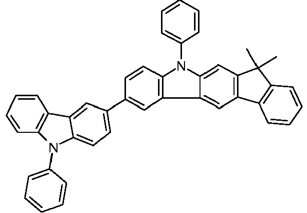 |
| IC1 | IC2 |
| 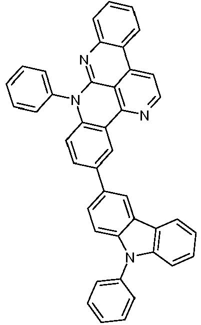 | 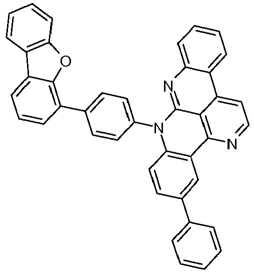 |
| EG1 | EG2 |
| 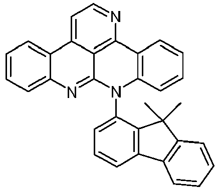 | 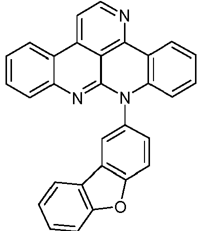 |
| EG3 | EG4 |
| 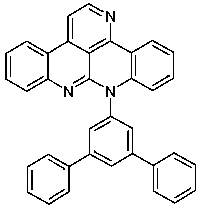 | 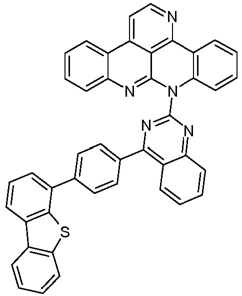 |
| EG5 | EG6 |
| 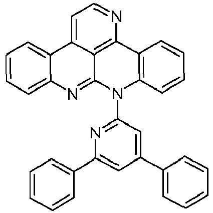 | 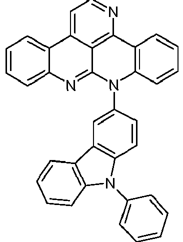 |
| EG7 | EG8 |
| 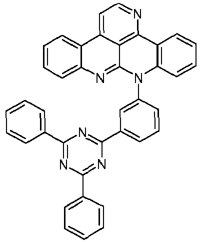 | 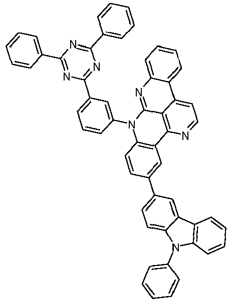 |
| EG9 | EG10 |
| 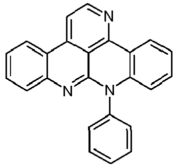 | 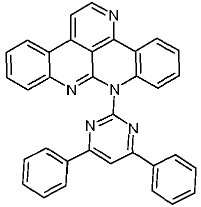 |
| EG11 | EG12 |
| 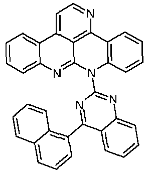 | 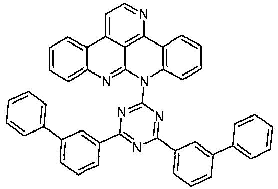 |
| EG13 | EG14 |
| 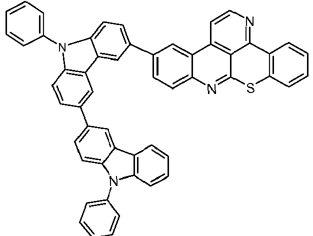 | 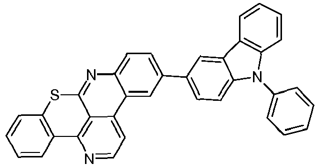 |
| EG15 | EG16 |
| 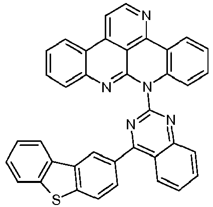 | 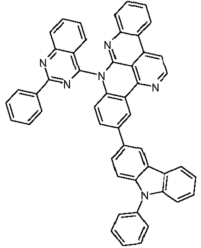 |
| EG17 | EG18 |
| 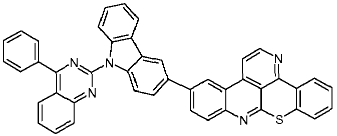 | 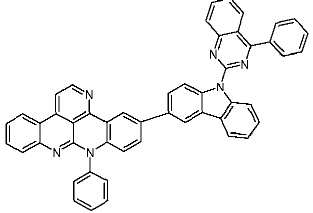 |
| EG19 | EG20 |
| 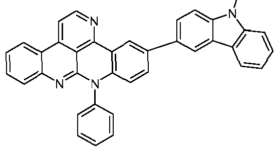 | |
| EG21 | |

## Patentansprüche

1. Verbindung gemäß Formel (1), (1') oder (4), 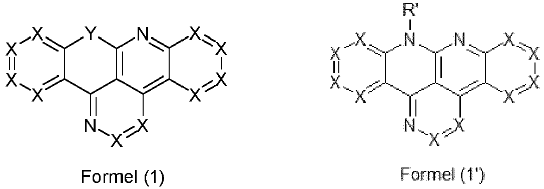 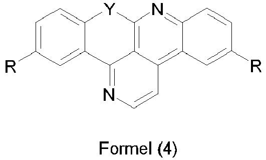
wobei für die verwendeten Symbole und Indizes gilt:
X ist bei jedem Auftreten gleich oder verschieden CR oder N, wobei maximal ein X für N steht;
Y ist NR', C(R")₂, C=O, BR', 0 oder S;
R, R" ist bei jedem Auftreten gleich oder verschieden H, D, F, Cl, Br, I, N(R¹)₂, NAr₂, CN, NO₂, OR¹, SR¹, COOR¹, C(=O)N(R¹)₂, Si(R¹)₃, C(=O)R¹, eine geradkettige Alkylgruppe mit 1 bis 20 C-Atomen oder eine Alkenyl- oder Alkinylgruppe mit 2 bis 20 C-Atomen oder eine verzweigte oder cyclische Alkylgruppe mit 3 bis 20 C-Atomen, wobei die Alkyl-, Alkenyl- oder Alkinylgruppe jeweils mit einem oder mehreren Resten R¹ substituiert sein kann und wobei eine oder mehrere nicht benachbarte CH₂-Gruppen durch Si(R¹)₂, C=O, NR¹, 0, S oder CONR¹ ersetzt sein können, oder ein aromatisches oder heteroaromatisches Ringsystem mit 5 bis 60 aromatischen Ringatomen, das jeweils durch einen oder mehrere Reste R¹ substituiert sein kann; dabei können zwei Reste R" auch miteinander ein aliphatisches, heteroaliphatisches, aromatisches oder heteroaromatisches Ringsystem bilden;
R' ist ein aromatisches oder heteroaromatisches Ringsystem mit 5 bis 40 aromatischen Ringatomen, das durch einen oder mehrere Reste R¹ substituiert sein kann;
Ar ist bei jedem Auftreten gleich oder verschieden ein aromatisches oder heteroaromatisches Ringsystem mit 5 bis 40 aromatischen Ringatomen, das durch einen oder mehrere Reste R¹ substituiert sein kann;
R¹ ist bei jedem Auftreten gleich oder verschieden H, D, F, Cl, Br, I, N(R²)₂, CN, NO₂, OR², SR², Si(R²)₃, C(=O)R², eine geradkettige Alkylgruppe mit 1 bis 20 C-Atomen oder eine Alkenyl- oder Alkinylgruppe mit 2 bis 20 C-Atomen oder eine verzweigte oder cyclische Alkylgruppe mit 3 bis 20 C-Atomen, wobei die Alkyl-, Alkenyl- oder Alkinylgruppe jeweils mit einem oder mehreren Resten R² substituiert sein kann und wobei eine oder mehrere nicht benachbarte CH₂-Gruppen durch Si(R²)₂, C=O, NR², O, S oder CONR² ersetzt sein können, oder ein aromatisches oder heteroaromatisches Ringsystem mit 5 bis 40 aromatischen Ringatomen, das jeweils durch einen oder mehrere Reste R² substituiert sein kann; dabei können zwei oder mehrere Reste R¹ miteinander ein Ringsystem bilden;
R² ist bei jedem Auftreten gleich oder verschieden H, D, F oder ein aliphatischer, aromatischer oder heteroaromatischer organischer Rest mit 1 bis 20 C-Atomen, in dem auch ein oder mehrere H-Atome durch F ersetzt sein können;
mit der Maßgabe, dass die Verbindung der Formel (1) genau einen Substituenten R enthält, der ausgewählt ist aus der Gruppe bestehend aus NAr₂ oder einem aromatischen oder heteroaromatischen Ringsystem mit 5 bis 60 aromatischen Ringatomen, welches durch einen oder mehrere Reste R¹ substituiert sein kann, und dass die übrigen Substituenten R in der Verbindung der Formel (1) = H sind;
und mit der Maßgabe, dass der Substituent R in der Verbindung der Formel (1') = H ist;
und mit der Maßgabe, dass die Verbindung der Formel (4) mindestens einen Substituenten R' enthält und/oder dass die Verbindung der Formel (4) mindestens einen Substituenten R enthält, der ausgewählt ist aus der Gruppe bestehend aus NAr₂ oder einem aromatischen oder heteroaromatischen Ringsystem mit 5 bis 60 aromatischen Ringatomen, welches durch einen oder mehrere Reste R¹ substituiert sein kann;
dabei sind die folgenden Verbindungen von der Erfindung ausgenommen:

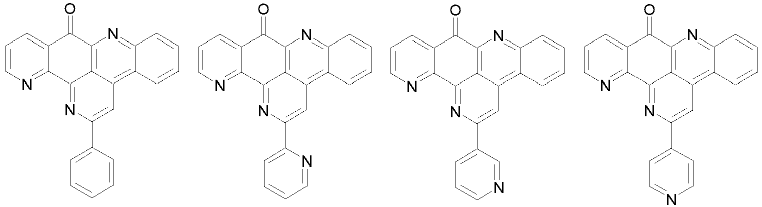

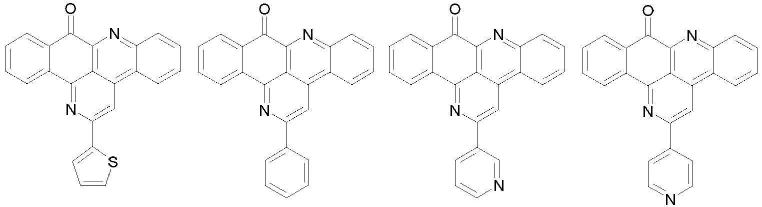

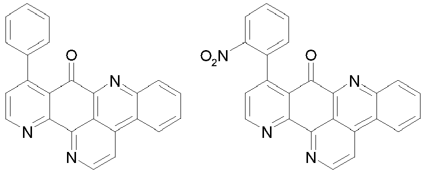

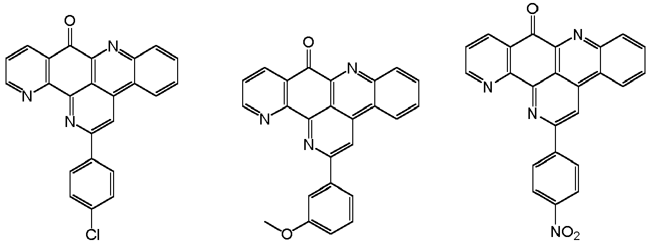

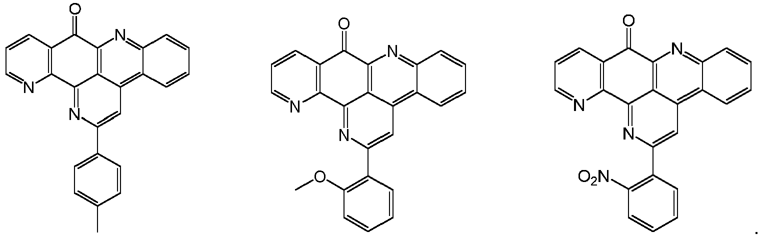

2. Verbindung nach Anspruch 1, **dadurch gekennzeichnet, dass** Y für NR', 0 oder S steht.

3. Verbindung nach Anspruch 1 oder 2 gemäß einer der Formeln (2a) bis (2k), 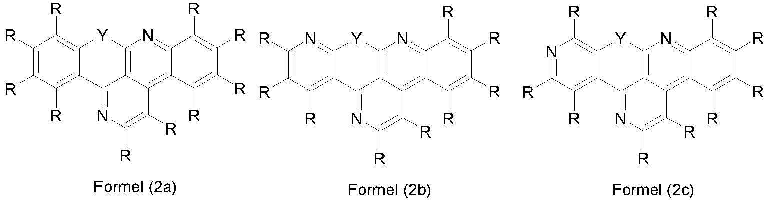 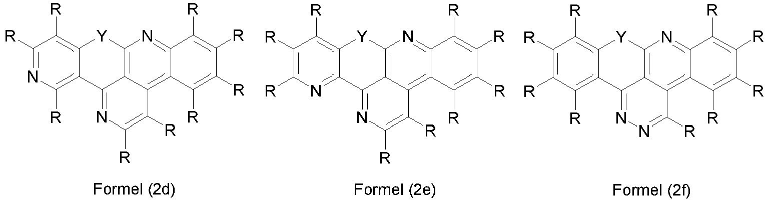 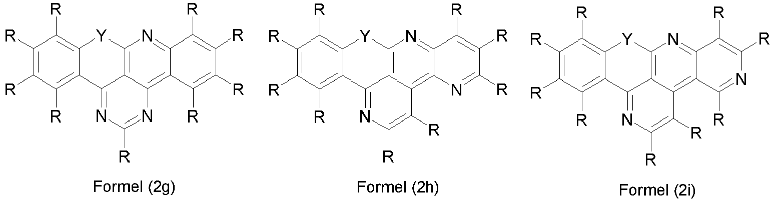 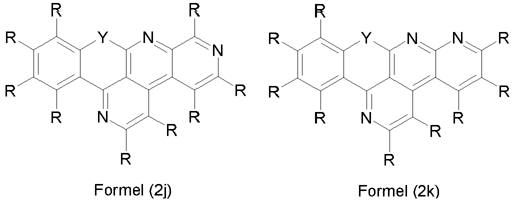 wobei die verwendeten Symbole die in Anspruch 1 genannten Bedeutungen aufweisen; und und mit der Maßgabe, dass die Verbindungen der Formeln (2a) bis (2k) mindestens einen Substituenten R' enthalten und/ oder dass die Verbindungen der Formeln (2a) bis (2k) mindestens einen Substituenten R enthalten, der ausgewählt ist aus der Gruppe bestehend aus NAr₂ oder einem aromatischen oder heteroaromatischen Ringsystem mit 5 bis 60 aromatischen Ringatomen, welches durch einen oder mehrere Reste R¹ substituiert sein kann, und dass die übrigen Substituenten R in den Verbindungen der Formeln (2a) bis (2k) = H sind.

4. Verbindung nach einem oder mehreren der Ansprüche 1 bis 3, gemäß den Formeln (2a') oder (2a"), 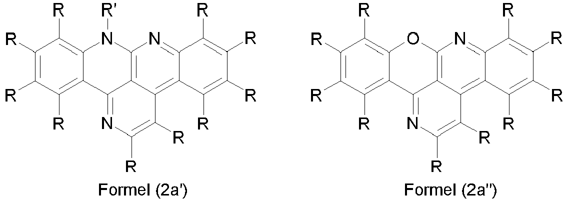 wobei R und R' die in Anspruch 1 genannten Bedeutungen aufweisen und keine oder genau eine Gruppe R in Formel (2a') und genau eine Gruppe R in Formel (2a") ausgewählt ist aus der Gruppe bestehend aus NAr₂ und einem aromatischen oder heteroaromatischen Ringsystem und wobei die übrigen Substituenten R in der Verbindung der Formeln (2a') und (2a") = H sind.

5. Verbindung nach einem oder mehreren der Ansprüche 1 bis 4 gemäß Formel (3), 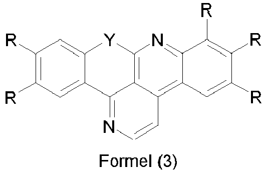 wobei die verwendeten Symbole die in Anspruch 1 genannten Bedeutungen aufweisen; und mit der Maßgabe, dass die Verbindung der Formel (3) mindestens einen Substituenten R' enthält und/oder dass die Verbindung der Formel (3) mindestens einen Substituenten R enthält, der ausgewählt ist aus der Gruppe bestehend aus NAr₂ oder einem aromatischen oder heteroaromatischen Ringsystem mit 5 bis 60 aromatischen Ringatomen, welches durch einen oder mehrere Reste R¹ substituiert sein kann, und wobei die übrigen Substituenten R in der Verbindung der Formel (3) = H sind.

6. Verbindung nach einem oder mehreren der Ansprüche 1 bis 5 gemäß Formel (5) oder Formel (6), 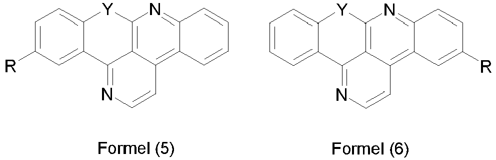 wobei die verwendeten Symbole die in Anspruch 1 genannten Bedeutungen aufweisen; und mit der Maßgabe, dass die Verbindungen der Formeln (5) und (6) mindestens einen Substituenten R' enthalten und/ oder dass die Verbindungen der Formeln (5) und (6) einen Substituenten R enthalten, der ausgewählt ist aus der Gruppe bestehend aus NAr₂ oder einem aromatischen oder heteroaromatischen Ringsystem mit 5 bis 60 aromatischen Ringatomen, welches durch einen oder mehrere Reste R¹ substituiert sein kann.

7. Verbindung nach einem oder mehreren der Ansprüche 1 bis 6 gemäß den Formeln (5'), (5"), (6') oder (6"), 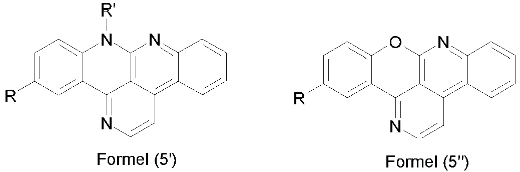 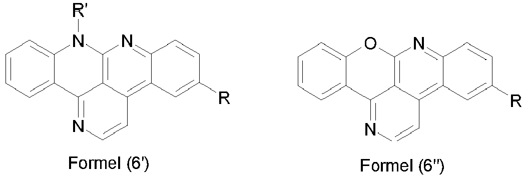 wobei R und R' die in Anspruch 1 genannten Bedeutungen aufweisen und die Gruppe R in den Formeln (5') und (6') ausgewählt ist aus der Gruppe bestehend aus H, NAr₂ und einem aromatischen oder heteroaromatischen Ringsystem und die Gruppe R in den Formeln (5") und (6") ausgewählt ist aus der Gruppe bestehend aus NAr₂ und einem aromatischen oder heteroaromatischen Ringsystem.

8. Verbindung nach einem oder mehreren der Ansprüche 1 bis 7, **dadurch gekennzeichnet, dass** R bzw. R' bzw. Ar, wenn diese für aromatische bzw. heteroaromatische Ringsystem stehen, ausgewählt sind aus Phenyl, Biphenyl, Terphenyl, Quaterphenyl, Fluoren, Spirobifluoren, Naphthalin, Indol, Benzofuran, Benzothiophen, Carbazol, Dibenzofuran, Dibenzothiophen, Indenocarbazol, Indolocarbazol, Pyridin, Pyrimidin, Pyrazin, Pyridazin, Triazin, Phenanthren, Triphenylen oder einer Kombination aus zwei oder drei dieser Gruppen, welche jeweils mit einem oder mehreren Resten R¹ substituiert sein können.

9. Formulierung, enthaltend mindestens eine Verbindung nach einem oder mehreren der Ansprüche 1 bis 8 und mindestens eine weitere Verbindung, wobei die weitere Verbindung ein oder mehrere Lösemittel und/oder eine weitere organische oder anorganische Verbindung ist.

10. Verwendung einer Verbindung nach einem oder mehreren der Ansprüche 1 bis 8 in einer elektronischen Vorrichtung, insbesondere in einer organischen Elektrolumineszenzvorrichtung.

11. Elektronische Vorrichtung enthaltend mindestens eine Verbindung nach einem oder mehreren der Ansprüche 1 bis 8.

12. Elektronische Vorrichtung nach Anspruch 11, wobei es sich um eine organische Elektrolumineszenzvorrichtung handelt, **dadurch gekennzeichnet, dass** die Verbindung nach einem oder mehreren der Ansprüche 1 bis 8 als Matrixmaterial für phosphoreszierende Emitter und/oder in einer Elektronentransportschicht und/oder in einer Lochblockierschicht eingesetzt wird.

## Claims

1. Compound of formula (1), (1') or (4) 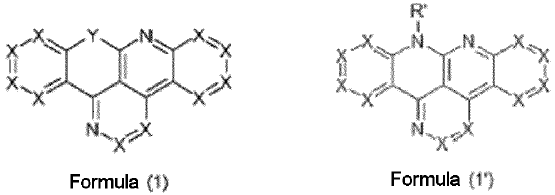 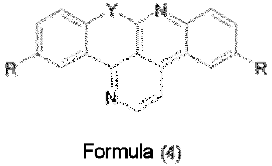
where the symbols and indices used are as follows:
X is the same or different at each instance and is CR or N, where not more than one X is N;
Y is NR', C(R")₂, C=O, BR', O or S;
R, R" are the same or different at each instance and are H, D, F, Cl, Br, I, N(R¹)₂, NAr₂, CN, NO₂, OR¹, SR¹, COOR¹, C(=O)N(R¹)₂, Si(R¹)₃, C(=O)R¹, a straight-chain alkyl group having 1 to 20 carbon atoms or an alkenyl or alkynyl group having 2 to 20 carbon atoms or a branched or cyclic alkyl group having 3 to 20 carbon atoms, where the alkyl, alkenyl or alkynyl group may in each case be substituted by one or more R¹ radicals and where one or more nonadjacent CH₂ groups may be replaced by Si(R¹)₂, C=O, NR¹, O, S or CONR¹, or an aromatic or heteroaromatic ring system which has 5 to 60 aromatic ring atoms and may be substituted in each case by one or more R¹ radicals; at the same time, two R" radicals together may also form an aliphatic, heteroaliphatic, aromatic or heteroaromatic ring system;
R' is an aromatic or heteroaromatic ring system which has 5 to 40 aromatic ring atoms and may be substituted by one or more R¹ radicals;
Ar is the same or different at each instance and is an aromatic or heteroaromatic ring system which has 5 to 40 aromatic ring atoms and may be substituted by one or more R¹ radicals;
R¹ is the same or different at each instance and is H, D, F, Cl, Br, I, N(R²)₂, CN, NO₂, OR², SR², Si(R²)₃, C(=O)R², a straight-chain alkyl group having 1 to 20 carbon atoms or an alkenyl or alkynyl group having 2 to 20 carbon atoms or a branched or cyclic alkyl group having 3 to 20 carbon atoms, where the alkyl, alkenyl or alkynyl group may in each case be substituted by one or more R² radicals and where one or more nonadjacent CH₂ groups may be replaced by Si(R²)₂, C=O, NR², O, S or CONR², or an aromatic or heteroaromatic ring system which has 5 to 40 aromatic ring atoms and may be substituted in each case by one or more R² radicals; at the same time, two or more R¹ radicals together may form a ring system;
R² is the same or different at each instance and is H, D, F or an aliphatic, aromatic or heteroaromatic organic radical having 1 to 20 carbon atoms, in which one or more hydrogen atoms may also be replaced by F;
with the proviso that the compound of the formula (1) contains exactly one substituent R selected from the group consisting of NAr₂ or an aromatic or heteroaromatic ring system which has 5 to 60 aromatic ring atoms and may be substituted by one or more R¹ radicals, and that the rest of the substituents R in the compound of the formula (1) are H;
and with the proviso that the substituent R in the compound of the formula (1') is H;
and with the proviso that the compound of the formula (4) contains at least one substituent R' and/or that the compound of the formula (4) contains at least one substituent R selected from the group consisting of NAr₂ or an aromatic or heteroaromatic ring system which has 5 to 60 aromatic ring atoms and may be substituted by one or more R¹ radicals;
where the following compounds are excluded from the invention:

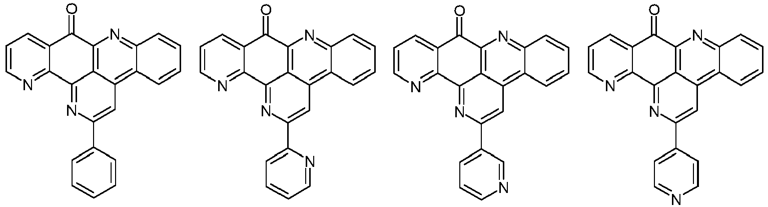

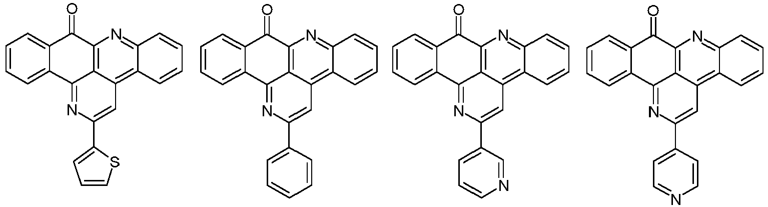

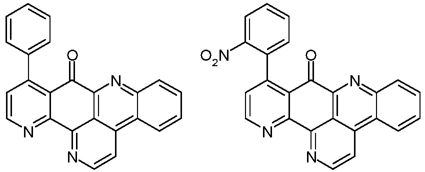

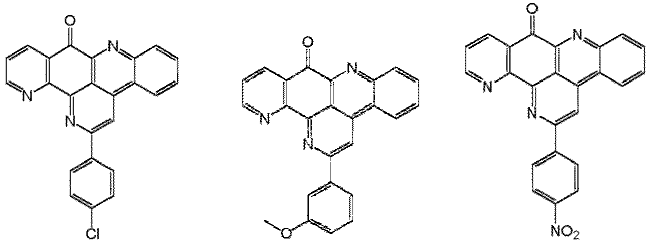

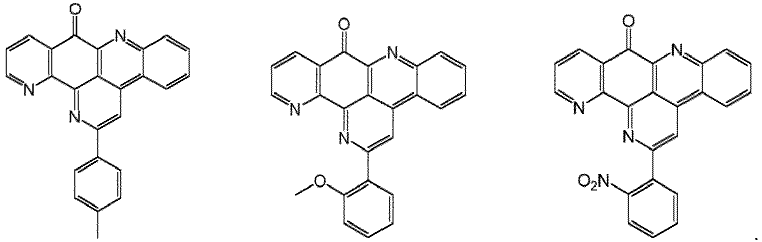

2. Compound according to Claim 1, **characterized in that** Y is NR', O or S.

3. Compound according to Claim 1 or 2 of one of the formulae (2a) to (2k) 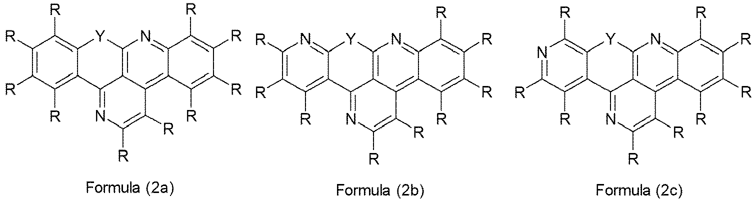 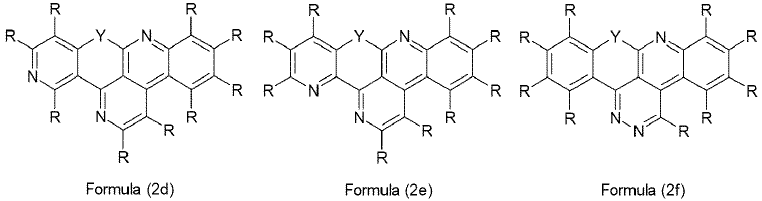 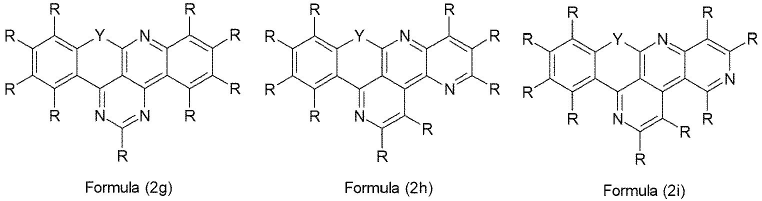 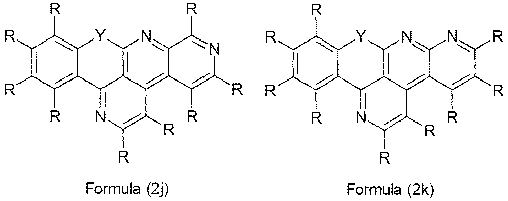 where the symbols used have the definitions given in Claim 1; and with the proviso that the compounds of the formulae (2a) to (2k) contain at least one substituent R' and/or that the compounds of the formulae (2a) to (2k) contain at least one substituent R selected from the group consisting of NAr₂ or an aromatic or heteroaromatic ring system which has 5 to 60 aromatic ring atoms and may be substituted by one or more R¹ radicals, and that the other substituents R in the compounds of the formulae (2a) to (2k) are H.

4. Compound according to one or more of Claims 1 to 3 of one of the formulae (2a') and (2a'') 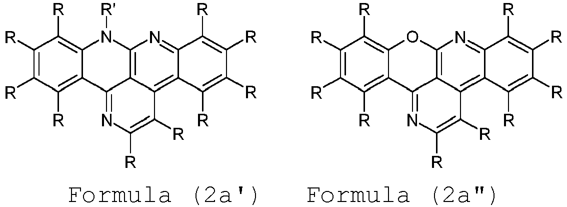 where R and R' have the definitions given in Claim 1 and no or exactly one R group in formula (2a') and exactly one R group in formula (2a") is selected from the group consisting of NAr₂ and an aromatic or heteroaromatic ring system, and where the other substituents R in the compound of the formulae (2a') and (2a") are H.

5. Compound according to one or more of Claims 1 to 4 of formula (3) 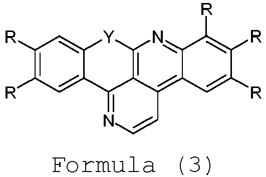 where the symbols used have the definitions given in Claim 1; and with the proviso that the compound of the formula (3) contains at least one substituent R' and/or that the compound of the formula (3) contains at least one substituent R selected from the group consisting of NAr₂ or an aromatic or heteroaromatic ring system which has 5 to 60 aromatic ring atoms and may be substituted by one or more R¹ radicals, and where the other substituents R in the compound of the formulae (3) are H.

6. Compound according to one or more of Claims 1 to 5 of formula (5) or formula (6) 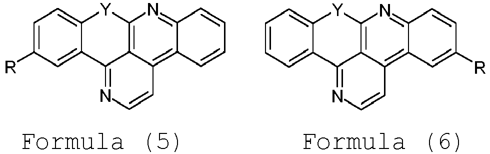 where the symbols used have the definitions given in Claim 1; and with the proviso that the compounds of the formulae (5) and (6) contain at least one substituent R' and/or that the compounds of the formulae (5) and (6) contain one substituent R selected from the group consisting of NAr₂ or an aromatic or heteroaromatic ring system which has 5 to 60 aromatic ring atoms and may be substituted by one or more R¹ radicals.

7. Compound according to one or more of Claims 1 to 6 of one of the formulae (5'), (5''), (6') and (6'') 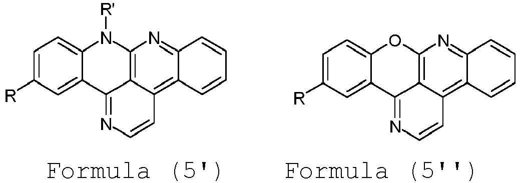 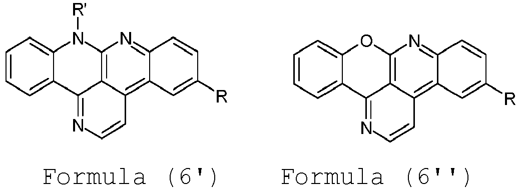 where R and R' have the definitions given in Claim 1 and the R group in the formulae (5') and (6') is selected from the group consisting of H, NAr₂ and an aromatic or heteroaromatic ring system and the R group in the formulae (5") and (6") is selected from the group consisting of NAr₂ and an aromatic or heteroaromatic ring system.

8. Compound according to one or more of Claims 1 to 7, **characterized in that** R or R' or Ar', when they are aromatic or heteroaromatic ring systems, are selected from phenyl, biphenyl, terphenyl, quaterphenyl, fluorene, spirobifluorene, naphthalene, indole, benzofuran, benzothiophene, carbazole, dibenzofuran, dibenzothiophene, indenocarbazole, indolocarbazole, pyridine, pyrimidine, pyrazine, pyridazine, triazine, phenanthrene, triphenylene or a combination of two or three of these groups, each of which may be substituted by one or more R¹ radicals.

9. Formulation comprising at least one compound according to one or more of Claims 1 to 8 and at least one further compound, where the further compound is one or more solvents and/or a further organic or inorganic compound.

10. Use of a compound according to one or more of Claims 1 to 8 in an electronic device, especially in an organic electroluminescent device.

11. Electronic device comprising at least one compound according to one or more of Claims 1 to 8.

12. Electronic device according to Claim 11 which is an organic electroluminescent device, **characterized in that** the compound according to one or more of Claims 1 to 8 is used as matrix material for phosphorescent emitters and/or in an electron transport layer and/or in a hole blocker layer.

## Revendications

1. Composé selon la formule générale (1), (1') ou (4), 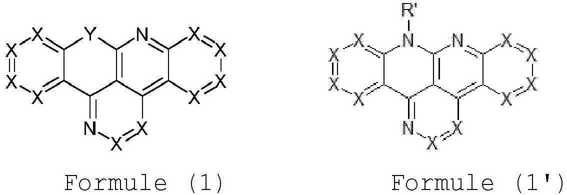 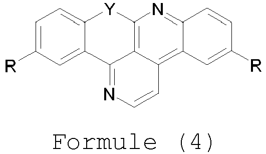
où, pour les symboles et indices utilisés, ce qui suit est d'application :
X représente en chaque occurrence, de manière identique ou différente, CR ou N, au maximum un X représentant N ;
Y représente NR', C(R")₂, C=O, BR', O ou S ;
R, R" représentent en chaque occurrence, de manière identique ou différente, H, D, F, Cl, Br, I, N(R¹)₂, NAr₂, CN, NO₂, OR¹, SR¹, COOR¹, C(=O)N(R¹)₂, Si(R¹)₃, C(=O)R¹, un groupe alkyle linéaire comprenant 1 à 20 atomes de carbone ou un groupe alcényle ou alcynyle comprenant 2 à 20 atomes de carbone ou un groupe alkyle ramifié ou cyclique comprenant 3 à 20 atomes de carbone, le groupe alkyle, alcényle ou alcynyle pouvant à chaque fois être substitué par un ou plusieurs radicaux R¹ et un ou plusieurs groupes CH₂ non adjacents pouvant être remplacés par Si(R¹)₂, C=O, NR¹, O, S ou CONR¹, ou un système cyclique aromatique ou hétéroaromatique comprenant 5 à 60 atomes de cycle aromatique, qui peut à chaque fois être substitué par un ou plusieurs radicaux R¹ ; deux radicaux R" pouvant également former ensemble un système cyclique aliphatique, hétéroaliphatique, aromatique ou hétéroaromatique ;
R' représente un système cyclique aromatique ou hétéroaromatique comprenant 5 à 40 atomes de cycle aromatique, qui peut être substitué par un ou plusieurs radicaux R¹ ;
Ar représente en chaque occurrence, de manière identique ou différente, un système cyclique aromatique ou hétéroaromatique comprenant 5 à 40 atomes de cycle aromatique, qui peut être substitué par un ou plusieurs radicaux R¹ ;
R¹ représente en chaque occurrence, de manière identique ou différente, H, D, F, Cl, Br, I, N(R²)₂, CN, NO₂, OR², SR², Si(R²)₃, C(=O)R², un groupe alkyle linéaire comprenant 1 à 20 atomes de carbone ou un groupe alcényle ou alcynyle comprenant 2 à 20 atomes de carbone ou un groupe alkyle ramifié ou cyclique comprenant 3 à 20 atomes de carbone, le groupe alkyle, alcényle ou alcynyle pouvant à chaque fois être substitué par un ou plusieurs radicaux R² et un ou plusieurs groupes CH₂ non adjacents pouvant être remplacés par Si(R²)₂, C=O, NR², O, S ou CONR², ou un système cyclique aromatique ou hétéroaromatique comprenant 5 à 40 atomes de cycle aromatique, qui peut à chaque fois être substitué par un ou plusieurs radicaux R² ; deux radicaux R¹ pouvant également former ensemble un système cyclique ;
R² représente en chaque occurrence, de manière identique ou différente, H, D, F ou un radical organique aliphatique, aromatique et/ou hétéroaromatique comprenant 1 à 20 atomes de carbone, dans lequel un ou plusieurs atomes H peuvent également être remplacés par F ;
sous réserve que le composé de formule (1) contienne exactement un substituant R, qui est choisi dans le groupe constitué par NAr₂ ou par un système cyclique aromatique ou hétéroaromatique comprenant 5 à 60 atomes de cycle aromatique, qui peut être substitué par un ou plusieurs radicaux R¹, et que les autres substituants R dans le composé de formule (1) = H ;
et sous réserve que le substituant R dans le composé de formule (1') = H ;
et sous réserve que le composé de formule (4) contienne au moins un substituant R' et/ou que le composé de formule (4) contienne au moins un substituant R qui est choisi dans le groupe constitué par NAr₂ ou par un système cyclique aromatique ou hétéroaromatique comprenant 5 à 60 atomes de cycle aromatique, qui peut être substitué par un ou plusieurs radicaux R¹ ;
les composés suivants étant exclus de l'invention :

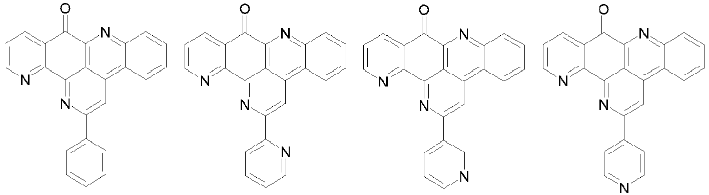

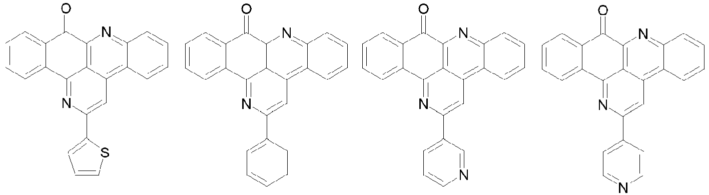

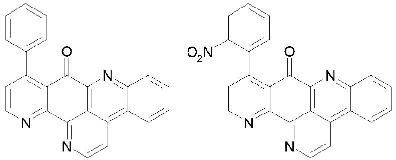

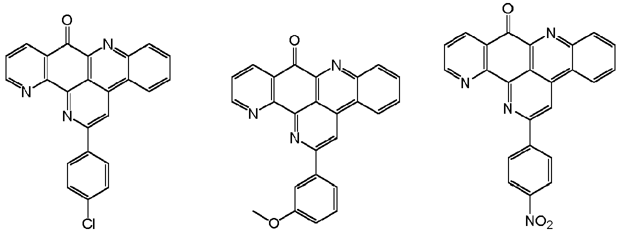

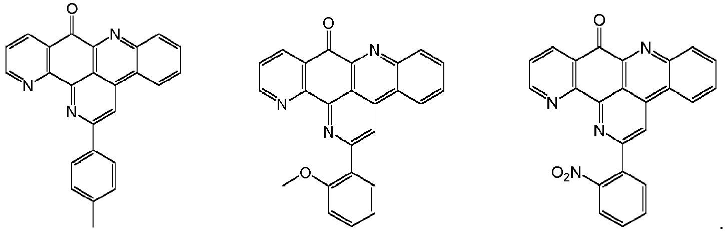

2. Composé selon la revendication 1, **caractérisé en ce que** Y représente NR', O ou S.

3. Composé selon la revendication 1 ou 2 selon l'une quelconque des formules (2a) à(2k), 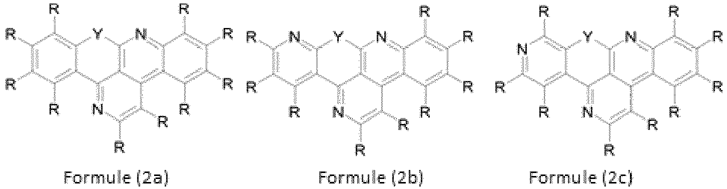 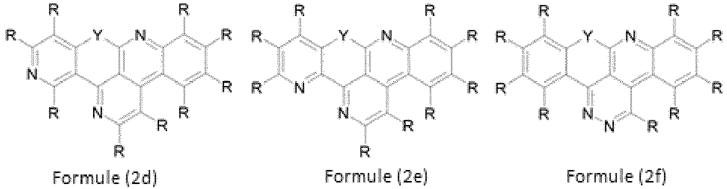 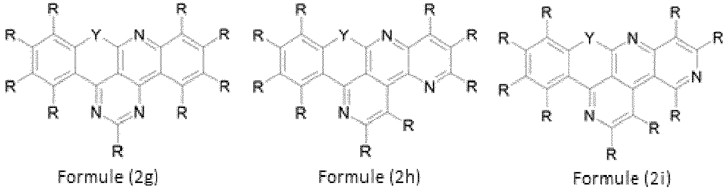 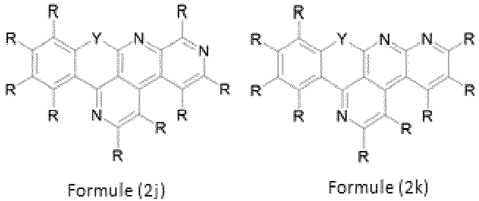 les symboles utilisés présentant les significations mentionnées dans la revendication 1 ; et sous réserve que les composés des formules (2a) à (2k) contiennent au moins un substituant R' et/ou que les composés des formules (2a) à (2k) contiennent au moins un substituant R qui est choisi dans le groupe constitué par NAr₂ ou par un système cyclique aromatique ou hétéroaromatique comprenant 5 à 60 atomes de cycle aromatique, qui peut être substitué par un ou plusieurs radicaux R¹ et que les autres substituants R dans les composés des formules (2a) à (2k) = H.

4. Composé selon l'une ou plusieurs des revendications 1 à 3, selon les formules (2a') ou (2a"), 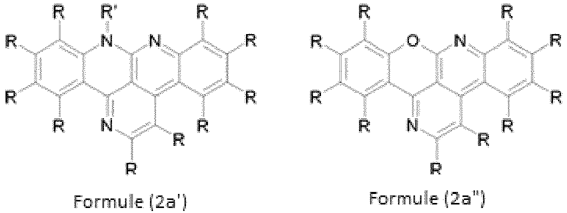 R et R' présentant les significations mentionnées dans la revendication 1 et aucun ou exactement un groupe R dans la formule (2a') et exactement un groupe R dans la formule (2a") étant choisis dans le groupe constitué par NAr₂ et par un système cyclique aromatique ou hétéroaromatique et les autres substituants R dans le composé des formules (2a') et (2a") = H.

5. Composé selon l'une ou plusieurs des revendications 1 à 4, selon la formule (3), 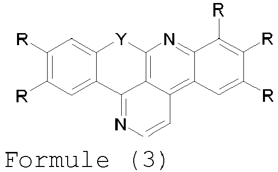 les symboles utilisés présentant les significations mentionnées dans la revendication 1 ; et sous réserve que le composé de formule (3) contienne au moins un substituant R' et/ou que le composé de formule (3) contienne au moins un substituant R qui est choisi dans le groupe constitué par NAr₂ ou par un système cyclique aromatique ou hétéroaromatique comprenant 5 à 60 atomes de cycle aromatique, qui peut être substitué par un ou plusieurs radicaux R¹ et les autres substituants R dans le composé de formule (3) = H.

6. Composé selon l'une ou plusieurs des revendications 1 à 5, selon la formule (5) ou la formule (6), 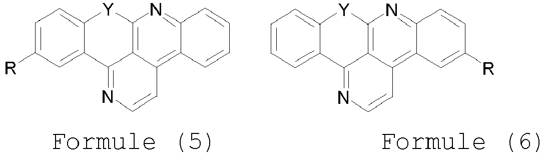 les symboles utilisés présentant les significations mentionnées dans la revendication 1 ; et sous réserve que les composés des formules (5) et (6) contiennent au moins un substituant R' et/ou que les composés des formules (5) et (6) contiennent un substituant R qui est choisi dans le groupe constitué par NAr₂ ou par un système cyclique aromatique ou hétéroaromatique comprenant 5 à 60 atomes de cycle aromatique, qui peut être substitué par un ou plusieurs radicaux R¹.

7. Composé selon l'une ou plusieurs des revendications 1 à 5, selon les formules (5'), (5"), (6') ou (6"), 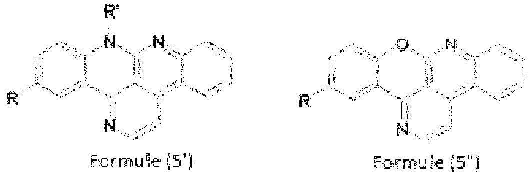 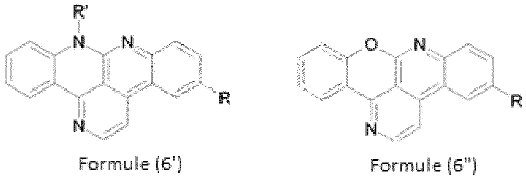 R et R' présentant les significations mentionnées dans la revendication 1 et le groupe R dans les formules (5') et (6') étant choisi dans le groupe constitué par H, NAr₂ et par un système cyclique aromatique ou hétéroaromatique et le groupe R dans les formules (5") et (6") étant choisi dans le groupe constitué par NAr₂ et par un système cyclique aromatique ou hétéroaromatique.

8. Composé selon l'une ou plusieurs des revendications 1 à 7, **caractérisé en ce que** R ou, selon le cas, R' ou, selon le cas, Ar, lorsque ceux-ci représentent un système cyclique aromatique ou hétéroaromatique, sont choisis parmi phényle, biphényle, terphényle, quaterphényle, fluorène, spirobifluorène, naphtalène, indole, benzofuranne, benzothiophène, carbazole, dibenzofuranne, dibenzothiophène, indénocarbazole, indolocarbazole, pyridine, pyrimidine, pyrazine, pyridazine, triazine, phénanthrène, triphénylène ou une combinaison de deux ou trois de ces groupes, qui peuvent à chaque fois être substitués par un ou plusieurs radicaux R¹.

9. Formulation, contenant au moins un composé selon l'une ou plusieurs des revendications 1 à 8 et au moins un autre composé, l'autre composé étant un ou plusieurs solvants et/ou un autre composé organique ou inorganique.

10. Utilisation d'un composé selon l'une ou plusieurs des revendications 1 à 8 dans un dispositif électronique, en particulier dans un dispositif organique électroluminescent.

11. Dispositif électronique contenant au moins un composé selon l'une ou plusieurs des revendications 1 à 8.

12. Dispositif électronique selon la revendication 11, le dispositif étant un dispositif organique électroluminescent, **caractérisé en ce que** le composé selon l'une ou plusieurs des revendications 1 à 8 est utilisé comme matériau de matrice pour des émetteurs phosphorescents et/ou dans une couche de transport d'électrons et/ou dans une couche de blocage de trous.
